(19) Europäisches Patentamt
European Patent Office
Office européen des brevets

(11) EP 4 053 179 B1

(12) EUROPEAN PATENT SPECIFICATION

(45) Date of publication and mention of the grant of the patent:
**27.08.2025 Bulletin 2025/35**

(21) Application number: **21160045.7**

(22) Date of filing: **01.03.2021**

(51) International Patent Classification (IPC):
***C08F 226/02*** (2006.01) ***A61K 31/785*** (2006.01)
***A61P 3/00*** (2006.01) ***A61P 7/00*** (2006.01)
***C08F 8/02*** (2006.01)

(52) Cooperative Patent Classification (CPC):
(C-Sets available)
**C08F 226/02; A61K 31/785; A61P 3/00; A61P 7/00; C08F 8/02** (Cont.)

# (54) CROSSLINKED POLY(ALLYLAMINE) POLYMER PHARMACEUTICAL COMPOSITIONS

PHARMAZEUTISCHE ZUSAMMENSETZUNGEN MIT VERNETZTEN POLY(ALLYLAMIN)-POLYMEREN

COMPOSITIONS PHARMACEUTIQUES À BASE DE POLYMÈRES RÉTICULÉS DE POLY(ALLYLAMINE)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(43) Date of publication of application:
**07.09.2022 Bulletin 2022/36**

(73) Proprietor: **Renosis, Inc.**
**Southlake, TX 76092 (US)**

(72) Inventors:
- **KLAERNER, Gerrit**
  **South San Francisco, CA California 94080 (US)**
- **CONNOR, Eric F.**
  **South San Francisco, CA California 94080 (US)**
- **GBUR, Randi K.**
  **South San Francisco, CA California 94080 (US)**
- **KADE, Matthew J.**
  **South San Francisco, CA California 94080 (US)**
- **KIERSTEAD, Paul H.**
  **South San Francisco, CA California 94080 (US)**
- **BIYANI, Kalpesh N.**
  **South San Francisco, CA California 94080 (US)**
- **NGUYEN, Son H.**
  **South San Francisco, CA California 94080 (US)**
- **TABAKMAN, Scott M.**
  **South San Francisco, CA California 94080 (US)**

(74) Representative: **Carpmaels & Ransford LLP**
**One Southampton Row**
**London WC1B 5HA (GB)**

(56) References cited:
**WO-A1-2016/094685**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08F 8/02, C08F 226/02;**
C08F 226/02, C08F 226/04

## Description

The present disclosure relates to crosslinked poly(allylamine) polymers of the present invention as defined in appended claim 1. The crosslinked poly(allylamine) polymers may be used as a non absorbed pharmaceutical for a therapeutic application, for example, treating metabolic acidosis. More specifically, it relates to pharmaceutical compositions comprising such polymers, pharmaceutical products comprising such polymers or pharmaceutical compositions thereof.

The present disclosure provides methods and processes for the preparation of crosslinked poly(allylamine) polymers. Those methods and processes are not claimed but are useful for understanding how to make crosslinked poly(allylamine) polymers of the present invention.

Any references to methods of treatment by therapy or surgery in this description are to be interpreted as references to crosslinked poly(allylamine) polymers, compositions, or pharmaceutical compositions for use in those methods.

In U.S. Patent No. 9,205,107, Klaerner et al. disclose crosslinked poly(allylamine) polymers that may be used to treat metabolic conditions in which removal of protons and/or chloride ions from the gastrointestinal tract would provide physiological benefits such as increasing serum bicarbonate concentrations. A range of crosslinked amine polymers are disclosed therein, including crosslinked poly(allylamine) polymers prepared by simultaneous polymerization of allylamine and crosslinking the polymer with a diallylamine.

Veverimer is a crosslinked poly(allylamine) polymer that may be used to treat metabolic conditions. Wesson et al. "Long-term safety and efficacy of veverimer in patients with metabolic acidosis in chronic kidney disease: a multicentre, randomised, blinded, placebo-controlled, 40-week extension", The Lancet, Volume 394, Issue 10196, P396-406, 2019 and Bushinsky et al. "Randomized, Controlled Trial of TRC101 to Increase Serum Bicarbonate in Patients with CKD", Clin. J. Am. Soc. Nephrol. 13: 26-35, 2018 disclose the treatment of metabolic acidosis with veverimer.

One way of making the polymeric drug veverimer is described in Exemplary Synthesis A of WO2019/236636 A1 and in WO2016/094685 A1. The veverimer polymer made in this way is unique ID 019070-A3 FA in Table S-1 of Exemplary Synthesis A of WO2019/236636 A1. Disclosed herein is an improved veverimer polymer, for example due to its improved stablilty in the presence of oxygen, as well as processes by which it can be made, and packaged veverimer designed to mitigate oxygen stability concerns.

Although the preparation of poly(allylamine) polymers from allylamine and a diallylamine as described by Bianchi et al., Inoue et al. and Klaerner et al. provide certain advantages over crosslinking a linear poly(allylamine) with epichlorohydrin, the resulting crosslinked amine polymers may have undesirable process-related impurities or degradation products such as allylamine or derivatives thereof. In some instances, incomplete incorporation of diallylamine or multiallylamines, such as 1,3-bis(allylamino)propane, or a salt thereof, can lead to unsaturated substituents covalently attached to the polymer backbone that can cause stability limiting generation of allylamine and related impurities during the work up including drying of the poly(allylamine) or upon storage. Such mechanisms of generating allylamine impurities include but are not limited to oxygen, temperature, water, acid and base mediated removal of the unsaturated substituents resulting in allylamine or related impurities.

One approach to improve the purity profile and stability profile of a polymer, such as veverimer, comprising the residues of 2-propen-1-ylamine, or a salt thereof, and 1,3-bis(allylamino)propane, or a salt thereof, is to improve the polymerization efficiency (*i.e.,* improve conversion of the allyl groups). In general, methods to improve polymerization efficiency are those that favor chain propagation over propagation-limiting processes, such as chain transfer, chain termination, and radical coupling. During the radical polymerization step, these methods include, but are not limited to:

a) increasing the concentration of 2-propen-1-ylamine, or a salt thereof, and 1,3-bis(allylamino)propane, or a salt thereof, in the monomer droplet solution,
b) increasing the relative concentration of 1,3-bis(allylamino)propane, or a salt thereof, by adding 2-propen-1-ylamine, or a salt thereof, in a semi-batch or continuous process over the course of the reaction,
c) reducing the reaction temperature to favor propagation, or
d) increasing the initiator concentration or amount.

Any one, or combination, of these or other methods could improve crosslinking efficiency as measured by residual polymer backbone attached (i.e., pendent) allyl groups in $^{13}C$ NMR and reduced allylamine formation in the final polymer, preferably veverimer. Specifically, by minimizing the number of unreacted allyl groups from the 1,3-bis(allylamino)propane, or a salt thereof, the purity profile and stability profile of the resulting polymer, preferably veverimer, can be improved.

Among the various aspects of the present disclosure, therefore, may be noted a process for the preparation of compositions having therapeutic applications. The process comprises

(a) in a first step, forming a poly(allylamine) polymer in the form of beads in a concurrent polymerization and

crosslinking reaction mixture comprising 2-propen-1-ylamine, or a salt thereof, 1,3-bis(allylamino)propane, or a salt thereof, a radical polymerization initiator, a surfactant, an acid, water and an organic solvent system wherein less than 1.1% of the total number of carbon atoms comprised by the crosslinked poly(allylamine) polymer are $sp^2$ allyl carbons, and

(b) in a second step, further crosslinking the poly(allylamine) polymer in a reaction mixture comprising 1,2-dichloroethane, a swelling agent for the poly(allylamine) polymer, and a dispersing solvent system to form a crosslinked poly(allylamine) polymer having a swelling ratio of less than 2.

A further aspect of the present disclosure is a pharmaceutical composition comprising a crosslinked poly(allylamine) polymer of the present invention.

**Veverimer is a polymeric drug that can have the following structural properties:**

Veverimer can be poly(allylamine-co-N,N'-diallyl-1,3-diaminopropane-co-1,2-diaminoethane).

More specifically, veverimer can be a poly[($N^1$,$N^3$-di(prop-2-en-1-yl)propane-1,3-diamine)-co-prop-2-en-1-amine], crosslinked with *N,N'*-ethane-1,2-diyl bridges (the mole ratio is ≈ 2:5:2 (i.e. it is about 2 : about 5: about 2 of $N^1$,$N^3$-di(prop-2-en-1-yl)propane-1,3-diamine : prop-2-en-1-amine : *N,N'*-ethane-1,2-diyl bridges)) polymer.

A structural representation of veverimer can be Formula 5:

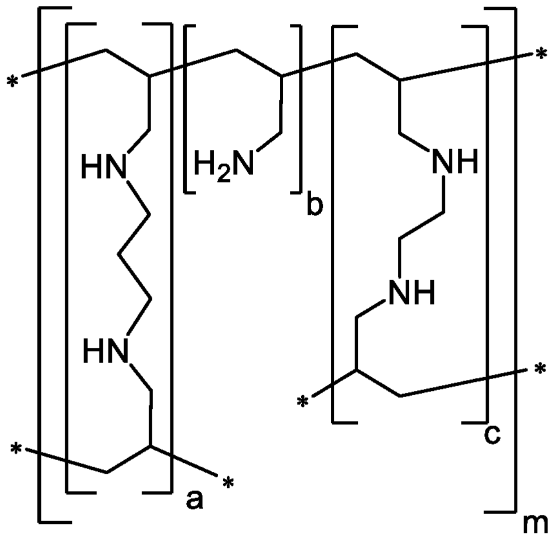

Formula 5,

wherein

a = residue of *N,N'*-diallyl-1,3-diaminopropane dihydrochloride,
b = residue of allylamine,
c = residue of 1,2-dichloroethane (ethylene crosslink between two amines); an ethylene linkage between two allylamine groups is shown as an example of one of many possible linkages between amines,
m = a large number indicating an extended polymer network.

Veverimer can comprise the following amounts of residues of monomers:

a) 20-25 mol% residue of N,N'-diallyl-1,3-diaminopropane, or a salt thereof, (also called 1,3-bis(allylamino)propane, or a salt thereof),
b) 50-60 mol% residue of 2-propen-1-ylamine, or a salt thereof, and
c) 20-25 mol% residue of 1,2-dichloroethane,

wherein the total mol% of the residues is not greater than 100 mol%.

Veverimer can have a carbon to nitrogen weight ratio in the range of about 3.7:1 to about 3.8:1, respectively. The carbon to nitrogen weight ratio may be determined by elemental analysis. For example, the carbon to nitrogen weight ratio may be determined by elemental analysis using a Perkin-Elmer 2400 Elemental Analyzer as more fully described elsewhere herein.

Veverimer can be a nonabsorbable composition that is insoluble, e.g. under physiological conditions.

Veverimer can have a median particle size of greater than 1 micrometer and less than 1 millimeter. The particle size of veverimer may be measured by wet laser diffraction using Mie theory.

**Veverimer may be prepared as follows:**

Veverimer is obtainable by first copolymerizing 2-propen-1-ylamine, or a salt thereof, and 1,3-bis(allylamino)propane, or a salt thereof, to form a poly(allylamine) polymer, followed by crosslinking the poly(allylamine) polymer with 1,2-dichloroethane.

For example, veverimer is obtainable by first copolymerizing 2-propen-1-ylamine hydrochloride and 1,3-bis(allylamino)propane dihydrochloride to form a poly(allylamine) polymer, followed by crosslinking the poly(allylamine) polymer with 1,2-dichloroethane.

## SUMMARY OF THE INVENTION

The invention is set out in the appended set of claims.

A further aspect of the present disclosure is a unit dosage form having an exterior and interior, the interior comprising a crosslinked poly(allylamine) polymer of the present invention wherein the oxygen transfer rate between the exterior and interior of the unit dosage form is less than about 0.050 CC/m2/day.

A further aspect of the present disclosure is a unit dosage form comprising a sealed enclosure containing a crosslinked poly(allylamine) polymer of the present invention the sealed enclosure comprising a multi-layer laminate of an inner contact layer, an outer layer; and a barrier layer disposed between the contact layer and outer layer, wherein the oxygen transfer rate between the multi-layer laminate is less than about 0.050 $CC/m^2/day$.

A further aspect of the present disclosure is a composition comprising a crosslinked poly(allylamine) polymer of the present invention for use in therapy.

A further aspect of the present disclosure is a composition comprising a crosslinked poly(allylamine) polymer of the present invention for treating an acid base disorder.

A further aspect of the present disclosure is a composition comprising a crosslinked poly(allylamine) polymer of the present invention for use in treating metabolic acidosis.

A further aspect of the present disclosure is a composition comprising a crosslinked poly(allylamine) polymer of the present invention for use in slowing kidney diease progression.

A further aspect of the present disclosure is a composition comprising a crosslinked poly(allylamine) polymer of the present invention for use in slowing chronic kidney diease progression.

A further aspect of the present disclosure is a composition comprising a crosslinked poly(allylamine) polymer of the present invention for use in slowing kidney diease progression in a patient with metabolic acidosis associated with chronic kidney disease.

A further aspect of the present disclosure is a composition comprising a crosslinked poly(allylamine) polymer of the present invention for use in improving the physical function of a patient.

A further aspect of the present disclosure is a composition comprising a crosslinked poly(allylamine) polymer of the present invention for use in improving the physical function of a patient with metabolic acidosis.

A further aspect of the present disclosure is a composition comprising a crosslinked poly(allylamine) polymer of the present invention for use in improving the quality of life of a patient.

A further aspect of the present disclosure is a composition comprising a crosslinked poly(allylamine) polymer of the present invention for use in improving the quality of life of a patient with metabolic acidosis.

A further aspect of the present disclosure is a crosslinked poly(allylamine) polymer of the present invention in a sealed container.

A further aspect of the present disclosure is a pharmaceutical product comprising a crosslinked poly(allylamine) polymer of the present invention in a sealed container comprising a moisture barrier.

A further aspect of the present disclosure is a pharmaceutical product comprising a crosslinked poly(allylamine) polymer of the present invention in a sealed container comprising an oxygen barrier.

A further aspect of the present disclosure is a pharmaceutical product comprising a crosslinked poly(allylamine) polymer of the present invention in a sealed container comprising a moisture barrier and an oxygen barrier.

A further aspect of the present disclosure is a pharmaceutical product comprising a crosslinked poly(allylamine) polymer of the present invention in a sealed sachet.

A further aspect of the present disclosure is a pharmaceutical product comprising a crosslinked poly(allylamine) polymer of the present invention in a sealed container comprising a polymer, metal, glass or ceramic material.

A further aspect of the present disclosure is a pharmaceutical product comprising a sealed container containing a crosslinked poly(allylamine) polymer of the present invention and an inert atmosphere.

A further aspect of the present disclosure is a pharmaceutical product comprising a sealed container and a crosslinked poly(allylamine) polymer of the present invention within the sealed container, the sealed container comprising

a multi-layer laminate of an inner contact layer, an outer layer; and a barrier layer disposed between the contact layer and outer layer.

A further aspect of the present disclosure is a pharmaceutical product comprising a sealed container and a crosslinked poly(allylamine) polymer of the present invention within the sealed container, the sealed container comprising a multi-layer laminate of an inner contact layer, an outer layer; and an oxygen-barrier layer disposed between the contact layer and outer layer.

A further aspect of the present disclosure is a pharmaceutical product comprising a sealed container and a crosslinked poly(allylamine) polymer of the present invention within the sealed container, the sealed container comprising a multi-layer laminate of an inner contact layer, an outer layer; and a moisture-barrier layer disposed between the contact layer and outer layer.

A further aspect of the present disclosure is a pharmaceutical product comprising a sealed container and a crosslinked poly(allylamine) polymer of the present invention within the sealed container, the sealed container comprising a multi-layer laminate of an inner contact layer, an outer layer; and an oxygen-barrier layer and a moisture-barrier layer disposed between the contact layer and outer layer.

A further aspect of the present disclosure is a pharmaceutical product comprising a sealed container and a crosslinked poly(allylamine) polymer of the present invention within the sealed container, the sealed container comprising a multi-layer laminate of an inner contact layer, an outer layer; and an oxygen-scavenging layer disposed between the contact layer and the outer layer.

A further aspect of the present disclosure is a method of treating an acid/base disorder in an animal by oral administration of a pharmaceutical composition comprising a crosslinked poly(allylamine) of the present invention.

A further aspect of the present disclosure is a method of treating an individual afflicted with an acid-base disorder characterized by a baseline serum bicarbonate value of less than 22 mEq/l, the method comprising oral administration of a daily dose of a pharmaceutical composition comprising a crosslinked poly(allylamine) of the present invention to increase the patient's serum bicarbonate value by at least 1 mEq/l from baseline within a treatment period not greater than 1 month.

Other aspects and features will be in part apparent and in part pointed out hereinafter.

## ABBREVIATIONS AND DEFINITIONS

The following definitions and methods are provided to better define the present invention and to guide those of ordinary skill in the art in the practice of the present invention. Unless otherwise noted, terms are to be understood according to conventional usage by those of ordinary skill in the relevant art. The term "absorption capacity" as used herein in connection with a polymer and a swelling agent (or in the case of a mixture of swelling agents, the mixture of swelling agents) is the amount of the swelling agent (or such mixture) absorbed during a period of at least 16 hours at room temperature by a given amount of a dry polymer (e.g., in the form of a dry bead) immersed in an excess amount of the swelling agent (or such mixture).

The abbreviations appearing in the following table shall have the indicated meaning:

| Abbreviation | Meaning |
|---|---|
| AA | Allylamine ($CH_2CHCH_2NH_2$) |
| AA at release | The amount of allylamine upon first isolating the polymer at the point of completion of manufacture |
| AA Stability | The amount (ppm) of allylamine formed per day in either the "In-Air Stability Assay (Stability Assay 1)", the "Heated Stability Assay (Stability assay 2)", or the "Stability assessment when packaged in a MylarFoil sachet assay (Stability Assay 3)" |
| AME at release | The amount of allylmethylether ($CH_2CHCH_2OCH_3$) upon first isolating the polymer at the point of completion of manufacture |
| AOH at release | The amount of allylalcohol ($CH_2CHCH_2OH$) upon first isolating the polymer at the point of completion of manufacture |
| DCE | 1,2-dichloroethane ($ClCH_2CH_2Cl$) |
| DCE at release | The amount of 1,2-dichloroethane upon first isolating the polymer at the point of completion of manufacture |
| DCP | 1,3-dichloropropane ($ClCH_2CH_2CH_2Cl$) |

The term "alicyclic" means a saturated monocyclic group of 3 to 8 carbon atoms and includes cyclopentyl,

cyclohexyl, cycloheptyl, and the like.

The term "alkyl group" as used, either alone or within other terms, encompasses saturated linear or branched carbon radicals having, for example, one to about twenty carbon atoms or, in specific embodiments, one to about twelve carbon atoms. In other embodiments, alkyl groups are "lower alkyl" groups having one to about six carbon atoms. Examples of such groups include, but are not limited thereto, methyl, ethyl, n-propyl, isopropyl, n-butyl, isobutyl, sec-butyl, tert-butyl, pentyl, iso-amyl, hexyl and the like. In more specific embodiments, lower alkyl groups have one to four carbon atoms.

The term "allyl" as used herein denotes a moiety having the structural formula $H_2C=CH-CH_2-*$, where * denotes the point of attachment of the moiety to the remainder of the molecule. In some embodiments, the point of attachment * is to a heteroatom in the remainder of the molecule, such as nitrogen.

The term "allyl equivalents" as used herein means the total number of moles of allyl groups comprised by 2-propen-1-ylamine, or a salt thereof, and 1,3-bis(allylamino)propane, or a salt thereof, in combination, present in a reaction mixture.

The terms "allylamine" and AAH denote a moiety having the structural formula $H_2C=CH-CH_2NH_2$.

The term "aqueous solid content" refers to the concentration of solids in the aqueous phase at the start of polymerization = (mass AAH + mass DAPDA + mass initiator (e.g. V-50)) / (mass AAH + mass DAPDA + mass initiator (e.g. V-50) + mass water); "mass initiator" constitutes the mass from the first initiator (e.g. V-50) addition only. To express aqueous solid content as a wt%, the value is multipled by 100.

The term "aromatic group" or "aryl group" means an aromatic group having one or more rings wherein such rings may be attached together in a pendent manner or may be fused. In particular embodiments, an aromatic group is one, two or three rings. Monocyclic aromatic groups may contain 5 to 10 carbon atoms, typically 5 to 7 carbon atoms, and more typically 5 to 6 carbon atoms in the ring. Typical polycyclic aromatic groups have two or three rings. Polycyclic aromatic groups having two rings typically have 8 to 12 carbon atoms, preferably 8 to 10 carbon atoms in the rings. Examples of aromatic groups include, but are not limited to, phenyl, naphthyl, tetrahydronaphthyl, indanyl, biphenyl, phenanthryl, anthryl or acenaphthyl.

The phrase "at release" shall mean in the as-manufactured state at the point of completion of manufacture.

A "batch process" as used herein denotes a process in which the reactants are fed into a reactor, the reaction is carried out, and the reaction product is removed from the reactor at the conclusion of the reaction.

The term "bead" is used to describe a crosslinked polymer that is substantially spherical in shape.

The term "bicarbonate equivalent" is used to describe an organic acid or anion that yields bicarbonate when metabolized. Citrate and succinate are exemplary bicarbonate equivalents.

The term "binds" as used herein in connection with a polymer and one or more ions, that is, a cation (e.g. "proton-binding" polymer) and an anion, is an "ion-binding" polymer and/or when it associates with the ion, generally though not necessarily in a noncovalent manner, with sufficient association strength that at least a portion of the ion remains bound under the in vitro or in vivo conditions in which the polymer is used for sufficient time to effect a removal of the ion from solution or from the body.

In certain embodiments, the term "clinically significant increase" as used herein in connection with a treatment refers to a treatment that improves or provides a worthwhile change in an individual from a dysfunctional state back to a relatively normal functioning state, or moves the measurement of that state in the direction of normal functioning, or at least a marked improvement to untreated. A number of methods can be used to calculate clinical significance. A non-exhaustive list of methods for calculating clinical significance includes: Jacobson-Truax, Gulliksen-Lord-Novick, Edwards-Nunnally, Hageman-Arrindell, and Hierarchical Linear Modeling (HLM).

A "continuous process" as used herein denotes a process in which one or more reactants are continuously fed into a reactor and a reaction product emerges as a continuous stream of product.

The term "crosslinker" as used, either alone or within other terms, encompasses ethylene crosslinkers, for example a dihaloethane selected from the group consisting of 1,2-dichloroethane, 1,2-dibromoethane, 1,2-diiodoethane, 1,2-difluoroethane, 1-chloro-2-iodoethane, 1-chloro-2-bromoethane, 1-chloro-2-fluoroethane, 1-bromo-2-iodoethane, 1-fluoro-2-iodoethane and 1-fluoro-2-bromoethane. Any reference herein to 1,2-dichloroethane could be replaced with any ethylene crosslinker, including those disclosed in this paragraph.

The term "diallylamine" denotes an amino moiety having two allyl groups.

The terms "dry bead" and "dry polymer" refer to beads or polymers that contain no more than 5% by weight of a non-polymer swelling agent or solvent. Often the swelling agent/solvent is water remaining at the end of a purification. This is generally removed by lyophilization or oven drying before storage or further crosslinking of a preformed poly(allylamine) polymer. The amount of swelling agent/solvent can be measured by heating (e.g., heating to 100-200°C) and measuring the resulting change in weight. This is referred to a "loss on drying" or "LOD."

The term "gel" is used to describe a crosslinked polymer that has an irregular shape.

The term "heteroaryl" means a monocyclic or bicyclic aromatic radical of 5 to 10 ring atoms, unless otherwise stated, where one or more, (in one embodiment, one, two, or three), ring atoms are heteroatom selected from N, O, or S, the

remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like. As defined herein, the terms "heteroaryl" and "aryl" are mutually exclusive. "Heteroarylene" means a divalent heteroaryl radical.

The term "heteroatom" means an atom other than carbon and hydrogen. Typically, but not exclusively, heteroatoms are selected from the group consisting of halogen, sulfur, phosphorous, nitrogen, boron and oxygen atoms. Groups containing more than one heteroatom may contain different heteroatoms.

The term "heterocyclo," "heterocyclic," or heterocyclyl" means a saturated or unsaturated group of 4 to 8 ring atoms in which one or two ring atoms are heteroatom such as N, O, B, P and $S(O)_n$, where n is an integer from 0 to 2, the remaining ring atoms being carbon. Additionally, one or two ring carbon atoms in the heterocyclyl ring can optionally be replaced by a -C(O)- group. More specifically the term heterocyclyl includes, but is not limited to, pyrrolidino, piperidino, homopiperidino, 2-oxopyrrolidinyl, 2-oxopiperidinyl, morpholino, piperazino, tetrahydro-pyranyl, thiomorpholino, and the like. When the heterocyclyl ring is unsaturated it can contain one or two ring double bonds provided that the ring is not aromatic. When the heterocyclyl group contains at least one nitrogen atom, it is also referred to herein as heterocycloamino and is a subset of the heterocyclyl group.

"Initiator" is a term used to describe a reagent that initiates a polymerization.

The mole percentage or "mol%" of a particular component of the crosslinked poly(allylamine) polymer can be calculated as follows:

$$mol\%\ of\ component = \frac{number\ of\ moles\ of\ component}{total\ number\ of\ moles\ of\ crosslinked\ poly(allylamine)\ polymer\ components} \times 100.$$

wherein the crosslinked poly(allylamine) polymer components are the residues specified in a given embodiment. For example, the residues of 2-propen-1-ylamine, or a salt thereof, 1,3-bis(allylamino)propane, or a salt thereof, and 1,2-dichloroethane.

The crosslinked poly(allylamine) polymer may be defined by the mol% of the components of the crosslinked poly(allylamine) polymer (e.g. the residues of the monomers and crosslinking agent). In these embodiments, the total mol% may not exceed 100 mol%. For example, the mol% of 1,3-bis(allylamino)propane, or a salt thereof, plus the mol% of 2-propen-1-ylamine, or a salt thereof, plus the mol% of 1,2-dichloroethane is ≤ 100 mol%.

A worked example of how the mole ratio and mol% may be calculated is provide below. This example uses the known input mole ratio of 2-propen-1-ylamine, or a salt thereof and 1,3-bis(allylamino)propane, or a salt thereof, used to produce the intermediate poly(allylamine) polymer and the amount of HCl generated by the second step 1,2-dichloroethane (DCE) crosslinking of the poly(allylamine) polymer to calculate the mol% of each component in the final crosslinked poly(allylamine) polymer.

This example is based on the principle that DCE can react with 2 amine groups so creates 2 equivalents of HCl when it introduces an ethylene residue into the crosslinked poly(allylamine) polymer. Thus the amount of HCl produced indicates how many ethylene residues have been incorporated into the crosslinked poly(allylamine) polymer.

This example is performed on a sample of crude crosslinked poly(allylamine) polymer (i.e. immediately after DCE crosslinking). The HCl is extracted from the sample of crude crosslinked poly(allylamine) polymer with sodium hydroxide solution and Cl is analysed by anion chromatography.

More specifically, about 2 grams of crude crosslinked poly(allylamine) polymer was sampled immediately after DCE crosslinking reaction. The sample was washed with 20 mL of methanol by mixing for two hours on an orbital shaker and then vacuum filtering over a frit. The methanol wash was repeated for a total of two washes. The loss on drying of the methanol washed polymer was determined by standard use of a moisture balance. About 100 mg of methanol-washed crude crosslinked poly(allylamine) polymer was accurately weighed into a vial and 10 mL of aqueous 50 mM sodium hydroxide solution was added. Cl was extracted from the polymer for 16 hours at 37 C, after which the supernatant was filtered through a 0.45 um nylon syringe filter. Anion chromatography was used to determine the Cl concentration of the supernatant. The IC (e.g. Dionex ICS-5000, Thermo Scientific) method consists of a AG19 guard column and AS19 analytical column, a potassium hydroxide (KOH) eluent generator, an injection volume of 25 microliters, with a run time of about 17 minutes and flow rate of 1.0 mL/min. The KOH concentration is 20 mM for 8 minutes, followed by a hold at 70 mM KOH for 4 minutes, and re-equilibration at 20 mM KOH for 5 minutes. A chloride standard was used for quantitation as follows

Chloride concentration in mM in the samples solutions was calculated as follows:

$$C = \frac{P}{P_s} \times C_s$$

Wherein:

C = concentration of chloride in the sample solution, mM;
Cs = concentration of chloride in Chloride Standard, mM;
P = peak area of the sample;
Ps = average peak area of 6 injections of Chloride Standard.

This calculation can be performed by the chromatography data system.
Determination of poly(allylamine) polymer mass extracted:

$$W_P\ (mg) = \frac{W_S \times (100 - LOD)}{100} - \frac{C \times 0.01 \times 98.96}{2}$$

wherein:

$W_P$ = Sample mass converted to poly(allylamine) polymer in dry, free amine form, mg;
Ws = Mass of sample, mg;
LOD = Loss on drying of sample (wt%);
100 = Conversion of percent into fraction for LOD;
C = Concentration of chloride in the extraction filtrate, mM;
0.01 = volume of 50 mM Sodium Hydroxide Solution, L;
98.96 = Molecular mass of 1,2-dichloroethane, g/mol;
2 = Molar ratio of chloride to 1,2-dichloroethane.

Determination of Reacted DCE:

$$Reacted\ DCE\ (\frac{mmol\ Cl}{g\ poly(allylamine)\ polymer}) = \frac{{}^{C}/_{2} \times 0.01}{W_P}$$

wherein:

C = Concentration of chloride in the extraction filtrate, mM;
$W_P$ = Mass of poly(allylamine) polymer calculated above, mg;
0.01 = volume of 50 mM Sodium Hydroxide Solution, L.

**Calculation of the weight percent of ethylene crosslinks per gram poly(allylamine) polymer**

For example, the following amount of ethylene may be calculated using the method described above: 3.97 mmol ethylene / g poly(allylamine) polymer.

3.97 mmol ethylene/g poly(allylamine) polymer = 111.1 mg ethylene/g poly(allylamine) polymer;

Wt% ethylene/g poly(allylamine) polymer = 111.1 mg ethylene/(1000 mg poly(allylamine) polymer + 111.1 mg ethylene) = 10 wt% ethylene or DCE residues in the crosslinked poly(allylamine) polymer.

**Calculation of the weight percent of the residues of 2-propen-1-ylamine, or a salt thereof, and 1,3-bis(allylamino)propane, or a salt thereof in the crosslinked poly(allylamine) polymer:**

The wt% of the residues of 2-propen-1-ylamine, or a salt thereof, and 1,3-bis(allylamino)propane, or a salt thereof in the crosslinked poly(allylamine) polymer can be calculated using the poly(allylamine) polymer wt fraction of crosslinked poly(allylamine) polymer and the known input mole ratios of 2-propen-1-ylamine and 1,3-bis(allylamino)propane used to produce the intermediate poly(allylamine) polymer:

For example, using 0.90 as the poly(allylamine) polymer wt fraction of crosslinked poly(allylamine) polymer (i.e. 10% of crosslinked poly(allylamine) polymer is ethylene, 90% is poly(allylamine) polymer) and 35.7 wt% residues of 2-propen-1-ylamine and 64.3 wt% residues of 1,3-bis(allylamino)propane calculated from the input mole ratios of 2-propen-1-ylamine and 1,3-bis(allylamino)propane used to make the poly(allylamine) polymer (60 mol% 2-propen-1-ylamine : 40 mol% 1,3-bis(allylamino)propane):

60 mol% 2-propen-1-ylamine can be converted into a wt% in the poly(allylamine) polymer as follows;

$$(57.1 \times 60) / 100 = 34.26;$$

$$(34.26 / 34.26 + 61.68) \times 100 = 35.7 \text{ wt\% residues of 2-propen-1-ylamine;}$$

40 mol% 1,3-bis(allylamino)propane can be converted into a wt% in the poly(allylamine) polymer as follows

$$(154.2 \times 40) / 100 = 61.68;$$

$$(61.68 / 34.26 + 61.68) \times 100 = 64.3 \text{ wt\% residues of 1,3-bis(allylamino)propane;}$$

The wt% of the residues of 2-propen-1-ylamine and 1,3-bis(allylamino)propane in the poly(allylamine) polymer can be converted to a wt% in the crosslinked poly(allylamine) polymer as follows;

The wt% of the residues of 2-propen-1-ylamine = 0.90 × 35.7 wt% = 32.1 wt% in the crosslinked poly(allylamine) polymer;

The wt% of the residues of 1,3-bis(allylamino)propane = 0.90 × 64.3 wt% = 57.9 wt% in the crosslinked poly(allylamine) polymer;

**Calculation of the mmoles /g of the residues of 2-propen-1-ylamine, or a salt thereof, and 1,3-bis(allylamino) propane, or a salt thereof, in the crosslinked poly(allylamine) polymer from the weight percent of the residues of 2-propen-1-ylamine, or a salt thereof, and 1,3-bis(allylamino)propane, or a salt thereof in the crosslinked poly(allylamine) polymer:**

From the wt% calculations above, in 1 g of crosslinked poly(allylamine) polymer there is: 0.32g residues of 2-propen-1-ylamine, 0.58g residues of 1,3-bis(allylamino)propane, 0.1g ethylene;

0.32 g residues of 2-propen-1-ylamine/57.1 g/mol = 5.6 mmol residues of 2- propen-1-ylamine;

0.58g residues of 1,3-bis(allylamino)propane/154.15 g/mol = 3.8 mmol residues of 1,3-bis(allylamino)propane;

$$0.10\text{g ethylene} / 28 \text{ g/mol} = 3.6 \text{ mmol ethylene.}$$

**Calculation of the mol% of the residues of 2-propen-1-ylamine, or a salt thereof, and 1,3-bis(allylamino)propane, or a salt thereof, in the crosslinked poly(allylamine) polymer from the weight percent of 2-propen-1-ylamine, or a salt thereof, and 1,3-bis(allylamino)propane, or a salt thereof in the crosslinked poly(allylamine) polymer:**

$$\text{mol\% residues of 2-propen-1-ylamine} = (5.6/(5.6+3.8+3.6) = 43\%;$$

$$\text{mol\% residues of 1,3-bis(allylamino)propane} = (3.8/(5.6+3.8+3.6) = 29\%;$$

$$\text{mol\% residues of 1,2-dichloroethane} = (3.6/(5.6+3.8+3.6) = 28\%.$$

Another worked example of how the mole ratio and mol% may be calculated is provide below. This example uses elemental analysis to determine the weight percent of the ethylene crosslinks. The weight percent of the ethylene crosslinks can then be used to calculate the mole ratio and mol% using the equations above.

**Calculation of mmol ethylene incorporated / g poly(allylamine) polymer using elemental analysis**

$$\frac{\left[\left(C_{wt\%-pp} \times \frac{N_{wt\%-pp}}{N_{wt\%-cpp}}\right) - C_{wt\%-cpp}\right] \times 1000}{2 \times 12.02 \times 100}$$

wherein:

C wt%-pp is the weight% of carbon in the poly(allylamine) polymer from elemental analysis;
C wt%-cpp is the weight% of carbon in the crosslinked poly(allylamine) polymer from elemental analysis;
N wt%-pp is the weight% of nitrogen in the poly(allylamine) polymer from elemental analysis;
N wt%-cpp is the weight% of nitrogen in the crosslinked poly(allylamine) polymer from elemental analysis;
1000 is conversion from moles to millimoles;
2 x 12.02 is ethylene carbon mass (weight of 2 moles of carbon);
100 is conversion from wt% to weight.

The C wt% and N wt% of the poly(allylamine) polymer and the crosslinked poly(allylamine) polymer may be determined using elemental analysis, for example the elemental analysis procedure described for determining the carbon to nitrogen weight ratio.

The term "monoallylamine" denotes an amino moiety having one allyl group.

The term "multiallylamine" denotes an amino moiety having two or more allyl groups and includes, for example, diallylamines, triallylamines, etc.

The term "nonabsorbable" as used herein takes its normal meaning in the art. Therefore, if something is nonabsorbable it is not absorbed during its passage through the human GI tract. This could be measured by any appropriate means. One option known to the skilled person would be to examine faeces to see if the nonabsorbable material is recovered after passing through the GI tract. As a practical matter, the amount of a nonabsorbable material recovered in this scenario will never be 100% of the material administered. For example, about 90 - 99% of the material might be recovered from the faeces. Another option known to the skilled person would be to look for the presence of the material in the lymph, blood, interstitial fluid, secretions from various organs (e.g., pancreas, liver, gut, etc.) or in the body of organs (e.g., liver, kidney, lungs, etc.) as oral administration of a nonabsorbable material would not result in an increase in the amount of that material in these matrices and tissues. Nonabsorbable compositions may be particulate compositions that are essentially insoluble in the human GI tract and have a particle size that is large enough to avoid passive or active absorption through the human GI tract. As an example, nonabsorbable compositions is meant to imply that the substance does not enter the lymph, blood, interstitial fluids or organs through the main entry points of the human GI tract, namely by paracellular entry between gut epithelial cells, by endocytic uptake through gut epithelial cells, or through entry via M cells comprising the gut epithelial antigen sampling and immune surveillance system (Jung, 2000), either through active or passive transport processes. There is a known size limit for a particulate to be absorbed in the human GI tract (Jung et al., European Journal of Pharmaceutics and Biopharmaceutics 50 (2000) 147-160; Jani et al., Internation. Journal of Pharmaceutics, 84 (1992) 245-252; and Jani et al., J. Pharm. Pharmacol. 1989, 41:809-812), so the skilled person would know that materials that, when in the GI tract, have a size of at least 1 micrometers would be nonabsorbable.

"Optional" or "optionally" means that the subsequently described event or circumstance may but need not occur, and that the description includes instances where the event or circumstance occurs and instances in which it does not. For example, "heterocyclyl group optionally substituted with an alkyl group" means that the alkyl may but need not be present, and the description includes embodiments in which the heterocyclyl group is substituted with an alkyl group and embodiments in which the heterocyclyl group is not substituted with alkyl.

The term "partially incorporated multiallylamine residue" as used herein denotes the residue of a multiallylamine that (i) has been incorporated into a poly(allylamine) polymer and (ii) possesses at least one pendant allyl group *(i.e.,* at least one allyl group that did not participate in a reaction to become a chain atom of and is merely "dangling" from the poly(allylamine) polymer backbone chain). For example, a partially incorporated diallylamine residue is the residue of a

diallylamine in which one of the two allyl groups of the incorporated diallylamine is a pendant allyl group of the polymer chain.

"Particle size" is measured by wet laser diffraction using Mie theory. Particles are dispersed in an appropriate solvent, such as water or methanol, and added to the sample chamber to achieve red channel obscuration of 10-20%. Sonication may be performed, and a dispersing agent, such as a surfactant (e.g. Tween-80), may be added in order to disrupt weak particle-particle interactions. The refractive index setting of the particles used for size distribution calculation is selected to minimize artifacts in the results and the R parameter value, determined by the laser diffraction software. The D(0.1), D(0.5), and D(0.9) values characterizing the particle size distribution by volume-basis are recorded.

For example, the particle size is measured by wet laser diffraction using Mie theory as follows. Particles are dispersed in methanol, and added to the sample chamber to achieve red channel obscuration of 10-20%. Sonication is performed. The refractive index setting of the particles used for size distribution calculation is selected to minimize artifacts in the results and the R parameter value, determined by the laser diffraction software. The D(0.1), D(0.5), and D(0.9) values characterizing the particle size distribution by volume-basis are recorded.

"Pharmaceutically acceptable" as used in connection with a carrier, diluent or excipient means a carrier, diluent or an excipient, respectively, that is useful in preparing a pharmaceutical composition that is generally safe, non-toxic and neither biologically nor otherwise undesirable for veterinary use and/or human pharmaceutical use.

The term "post polymerization crosslinking" is a term that describes a reaction to an already formed bead or gel, where more crosslinking is introduced to the already formed bead or gel to create a bead or gel that has an increased amount of crosslinking.

The term "post polymerization modification" is a term that describes a modification to an already formed bead or gel, where a reaction or a treatment introduces an additional functionality. This functionality can be linked either covalently or non-covalently to the already formed bead.

The term "semi-batch process" as used herein denotes refers to a variation of a batch process in which one or more reactants is added intermittently or continuously to the reactor.

"Simulated Gastric Fluid" or "SGF" Assay describes a test to determine total chloride binding capacity for a test polymer using a defined buffer that simulates the contents of gastric fluid as follows: Simulated gastric fluid (SGF) consists of 35 mM NaCl, 63 mM HCl, pH 1.2. To perform the assay, the free-amine polymer being tested is prepared at a concentration of 2.5 mg/ml (25 mg dry mass) in 10 mL of SGF buffer. The mixture is incubated at 37 °C overnight for ~12-16 hours with agitation on a rotisserie mixer. Unless another time period is otherwise stated, SGF binding data or binding capacities recited herein are determined in a time period of this duration. After incubation and mixing, the tubes containing the polymer are centrifuged for 2 minutes at 500-1000Xg to pellet the test samples. Approximately 750 microliters of supernatant are removed and filtered using an appropriate filter, for example a 0.45 micrometer pore-size syringe filter or an 800 microliter, 1 micrometer pore-size, 96-well, glass filter plate that has been fitted over a 96-well 2 mL collection plate. With the latter arrangement multiple samples tested in SGF buffer can be prepared for analysis, including a control tube containing blank buffer that is processed through all of the assay steps. With the samples arrayed in the filter plate and the collection plate fitted on the bottom, the unit is centrifuged at 1000Xg for 1 minute to filter the samples. In cases of small sample sets, a syringe filter may be used in lieu of the filter plate, to retrieve ~2-4 mL of filtrate into a 15 mL container. After filtration, the respective filtrates are diluted 4X with water and the chloride content of the filtrate is measured via ion chromatography (IC). The IC method (e.g. Dionex ICS-2100, Thermo Scientific) consists of an AS11 column and a 15 mM KOH mobile phase, an injection volume of 5 microliters, with a run time of 3 minutes, a washing/rinse volume of 1000 microliters, and flow rate of 1.25 mL /min. To determine the chloride bound to the polymer, the following calculation is completed:

$$\frac{(\text{Cl start - Cl eq}) \times 4}{2.5}.$$

Binding capacity expressed as mmol chloride/g polymer: where Cl start corresponds to the starting concentration of chloride in the SGF buffer, Cl eq corresponds to the equilibrium value of chloride in the diluted measured filtrates after exposure to the test polymer, 4 is the dilution factor and 2.5 is the polymer concentration in mg/ml.

"Simulated Small Intestine Inorganic Buffer" or "SIB" is a test to determine the chloride and phosphate binding capacity of free amine test polymers in a selective specific interfering buffer assay (SIB). The chloride and phosphate binding capacity of free amine test polymers was determined using the selective specific interfering buffer assay (SIB) as follows: The buffer used for the SIB assay comprises 36 mM NaCl, 20 mM $NaH_2PO_4$, 50 mM 2-(N-morpholino) ethanesulfonic acid (MES) buffered to pH 5.5. The SIB buffer contains concentrations of chloride, phosphate and pH that are present in the human duodenum and upper gastrointestinal tract (Stevens T, Conwell DL, Zuccaro G, Van Lente F, Khandwala F, Purich E, et al. Electrolyte composition of endoscopically collected duodenal drainage fluid after synthetic porcine secretin stimulation in healthy subjects. Gastrointestinal endoscopy. 2004;60(3):351-5, Fordtran J, Locklear T.

Ionic constituents and osmolality of gastric and small-intestinal fluids after eating. Digest Dis Sci. 1966;11(7):503-21) and is an effective measure of the selectivity of chloride binding compared to phosphate binding by a polymer. To perform the assay, the free amine polymer being tested is prepared at a concentration of 2.5 mg/ml (25 mg dry mass) in 10 mL of SIB buffer. The mixture is incubated at 37 °C for 1 hour with agitation on an orbital shaker at 200 to 300 rotations per minute. Unless another time period is otherwise stated, SIB binding data or binding capacities recited herein are determined in a time period of this duration. After incubation and mixing, the tubes containing the polymer are centrifuged for 2 minutes at 1000Xg to pellet the test samples. 750 microliter of supernatant is removed and filtered using an 800 microliter, 1 micrometer pore-size, 96-well, glass filter plate that has been fitted over a 96-well 2 mL collection plate; with this arrangement multiple samples tested in SIB buffer can be prepared for analysis, including the standard controls of free amine sevelamer, free amine bixalomer and a control tube containing blank buffer that is processed through all of the assay steps. With the samples arrayed in the filter plate and the collection plate fitted on the bottom, the unit is centrifuged at 1000Xg for 1 minute to filter the samples. In cases of small sample sets, a syringe filter (0.45 micrometer) may be used in lieu of the filter plate, to retrieve ~2-4 mL of filtrate into a 15 mL vial. After filtration into the collection plate, the respective filtrates are diluted before measuring for chloride or phosphate content. For the measurement of chloride and phosphate, the filtrates under analysis are diluted 4X with water. The chloride and phosphate content of the filtrate is measured via ion chromatography (IC). The IC method (e.g. Dionex ICS-2100, Thermo Scientific) consists of an AS24A column, a 45 mM KOH mobile phase, an injection volume of 5 microliters, with a run time of about 10 minutes, a washing/rinse volume of 1000 microliter, and flow rate of 0.3 mL/min. To determine the chloride bound to the polymer, the following calculation is completed:

$$\text{Binding capacity expressed as mmol chloride/g polymer} = \frac{(Cl_{start} - Cl_{final}) \times 4}{2.5}$$

where $Cl_{start}$ corresponds to the starting concentration of chloride in the SIB buffer, $Cl_{final}$ corresponds to the final value of chloride in the measured diluted filtrates after exposure to the test polymer, 4 is the dilution factor and 2.5 is the polymer concentration in mg/ml. To determine the phosphate bound to the polymer, the following calculation is completed:

$$\text{Binding capacity expressed as mmol phosphate/g polymer} = \frac{(P_{start} - P_{final}) \times 4}{2.5}$$

where $P_{start}$ corresponds to the starting concentration of phosphate in the SIB buffer, $P_{final}$ corresponds to the final value of phosphate in the measured diluted filtrates after exposure to the test polymer, 4 is the dilution factor and 2.5 is the polymer concentration in mg/ml.

The term "$sp^2$ allyl carbon" as used herein denotes each of the two $sp^2$ hybridized carbon atoms comprised by an allyl moiety.

The term "substituted hydrocarbyl," "substituted alkyl," "substituted alkenyl," "substituted aryl," "substituted heterocyclo," or "substituted heteroaryl" as used herein denotes hydrocarbyl, alkyl, alkenyl, aryl, heterocyclo, or heteroaryl moieties which are substituted with at least one atom other than carbon and hydrogen, including moieties in which a carbon chain atom is substituted with a hetero atom such as nitrogen, oxygen, silicon, phosphorous, boron, sulfur, or a halogen atom. These substituents include halogen, heterocyclo, alkoxy, alkenoxy, alkynoxy, aryloxy, hydroxy, keto, acyl, acyloxy, nitro, amino, amido, nitro, cyano, thiol, ketals, acetals, esters and ethers.

"Swelling Ratio" or simply "Swelling" describes the amount of water absorbed by a given amount of polymer divided by the weight of the polymer aliquot. The Swelling Ratio is expressed as: swelling = (g swollen polymer - g dry polymer)/g dry polymer. The method used to determine the Swelling Ratio for any given polymer comprised the following:

a. 50-100 mg of dry (less than 5 weight % water content) polymer is placed into an 11 mL sealable test tube (with screw cap) of known weight (weight of tube = Weight A).

b. Deionized water (10mL) is added to the tube containing the polymer. The tube is sealed and tumbled for 16 hours (overnight) at room temperature. After incubation, the tube is centrifuged at 3000xg for 3 minutes and the supernatant is carefully removed by vacuum suction. For polymers that form a very loose sediment, another step of centrifugation is performed.

c. After step (b), the weight of swollen polymer plus tube (Weight B) is recorded.

d. Freeze at -40 ºC for 30 minutes. Lyophilize for 48 h. Weigh dried polymer and test tube (recorded as Weight C).

e. Calculate g water absorbed per g of polymer, defined as: [( Weight B-Weight A)-( Weight C- Weight A)]/( Weight C- Weight A).

A "target ion" is an ion to which the polymer binds, and usually refers to the major ions bound by the polymer, or the ions whose binding to the polymer is thought to produce the therapeutic effect of the polymer (e.g. proton and chloride binding which leads to net removal of HCl).

The term "theoretical capacity" represents the calculated, expected binding of hydrochloric acid in an "SGF" assay, expressed in mmol/g. The theoretical capacity is based on the assumption that 100 % of the amines from the monomer(s) and crosslinker(s) are incorporated in the crosslinked polymer based on their respective feed ratios. Theoretical capacity is thus equal to the concentration of amine functionalities in the polymer (mmol/g). The theoretical capacity assumes that each amine is available to bind the respective anions and cations and is not adjusted for the type of amine formed (e.g. it does not subtract capacity of quaternary amines that are not available to bind proton).

"Therapeutically effective amount" means the amount of a proton-binding crosslinked amine polymer that, when administered to a patient for treating a disease, is sufficient to effect such treatment for the disease. The amount constituting a "therapeutically effective amount" will vary depending on the polymer, the severity of the disease and the age, weight, etc., of the mammal to be treated.

"Treating" or "treatment" of a disease includes (i) inhibiting the disease, *i.e.,* arresting or reducing the development of the disease or its clinical symptoms; or (ii) relieving the disease, *i.e.,* causing regression of the disease or its clinical symptoms. Inhibiting the disease, for example, would include prophylaxis.

The term "triallylamine" denotes an amino moiety having three allyl groups.

The term "vinyl" denotes a moiety having the structural formula $R_xH_yC=CH-*$, where * denotes the point of attachment of the moiety to the remainder of the molecule, and wherein X and Y are independently 0, 1 or 2, such that X+Y=2, and R is hydrocarbyl or substituted hydrocarbyl. In some embodiments, the point of attachment * is to a heteroatom in the remainder of the molecule, such as nitrogen.

The term "water equivalents" means the number of moles of water.

The term "weight percent crosslinker" represents the calculated percentage, by mass, of a polymer sample that is derived from the crosslinker. Weight percent crosslinker is calculated using the feed ratio of the polymerization, and assumes full conversion of the monomer and crosslinker(s). The mass attributed to the crosslinker is equal to the expected increase of molecular weight in the infinite polymer network after reaction (e.g. 1,3-dichloropropane is 113 amu, but only 42 amu are added to a polymer network after crosslinking with DCP because the chlorine atoms, as leaving groups, are not incorporated into the polymer network).

Protocol for Other Assays and Determinations Referenced Herein

**Cation IC Extraction Procedure:** To a tightly closing vial with capacity between 15 and 50 mL, poly(allylamine) polymer (~1.0 g) is added 10 mL of 1.2 M HCl. The vial is shaken at room temperature for at least 24 hours. The supernatant is filtered through a 0.45 micrometer syringe filter followed by a neutralization column before being analyzed by ion chromatography (IC). The IC (e.g. Dionex ICS-5000, Thermo Scientific) method consists of a CG19 guard column and CS19 analytical column, a methane sulfonic acid (MSA) eluent generator, an injection volume of 25 microliters, with a run time of about 40 minutes and flow rate of 0.3 mL/min. The MSA concentration is 2 mM for 10 minutes, followed by a ramp to 70 mM MSA from 10 to 28 minutes, a hold at 70 mM MSA for 3 minutes, and re-equilibration at 2mM MSA for 9 minutes. This procedure is used to measure allylamine content of 0.25 ppm or higher. If samples contain less than 0.25 ppm then the extraction supernatant are analyzed by LCMS as described in the following procedure.

To a tightly closing vial with capacity between 15 and 50 mL, polyallylamine polymer (~1.0 g) is added 10 mL of 1.2 M HCl. The vial is shaken at 200 RPM at room temperature for at least 24 hours. The supernatant is filtered through a 0.45 micrometer syringe filter. The sample is diluted two-fold with internal standard (IS), consisting of 10 micrograms/mL diethylamine in 0.1% heptaflurobutyric acid (HFBA) in water. The HPLC method (e.g. Agilent 1260 HPLC) consists of a Acclaim 120 C18 2.1 x 50 mm column with 5 micrometer particle size, an injection volume of 5 microliters, mobile phase consisting of A) 0.1% HFBA in water and B) 0.1% HFBA in acetonitrile, with a gradient of 0% B for 3 minutes, ramp to 100% B from 3-3.5 minutes, hold at 100% B from 3.5-6 minutes, and equilibration at 0% B until 10 minutes, with a flow rate of 0.4 mL/min. The mass spectrometer (MS) method (e.g. API 4000 triple quadrupole tandem MS) with electrospray ionization with source temperature approximately 500°C and voltage of 5000 V. in positive ion, multiple reaction monitoring mode with allylamine Q1 and Q3 masses approximately 58.7 and 58.1 amu, respectively and IS Q1 and Q3 masses approximately 74.7 and 73.1 amu. Gas source pressure, collision energy, declustering potential, entrance potential and exit potential are optimized for the instrumentation employed.

**Determination of percent $sp^2$ allyl carbons by quantitative $^{13}C$ solid state magic angle spinning (MAS) NMR:** The quantitative $^{13}C$ solid state magic angle spinning (MAS) NMR measurement is performed on a Bruker AVANCE III 800 MHz (18.8T) standard bore spectrometer, operating at 800.25 MHz and 201.24 MHz for $^1H$ and $^{13}C$, respectively, with a 4 mm zirconia rotor system at a spinning frequency of 16 kHz. A single pulse experiment is performed with a 30 degree excitation pulse of 1.2 $\mu$s using an 8 s relaxation delay, which is optimized for quantitative analysis, an 8.6 ms acquisition time, and an acumination of about 20,000 scans. 100 kHz proton decoupling was applied during $^{13}C$ data

acquisition. The chemical shift is referenced to the TMS standard. Integration of the allyl carbon peaks between 110 - 150 ppm and alkyl carbon peaks between 0 - 80 ppm is used to quantitate the percent $sp^2$ allyl carbons of the poly(allylamine) polymer using the following formula:

$$\%\ \mathrm{sp2}\ allyl\ carbons = \frac{peak\ integration\ (110 - 150\ ppm)}{peak\ integration\ (0 - 80\ ppm) + peak\ integration\ (110 - 150\ ppm)} \times 100$$

When introducing elements of the present invention or the preferred embodiments(s) thereof, the articles "a", "an", "the" and "said" are intended to mean that there are one or more of the elements. The terms "comprising", "including" and "having" are intended to be inclusive and not exclusive (*i.e.,* there may be other elements in addition to the recited elements).

**Determination of percent $sp^2$ allyl carbons by quantitative $^{13}C$ solid state cross-polarization magic angle spinning (CPMAS) NMR:**

The quantitative $^{13}C$ solid state cross-polarization magic angle spinning (CPMAS) NMR measurement is performed on poly(allylamine) polymer samples using a Redstone 360 MHz spectrometer, operating at 363.331 MHz and 91.369 MHz for $^1H$ and $^{13}C$, respectively, with a 7 mm zirconium probe at a spinning frequency of 7 kHz. The cross-polarization experiment is performed with a 90 degree $^1H$ excitation pulse of 5 $\mu$s, 2.5 ms contact time, and 3 s recycle delay, which was calibrated for poly(allylamine) polymer analysis based on quantitative single-pulse spectra. Proton decoupling of 12V and 18V and line broadening of 35 Hz were used, and about 3500 spectral acquisitions were accumulated. Integration of the allyl carbon peaks between 110 - 150 ppm and alkyl carbon peaks between 0 - 80 ppm is used to quantitate the percent $sp^2$ allyl carbons of the poly(allylamine) polymer using the following formula:

$$\%\ \mathrm{sp2}\ allyl\ carbons = \frac{peak\ integration\ (110 - 150\ ppm)}{peak\ integration\ (0 - 80\ ppm) + peak\ integration\ (110 - 150\ ppm)}\ x100.$$

**Determination of the carbon to nitrogen weight ratio of the crosslinked poly(allylamine) polymer by elemental analysis:**

Elemental analysis is a standard method for measuring carbon, hydrogen and nitrogen content of organic substances that belongs to the skilled person's common general knowledge. The skilled person would appreciate that all elemental analysis measurement methodologies yield the same result within the appropriate limit of measurement accuracy. The elemental analysis may be performed by any elemental analyzer suitable for measurement of organic carbon, hydrogen, and nitrogen. The following elemental analysis method is provided as an example of how the elemental analysis may be performed.

Carbon, Hydrogen, Nitrogen are determined using a Perkin-Elmer 2400 Elemental Analyzer. This analyzer uses combustion to convert the sample elements to simple gases, i.e., $CO_2$, $H_2O$, and $N_2$. Upon entering the analyzer, the sample is combusted in a pure oxygen environment. The product gases are separated under steady state conditions, and measured as a function of thermal conductivity. The instrument is calibrated prior to sample analysis with a National Institute of Standards and Technology (NIST) traceable organic standard. For example, the standard may have a nitrogen content in the range of about 11-45 wt%. System suitability is confirmed by analyzing an NIST traceable organic standard. The standard must check to within $\pm$0.1% of its theoretical value for all three of the elements carbon, hydrogen and nitrogen. Crosslinked poly(allylamine) polymer samples for analysis are typically dried by at 60°C in an oven under vacuum prior to analysis to remove moisture.

The carbon to nitrogen weight ratio of the poly(allylamine) polymer used to make the crosslinked poly(allylamine) polymer may also be determined by elemental analysis. The procedure used to determine the carbon to nitrogen weight ratio of the poly(allylamine) polymer can be as described above.

**GC-FID Extraction Procedure:** To a tightly closing vial with capacity between 10 and 20 mL, containing poly(allylamine) polymer (~0.1g) is added 5 mL of acetonitrile. To a second tightly closing vial with capacity between 10 and 20 mL, containing polyallylamine polymer (~1.5 g) is added 5 mL of methanol. The vials are closed and shaken at room temperature at 200 RPM for 24 hours. The supernatant is filtered through a 0.45 micrometer syringe filter before being

analyzed by gas chromatography with flame ionization detection (GC-FID). The GC (e.g. Agilent 6890) method consists of an approximately 4 meter DB-1 column, 0.32 mm inner diameter coupled in series with a 30 meter DB-wax column, 0.32 mm inner diameter, with constant helium flow of 2.5 mL/min. Injection of 2 microliters is performed into an inlet at 200°C, with a split ratio of 1:10. The oven gradient program consists of a 5 minute hold at 40°C, followed by a 10°C/min ramp to 180°C, followed by a 20°C/min ramp to 240°C and a hold of 3 minutes. FID acquisition is performed at a temperature of 300°C.

**Heated Stability Assay (Stability Assay 2):** Into two separate tightly closing vials with capacity between 15 and 50 mL, is massed poly(allylamine) polymer (1.0 g each). The sample from one vial is extracted directly to determine the starting allylamine content according to the Cation IC Extraction Procedure. The remaining sample is sealed tightly, and then put into a convection oven set to 60°C. After 72 hours, the samples are removed from the oven, cooled to 4°C, and then extracted in the same way described by Cation IC Extraction Procedure.

**Impurity Analysis at Release Assay:** Into three separate tightly closing vials with capacity between 15 and 50 mL, is massed dried poly(allylamine) polymer (1.0 g in the first vial, 1.5 g in the second vial, and 0.1 g into the third vial). The sample from one vial containing 1.0 g poly(allylamine) polymer is extracted to determine the starting allylamine content according to the Cation IC Extraction Procedure. The remaining two samples are extracted to determine the starting allyl methyl ether, and allyl alcohol content according to the GC-FID extraction procedure.

**In-Air Stability Assay (Stability Assay 1):** To a 35 mL HDPE bottle is added 4.5 g of poly(allylamine) polymer. The bottle is closed, and then stored in a 60% humidity controlled chamber held at 25°C for 7 days. The bottle is removed from the chamber and the polymer sample extracted to determine allylamine content according to the Cation IC Extraction Procedure.

**Stability assessment when packaged in a MylarFoil sachet assay (Stability Assay 3):** To a 3-side sealed approximately 2.5" x 3" sachet composed of a laminate containing a metal foil layer, is added about 3 g of poly(allylamine) polymer and then the sachet is heat sealed. Multiple sachets of poly(allylamine) polymer are prepared and then placed into individual test chambers that were controlled at 25°C and 60% relative humidity, and 40°C and 75% relative humidity for up to 6 months. At the desired time, sachets are removed from each test chamber and the polymer samples are extracted to determine allylamine content according to the Cation IC Extraction Procedure.

## DESCRIPTION OF FIGURES

Figure 1A: Allylamine (AA) concentration (ppm) present when veverimer is packaged in packet materials A, B and C at 25°C/60% RH over 6 weeks. Unit dosage forms A, B and C were stored at 25°C/60% RH for 6 weeks and the allylamine concentration was measured at T0 (initial) and at weeks 1, 2, 4, and 6. On Figure 1A, unit dosage form A is depicted by squares (e.g. the top line at week 6), unit dosage form B is depicted by circles (e.g. the bottom line at week 6), and unit dosage form C is depicted by crosses (x) (e.g. the middle line at week 6), and. Unit dosage form A shows an increasing concentration of allylamine over the 6 week period not seen with unit dosage forms B and C.

Figure 1B: Allylamine (AA) concentration (ppm) present when veverimer is packaged in unit dosage forms A, B and C at 40°C/75% RH over 6 weeks. Unit dosage forms A, B and C were stored at 40°C/75% RH for 6 weeks and the allylamine concentration was measured at T0 (initial) and at weeks 1, 2, 4, and 6. On Figure 1B, unit dosage form A is depicted by squares (e.g. the top line at week 6), B is depicted by circles (e.g. the middle line at week 6), and C is depicted by crosses (x) (e.g. the bottom line at week 6). Unit dosage form A shows an increasing concentration of allylamine over the 6 week period under the conditions not seen with unit dosage forms B and C.

Figure 2: Allylamine (AA) concentration (ppm) in 1.5, 3.0 and 4.5 g unit dosage forms at 25°C/60% RH over 3 months. The 1.5, 3.0 and 4.5 g unit dosage forms were stored at 25°C/60% RH for 3 months and the allylamine concentration was measured at T0 (initial) and monthly thereafter. On Figure 2, 1.5 g is depicted by circles (e.g. the top line at month 3), 3.0 g is depicted by squares (e.g. the middle line at month 3), and 4.5 g is depicted by triangles (e.g. the bottom line at month 3).The 1.5, 3.0 and 4.5 g unit dosage forms show that the concentration of AA increases over time.

Figure 3: Allylamine (AA) concentration (ppm) in 1.5, 3.0 and 4.5 g unit dosage forms at 40°C/75% RH over 3 months. The 1.5, 3.0 and 4.5 g unit dosage forms were stored at 40°C/75% RH for 3 months and the allylamine concentration was measured at T0 (initial) and monthly thereafter. On Figure 3, 1.5 g is depicted by circles (e.g. the top line at month 3), 3.0 g is depicted by squares (e.g. the middle line at month 3), and 4.5 g is depicted by triangles (e.g. the bottom line at month 3). The 1.5, 3.0 and 4.5 g unit dosage forms show that the concentration of AA increases over time.

Figure 4: Allylamine (AA) concentration (ppm) in 1.5 g unit dosage forms with an oxygen scavenger at 25°C/60% RH and 40°C/75% RH over 6 months. The allylamine concentration in the unit dosage form was measured at T0 and at various intervals thereafter. On Figure 4, the 1.5 g unit dosage form at 25°C/60% RH is depicted by crosses (X) (e.g. the top line at month 2), and the 1.5 g unit dosage form at 40°C/75% RH is depicted by triangles (e.g. the bottom line at month 2). The unit dosage forms show that the concentration of AA is approximately constant over 6 months.

Figure 5: Allylamine (AA) concentration (ppm) in 1.5 g unit dosage forms with environments of air, 8% oxygen and 92% nitrogen, and 99+% nitrogen 25°C/60% RH over 6 months. The allylamine concentration in the unit dosage form was measured at T0 and at various intervals thereafter. On Figure 5, the 1.5 g unit dosage form in air is depicted by crosses (X) (e.g. the top line at month 6), the 1.5 g unit dosage form in 8% oxygen and 92% nitrogen is depicted by triangles (e.g. the middle line at month 6), and the 1.5 g unit dosage form in 99+% nitrogen is depicted by circles (e.g. the bottom line at month 6). The unit dosage forms show that the concentration of AA is approximately constant over 6 months.

Figure 6: Allylamine (AA) concentration (ppm) in 1.5 g unit dosage forms with environments of air, 8% oxygen and 92% nitrogen, and 99+% nitrogen 40°C/75% RH over 6 months. The allylamine concentration in the unit dosage form was measured at T0 and at various intervals thereafter. On Figure 6, the 1.5 g unit dosage form in air is depicted by crosses (X) (e.g. the top line at month 6), the 1.5 g unit dosage form in 8% oxygen and 92% nitrogen is depicted by triangles (e.g. the middle line at month 6), and the 1.5 g unit dosage form in 99+% nitrogen is depicted by circles (e.g. the bottom line at month 6).The unit dosage forms show that the concentration of AA is approximately constant over 6 months.

## EMBODIMENTS

The present disclosure includes the observation that during the synthesis of a crosslinked poly(allylamine) polymer, such as veverimer, unincorporated 2-propen-1-ylamine monomer and/or partially incorporated 1,3-bis(allylamino)propane monomer residues, can be a source of allylamine impurities (*i.e.,* allylamine and derivatives thereof such as allylalkylethers and allylalcohols) in the poly(allylamine) product. Those monomers may be present as a salt. Without wishing to be bound by theory, it is thought that the residual unincorporated 2-propen-1-ylamine monomer and/or partially incorporated 1,3-bis(allylamino)propane monomer residues present in the crosslinked poly(allylamine) polymer, such as veverimer, may react with oxygen after the polymer has been made and thus produce allylamine impurities (e.g. $H_2C=CHCH_2NH_2$). For example, it was found that the veverimer polymer referred to as unique ID 019070-A3 FA in Table S-1 of Exemplary Synthesis A of WO2019/236636 A1 produced $H_2C=CHCH_2NH_2$ when exposed to oxygen. High levels of impurities, such as $H_2C=CHCH_2NH_2$, are undesirable in the final product to be administered to patients, e.g. due to potential safety concerns.

Several ways to reduce the levels of allylamine impurities such as $H_2C=CHCH_2NH_2$ in a crosslinked poly(allylamine) polymer of the present invention are disclosed herein.

One approach disclosed herein is to produce a crosslinked poly(allylamine) polymer, such as veverimer, that contains less unincorporated 2-propen-1-ylamine monomer, or a salt thereof, and/or partially incorporated 1,3-bis(allylamino)propane monomer residues, or a salt thereof. Reducing the amount of such unincorporated and/or partially incorporated monomers can reduce the amount of allylamine impurities present in the final product. Therefore, disclosed herein are methods for making a crosslinked poly(allylamine) polymer of the present invention with reduced levels of such unincorporated and/or partially incorporated monomers, for example as measured by reference to the levels of sp2 carbons present in the product. Also disclosed herein are crosslinked poly(allylamine) polymers of the present invention with reduced levels of such unincorporated and/or partially incorporated monomers, for example as measured by reference to the levels of sp2 carbons present in the product. Such products are shown to have desirable properties, e.g. the levels of allylamine impurities (e.g. $H_2C=CHCH_2NH_2$) in such products are reduced.

Another approach disclosed herein is to package the crosslinked poly(allylamine) polymer of the present invention in such a way that its exposure to oxygen is reduced. The approach of using packaging to mitigate concerns regarding impurities, such as the levels of allylamine impurities (e.g. $H_2C=CHCH_2NH_2$), can be used in combination with a crosslinked poly(allylamine) polymer of the present invention.

In accordance with one aspect of the present disclosure, the crosslinked poly(allylamine) polymers of the present invention may be used therapeutically. In one embodiment, the crosslinked poly(allylamine) polymer may be used to bind HCl from the gastrointestinal tract of an animal including, for example, humans, upon administration of a therapeutically effective amount (*i.e.,* an effective dose) of the crosslinked poly(allylamine) polymer to achieve a therapeutic or prophylactic benefit.

Methods of treatment and medical uses of the crosslinked poly(allylamine) polymers of the present invention are described in WO2014/197725 A1, WO2016/094685 A1, WO2017/193050 A1, WO2017/193064 A1, WO2017/193024 A1, WO2019/090176 A1, WO2019/090177 A1, WO2019/236639 A1, WO2019/236636 A1 and WO2019/236124 A1.

In accordance with one aspect of the present disclosure, the crosslinked poly(allylamine) polymers of the present invention are for use in any of the methods of treatment or medical uses described in any of WO2014/197725 A1, WO2016/094685 A1, WO2017/193050 A1, WO2017/193064 A1, WO2017/193024 A1, WO2019/090176 A1, WO2019/090177 A1, WO2019/236639 A1, WO2019/236636 A1 and WO2019/236124 A1.

In accordance with one aspect of the present disclosure, a crosslinked poly(allylamine) polymer may be formed in steps: a (first) concurrent polymerization and crosslinking step (sometimes referred to as the "first crosslinking step" or, more simply, the "first step") and, optionally, a (second) post-polymerization crosslinking step (sometimes referred to as the "second crosslinking step" or, more simply, the "second step"). In the first step, the crosslinking is preferably capacity-sparing, *i.e.*, free amine sparing, crosslinking from carbon to carbon. In the second step, the crosslinking is amine-consuming and is directed towards tuning for selectivity for the target species. Based on the desired high capacity, the C-N weight ratio is preferably optimized to maximize amine functionalities for target species binding, while still maintaining a spherical polymer particle of controlled particle size to ensure non absorption and acceptable mouth feel that is stable under GI conditions. It should be noted that the terms "first" and "second" are merely used to designate relative order as between these two steps, and that other steps may be contemplated as part of the process, either before, after, or between the "first step" and the "second step."

In the first step, 2-propen-1-ylamine, or a salt thereof, and 1,3-bis(allylamino)propane, or a salt thereof, are concurrently polymerized and crosslinked in a heterogeneous reaction mixture comprising 2-propen-1-ylamine, or a salt thereof, the 1,3-bis(allylamino)propane, or a salt thereof, a radical initiator, water and an organic solvent to form a polymer network that is crosslinked through a carbon backbone. Advantageously, each crosslinking reaction in this step forms a carbon-carbon bond (as opposed to substitution reactions in which a carbon-heteroatom bond is formed during crosslinking), and the amine functionalities of the 2-propen-1-ylamine, or a salt thereof, and the 1,3-bis(allylamino)propane, or a salt thereof, do not undergo crosslinking reactions and are preserved in the final polymer (*i.e.,* primary amines of the 2-propen-1-ylamine, or a salt thereof, and the 1,3-bis(allylamino)propane, or a salt thereof, remain primary, secondary amines remain secondary, and tertiary amines remain tertiary). The resulting poly(allylamine) polymer may then be further crosslinked in a second step with 1,2-dichloroethane.

As previously noted, unincorporated 2-propen-1-ylamine, or a salt thereof, (*i.e.,* 2-propen-1-ylamine, or a salt thereof, that is not covalently incorporated into the polymer) and partially incorporated 1,3-bis(allylamino)propane, or a salt thereof, residues can be a source of allylamine impurities (*i.e.,* allylamine and derivatives thereof such as allylalkylethers and allylalcohols) in the poly(allylamine) product. In accordance with one embodiment of the present disclosure, process parameters may be controlled to limit the amount of allyl impurities (i) released by the crosslinked poly(allylamine) polymer in the as-manufactured state (*i.e.,* upon completion of the second step, also sometimes referred to herein as "at-release")), and/or (ii) released by the crosslinked poly(allylamine) polymer as a function of storage and time.

In general, it is preferred that the crosslinked poly(allylamine) polymer contain less than 20 ppm allylamine in the as-manufactured state. For example, in one embodiment the crosslinked poly(allylamine) polymer contains less than 15 ppm allylamine in the as-manufactured state. By way of further example, in one such embodiment the crosslinked poly(allylamine) polymer contains less than 12.5 ppm allylamine in the as-manufactured state. By way of further example, in one such embodiment the crosslinked poly(allylamine) polymer contains less than 10 ppm allylamine in the as-manufactured state. By way of further example, in one such embodiment the crosslinked poly(allylamine) polymer contains less than 7.5 ppm allylamine in the as-manufactured state. By way of further example, in one such embodiment the crosslinked poly(allylamine) polymer contains less than 5 ppm allylamine in the as-manufactured state. By way of further example, in one such embodiment the crosslinked poly(allylamine) polymer contains less than 4 ppm allylamine in the as-manufactured state. By way of further example, in one such embodiment the crosslinked poly(allylamine) polymer contains less than 3 ppm allylamine in the as-manufactured state. By way of further example, in one such embodiment the crosslinked poly(allylamine) polymer contains less than 2 ppm allylamine in the as-manufactured state. By way of further example, in one such embodiment the crosslinked poly(allylamine) polymer contains less than 1 ppm allylamine in the as-manufactured state. By way of further example, in one such embodiment the crosslinked poly(allylamine) polymer contains less than 500 ppb allylamine in the as-manufactured state. By way of further example, in one such embodiment the crosslinked poly(allylamine) polymer contains less than 100 ppb allylamine in the as-manufactured state. By way of further example, in one such embodiment the crosslinked poly(allylamine) polymer contains less than 50 ppb allylamine in the as-manufactured state. By way of further example, in one such embodiment the crosslinked poly(allylamine) polymer contains less than 1 ppb allylamine in the as-manufactured state. By way of further example, in one such embodiment the amount of allylamine, if any, in the crosslinked poly(allylamine) polymer is less than the detection limit for allylamine in the as-manufactured state. In each such exemplary embodiment recited in this paragraph, the allylamine content may be determined by the Impurity Analysis at Release Assay followed by the Cation IC Extraction Procedure.

In one embodiment, the crosslinked poly(allylamine) polymer contains less than 20ppm allylamine upon storage in a sealed enclosure for 3 months at 25°C after manufacture. In one embodiment, the crosslinked poly(allylamine) polymer contains less than 20 ppm allylamine upon storage in a sealed enclosure for 6 months at 25°C after manufacture. In one embodiment, the crosslinked poly(allylamine) polymer contains less than 20 ppm allylamine upon storage in a sealed enclosure for 9 months at 25°C after manufacture. In one embodiment, the crosslinked poly(allylamine) polymer contains less than 20 ppm allylamine upon storage in a sealed enclosure for 12 months at 25°C after manufacture. In one embodiment, the allylamine content of the crosslinked poly(allylamine) polymer increases less than 20 ppm allylamine upon storage in a sealed enclosure for 3 months at 25°C. In one embodiment, the allylamine content of the crosslinked poly(allylamine) polymer increases less than 20 ppm allylamine upon storage in a sealed enclosure for 6 months at 25°C. In one embodiment, the allylamine content of the crosslinked poly(allylamine) polymer increases less than 20 ppm allylamine upon storage in a sealed enclosure for 9 months at 25°C. In one embodiment, the allylamine content of the crosslinked poly(allylamine) polymer increases less than 20 ppm allylamine upon storage in a sealed enclosure for 12

months at 25°C. In each such exemplary embodiment recited in this paragraph, the allylamine content may be determined by the Cation IC Extraction Procedure.

To facilitate the concurrent polymerization and crosslinking reaction, the 2-propen-1-ylamine, or a salt thereof, and the 1,3-bis(allylamino)propane, or a salt thereof, are preferably protonated in the reaction mixture. In one embodiment, therefore, the 2-propen-1-ylamine, or a salt thereof, and/or the 1,3-bis(allylamino)propane, or a salt thereof, are introduced to the reaction mixture as their respective acid salts (*e.g.,* hydrochloric acid, phosphoric acid, sulfuric acid or hydrobromic acid salt form). Alternatively, the 2-propen-1-ylamine, or a salt thereof, and/or the 1,3-bis(allylamino) propane, or a salt thereof, may be introduced to the reaction mixture in their free amine form(s) and acid can be separately added to the reaction mixture. For example, the acid may be sulfuric acid, phosphoric acid or hydrochloric acid (HCl). By way of further example, the acid is HCl. In either embodiment, the reaction mixture comprises sufficient acid to maintain the 2-propen-1-ylamine, or a salt thereof, and the 1,3-bis(allylamino)propane, or a salt thereof, in the aqueous phase. Typically, the reaction mixture will contain at least 0.5 equivalents of acid per allylamine equivalent in the reaction mixture (independent of whether the acid was introduced as the acid salt of the 2-propen-1-ylamine, or a salt thereof, and/or the 1,3-bis(allylamino)propane, or a salt thereof, or whether it was separately added to the reaction mixture). In certain embodiments, the reaction mixture will contain at least 0.75 equivalents of acid per allylamine equivalent in the reaction mixture. In certain embodiments, the reaction mixture will contain at least 1 equivalent of acid per allylamine equivalent in the reaction mixture.

2-Propen-1-ylamine and 1,3-Bis(allylamino)propane are listed in Table C. As illustrated, 2-Propen-1-ylamine and 1,3-Bis(allylamino)propane are in the HCl salt form. As previously noted, each of 2-Propen-1-ylamine and 1,3-Bis(allylamino)propane may be introduced to the reaction mixture in a salt form (*e.g.,* as a hydrochloric acid salt, sulfuric acid salt, phosphoric acid salt, hydrobromic acid salt or a combination thereof), in the free base form, or a combination thereof.

Table C

| Abbreviation | Common name | IUPAC name | | MW (g/mol) |
|---|---|---|---|---|
| DAPDA | Diallylpropyldiamine | 1,3-Bis(allylamino)propa ne | 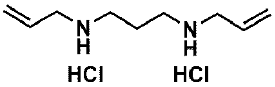 | 227.17 |
| AAH | Allylamine | 2-Propen-1-ylamine | 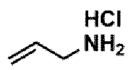 | 93.5 |

The concurrent polymerization and crosslinking step reaction mixture comprises a radical polymerization initiator in addition to the 2-propen-1-ylamine, or a salt thereof, and the 1,3-bis(allylamino)propane, or a salt thereof,. The initiator may be selected from any of a wide range of initiators, including cationic and radical polymerization initiators. Exemplary polymerization initiators for the concurrent polymerization and crosslinking step reaction include peroxy and azo free-radical initiators, such as azodiisobutyronitrile, azodiisovaleronitrile, dimethylazodiisobutyrate, 2,2'azo bis(isobutyronitrile), 2,2'-azobis(N,N'-dimethyl-eneisobutyramidine)dihydrochloride, 2,2'-azobis(2-methylpropionamidine)dihydrochloride, 2,2'-azobis(2-amidinopropane)dihydrochloride, 2,2'-azobis(N,N'-dimethyleneisobutyramidine), 1,1'-azo bis(I-cyclohexanecarbo-nitrile), 4,4'-azobis(4-cyanopentanoic acid), 2,2'-azobis(isobutyramide)dihydrate, 2,2'-azobis(2-methylpropane), 2,2'-azobis(2-methylbutyronitrile), VAZO 67, cyanopentanoic acid, the peroxypivalates, dodecylbenzene peroxide, benzoyl peroxide, di-t-butyl hydroperoxide, t-butyl peracetate, acetyl peroxide, dicumyl peroxide, cumylhydroperoxide, dimethyl bis(butylperoxy)hexane. For example, the radical polymerization initiator is V-50 (2,2'-azobis(2-methylpropionamidine)dihydrochloride).

Experience to-date has shown that the amount of initiator relative to the amount of 2-propen-1-ylamine, or a salt thereof, and 1,3-bis(allylamino)propane, or a salt thereof, in the reaction mixture has an influence on the characteristics of the resulting polymer. For example, the amount of partially incorporated 1,3-bis(allylamino)propane, or a salt thereof, tends to increase as the ratio of the number of allyl equivalents to the number of initiator equivalents in the reaction mixture increases. Typically, therefore, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be in the range of about 6:1 to about 70:1, respectively. For example, in one such embodiment, the ratio of the number of allyl equivalents possessed by the 2-propen-1-ylamine, or a salt thereof, and 1,3-bis(allylamino) propane, or a salt thereof, in combination, to the number of initiator equivalents introduced to the reaction mixture will be about 7:1 to about 60:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be about 8:1 to about 50:1,

respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be about 10:1 to about 45:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be about 15:1 to about 40:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be about 17.5:1 to about 35:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be about 20:1 to about 30:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be about 22.5:1 to about 30:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of initiator equivalents introduced to the reaction mixture will be about 25:1 to about 27.5:1, respectively.

Experience to-date has also shown that the combined amount of 2-propen-1-ylamine, or a salt thereof, and 1,3-bis(allylamino)propane, or a salt thereof, relative to the amount of water in the reaction mixture has an influence on the characteristics of the resulting polymer. Typically, therefore, the weight ratio of the combined amount of 2-propen-1-ylamine, or a salt thereof, and 1,3-bis(allylamino)propane, or a salt thereof, to the amount of water in the reaction mixture will be in the range of about 0.01 to about 3, respectively. For example, in one embodiment the weight ratio of the combined amount of 2-propen-1-ylamine, or a salt thereof, and 1,3-bis(allylamino)propane, or a salt thereof, to the amount of water in the reaction mixture will be in the range of about 0.05 to about 2.75, respectively. By way of further example, in one such embodiment the weight ratio of the combined amount of 2-propen-1-ylamine, or a salt thereof, and 1,3-bis(allylamino) propane, or a salt thereof, to the amount of water in the reaction mixture will be in the range of about 0.07 to about 2.5, respectively. By way of further example, in one such embodiment the weight ratio of the combined amount of 2-propen-1-ylamine, or a salt thereof, and 1,3-bis(allylamino)propane, or a salt thereof, to the amount of water in the reaction mixture will be in the range of about 0.1 to about 2.25, respectively. By way of further example, in one such embodiment the weight ratio of the combined amount of 2-propen-1-ylamine, or a salt thereof, and 1,3-bis(allylamino)propane, or a salt thereof, to the amount of water in the reaction mixture will be in the range of about 0.15 to about 2, respectively. By way of further example, in one such embodiment the weight ratio of the combined amount of 2-propen-1-ylamine, or a salt thereof, and 1,3-bis(allylamino)propane, or a salt thereof, to the amount of water in the reaction mixture will be in the range of about 0.2 to about 1.75, respectively. By way of further example, in one such embodiment the weight ratio of the combined amount of 2-propen-1-ylamine, or a salt thereof, and 1,3-bis(allylamino)propane, or a salt thereof, to the amount of water in the reaction mixture will be in the range of about 0.25 to about 1.5, respectively. By way of further example, in one such embodiment the weight ratio of the combined amount of 2-propen-1-ylamine, or a salt thereof, and 1,3-bis(allylamino) propane, or a salt thereof, to the amount of water in the reaction mixture will be in the range of about 0.25 to about 1.25, respectively. By way of further example, in one such embodiment the weight ratio of the combined amount of 2-propen-1-ylamine, or a salt thereof, and 1,3-bis(allylamino)propane, or a salt thereof, to the amount of water in the reaction mixture will be in the range of about 0.3 to about 1, respectively. By way of further example, in one such embodiment the weight ratio of the combined amount of 2-propen-1-ylamine, or a salt thereof, and 1,3-bis(allylamino)propane, or a salt thereof, to the amount of water in the reaction mixture will be in the range of about 0.35 to about 0.75, respectively. By way of further example, in one such embodiment the weight ratio of the combined amount of 2-propen-1-ylamine, or a salt thereof, and 1,3-bis(allylamino)propane, or a salt thereof, to the amount of water in the reaction mixture will be in the range of about 0.4 to about 0.5, respectively. In each of these exemplary embodiments in this paragraph, it is assumed that the 2-propen-1-ylamine, or a salt thereof, and 1,3-bis(allylamino)propane, or a salt thereof, species are in their respective free amine forms for purposes of the weight ratio calculation.

Experience to-date has further shown that the ratio of the number of allyl equivalents to the number of water equivalents in the reaction mixture has an influence on the characteristics of the resulting polymer. Typically, therefore, the ratio of the number of allyl equivalents possessed by the 2-propen-1-ylamine, or a salt thereof, and 1,3-bis(allylamino) propane, or a salt thereof, in combination, to the number of water equivalents introduced to the reaction mixture will be in the range of about 0.01:1 to about 1:1, respectively. For example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.015:1 to about 0.75:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.02:1 to about 0.5:1, respectively. By way of further

example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.03:1 to about 0.4:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.04:1 to about 0.3:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.05:1 to about 0.25:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.06:1 to about 0.2:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.07:1 to about 0.175:1, respectively. By way of further example, in one such embodiment, the ratio of the number of allyl equivalents possessed by the 2-propen-1-ylamine, or a salt thereof, and 1,3-bis(allylamino)propane, or a salt thereof, in combination, to the number of water equivalents introduced to the reaction mixture will be about 0.08:1 to about 0.15:1, respectively.

To produce polymeric beads rather than a gel, the reaction mixture for the concurrent polymerization and crosslinking step is preferably a heterogeneous reaction mixture comprising a surfactant, water and an organic solvent system (in addition to the 2-propen-1-ylamine, or a salt thereof, 1,3-bis(allylamino)propane, or a salt thereof, acid and initiator). Advantageously, heterogeneous polymerization processes produce polymer particles in the form of substantially spherical beads, whose diameter may be controlled in the 3 to 1000 micrometer range, preferably in the 10 to 500 micrometer range, and, in some embodiments, in the 40-180 micrometer range.

In general, the surfactant comprised by the reaction mixture for the concurrent polymerization and crosslinking step may be ionic or non-ionic. Exemplary surfactants include sorbitan monolaurate, sorbitan monooleate, sorbitan monostearate, sorbitan monopalmitate, ethylene glycol monostearate, glyceryl monostearate, polyethylene glycol monostearate, polyethylene glycol hydrogenated castor oil, polyoxyethylene sorbitan monolaurate, polyoxyethylene sorbitan monooleate, polyethylene glycol and diisooctyl sulfosuccinate, branched dodecylbenzenesulfonic acid, linear dodecylbenzenesulfonic acid, sodium branched alkylbenzenesulfonate, sodium branched dodecylbenzenesulfonate, sodium alpha olefin sulfonate, sodium linear alkylbenzenesulfonate, isopropylamine branched alkylbenzenesulfonate, sodium lauryl sulfate. For example, the surfactant is branched dodecylbenzenesulfonic acid.

The organic solvent system comprised by the concurrent polymerization and crosslinking step reaction mixture may be any of a wide range of water-immiscible organic solvents that may be used to disperse an aqueous phase. Exemplary organic solvent systems may comprise hexane, cyclohexane, heptane, octane, decane, petroleum ether, liquid paraffin, chlorobenzene, toluene, xylenes, ethyl acetate, propyl acetate and isopropyl acetate, or a combination of two or more thereof. For example, the organic solvent system may comprise heptane.

The concurrent polymerization and crosslinking step reaction mixture contain any of a wide range of acids. For example, in one embodiment the concurrent polymerization and crosslinking step reaction mixture contains a mineral acid or an organic acid. Exemplary mineral acids include hydrochloric acid, sulfuric acid and phosphoric acid. Exemplary organic acids include formic acid, acetic acid, and citric acid. In one embodiment, the concurrent polymerization and crosslinking reaction step reaction mixture comprises an acid selected from the group consisting of hydrochloric acid, phosphoric acid, sulfuric acid, acetic acid, methylphosphoric acid, formic acid, citric acid, and combinations thereof. In one embodiment, the concurrent polymerization and crosslinking reaction step reaction mixture comprises an acid selected from the group consisting of hydrochloric acid, phosphoric acid, sulfuric acid, and combinations thereof. In one embodiment, the concurrent polymerization and crosslinking reaction step reaction mixture comprises hydrochloric acid. In general, the concurrent polymerization and crosslinking reaction step reaction mixture comprises at least 0.4 equivalents of acid per allyl amine equivalent. For example, in one embodiment the concurrent polymerization and crosslinking reaction step reaction mixture comprises at least 0.6 equivalents of acid per allyl amine equivalent. By way of further example, in one such embodiment the concurrent polymerization and crosslinking reaction step reaction mixture comprises at least 0.8 equivalents of acid per allyl amine equivalent. By way of further example, in one such embodiment the concurrent polymerization and crosslinking reaction step reaction mixture comprises at least 0.9 equivalents of acid per allyl amine equivalent. By way of further example, in one such embodiment the concurrent polymerization and crosslinking reaction step reaction mixture comprises at least 0.95 equivalents of acid per allyl amine equivalent. By way of further example, in one such embodiment the concurrent polymerization and crosslinking reaction step reaction mixture comprises at least 1.0 equivalents of acid per allyl amine equivalent. By way of further example, in one such embodiment the concurrent polymerization and crosslinking reaction step reaction mixture comprises at least 1 equivalent of acid per allyl amine equivalent.

In each of the foregoing embodiments, the acid may be introduced to the concurrent polymerization and

crosslinking reaction step reaction mixture independent of the addition of the 2-propen-1-ylamine, or a salt thereof, and 1,3-bis(allylamino)propane, or a salt thereof, to the first step reaction mixture. Alternatively, in each of the foregoing embodiments, the acid may be introduced to the concurrent polymerization and crosslinking reaction step reaction mixture as a component of the acid salt of the 2-propen-1-ylamine, or a salt thereof, the 1,3-bis(allylamino)propane, or a salt thereof, or both the 2-propen-1-ylamine, or a salt thereof, and the 1,3-bis(allylamino)propane, or a salt thereof.

The concurrent polymerization and crosslinking reaction step may be carried out at a temperature in the range of about 25 °C to about 85 °C. Typically, the concurrent polymerization and crosslinking reaction step will be carried out at a temperature in the range of about 30 °C to about 85 °C. In some embodiments, the concurrent polymerization and crosslinking reaction step will be carried out at a temperature in the range of about 35 °C to about 85 °C. In some embodiments, the concurrent polymerization and crosslinking reaction step will be carried out at a temperature in the range of about 40 °C to about 85 °C. In some embodiments, the concurrent polymerization and crosslinking reaction step will be carried out at a temperature in the range of about 45 °C to about 85 °C. In some embodiments, the concurrent polymerization and crosslinking reaction step will be carried out at a temperature of about 60-80 °C. In general, the temperature may be held relatively constant over the course of the reaction or it may be ramped in a continuous or step-wise manner.

The concurrent polymerization and crosslinking reaction step may be carried out for a reaction time of at least about 2 hours. Typically, the concurrent polymerization and crosslinking reaction step will be carried out for a reaction time of at least about 5 hours. In some embodiments, the concurrent polymerization and crosslinking reaction step will be carried out for a reaction time of at least about 10 hours. In some embodiments, the concurrent polymerization and crosslinking reaction step will be carried out for a reaction time of at least about 15 hours. In some embodiments, the concurrent polymerization and crosslinking reaction step will be carried out for a reaction time of at least about 20 hours. In some embodiments, the concurrent polymerization and crosslinking reaction step will be carried out for a reaction time of at least about 25 hours. In some embodiments, the concurrent polymerization and crosslinking reaction step will be carried out for a reaction time of at least about 30 hours. In some embodiments, the concurrent polymerization and crosslinking reaction step will be carried out for a reaction time of at least about 35 hours. In some embodiments, the concurrent polymerization and crosslinking reaction step will be carried out for a reaction time of at least about 40 hours. Typically, however, the concurrent polymerization and crosslinking reaction step will be carried out for a reaction time of no more than about 50 hours.

The aqueous solid content (ASC) of the concurrent polymerization and crosslinking reaction step may be about 20 to about 60 wt%. In some embodiments, the aqueous solid content is about 30 to about 50 wt%. In some embodiments, the aqueous solid content is about 30 to about 45 wt%. In some embodiments, the aqueous solid content is about 43 wt%.

In general, the concurrent polymerization and crosslinking reaction step may include, a lone polymerization reaction, stepwise addition of individual starting materials via a series of reactions, the stepwise addition of blocks of monomers, or combinations thereof. The reaction may be carried out as a batch, semi-batch or continuous process.

In one embodiment, the concurrent polymerization and crosslinking step yields preformed poly(allylamine) polymer beads having a target species binding capacity, and a target Swelling Ratio. For example, in one such embodiment the beads have a chloride binding capacity of at least 10 mmol/g in Simulated Gastric Fluid ("SGF") and a Swelling Ratio in the range of 1 to 10. In one embodiment, the preformed poly(allylamine) polymer beads are characterized by Swelling Ratio of about 2 and 10, more typically about 2 to about 8, and in some embodiments about 2 to 3, about 3 to 4 or about 4 to about 6.

Additionally, if the preformed poly(allylamine) polymer beads resulting from the first polymerization step are protonated, this may reduce the amount of nitrogen-nitrogen crosslinking in the second crosslinking step. Accordingly, in certain embodiments the preformed poly(allylamine) polymer is at least partially deprotonated by treatment with a base, preferably a strong base such as a hydroxide base. For example, in one embodiment the base may be NaOH, KOH, $NH_4OH$, $NaHCO_3$, $Na_2CO_3$, $K_2CO_3$, LiOH, $Li_2CO_3$, CsOH or other metal hydroxides. By way of further example, the base is NaOH. If the charges are removed from the preformed crosslinked amine polymer bead by deprotonation, the bead will tend to collapse and the 1,2-dichloroethane crosslinking agent used in the second crosslinking step may not be able to access binding sites on the polymer unless the bead is prevented from collapsing. One means of preventing the crosslinked polymer bead from collapsing is the use of a swelling agent such as water to swell the bead, thereby allowing the 1,2-dichloroethane second-step crosslinker to access binding sites.

As previously noted, 1,3-bis(allylamino)propane, or a salt thereof, monomers that are only partially incorporated into poly(allylamine) polymer in the first step will introduce pendant allyl groups (*i.e.,* at least one allyl group that did not participate in a reaction to become a chain atom of and is merely "dangling" from the poly(allylamine) polymer backbone chain) to the poly(allylamine) polymer. As such, a determination of the number of $sp^2$ allyl carbons pendent to the crosslinked poly(allylamine) polymer backbone remaining in the poly(allylamine) polymer upon completion of the concurrent polymerization and crosslinking step (i.e., the first step) may be used to determine the amount of partially-incorporated 1,3-bis(allylamino)propane, or a salt thereof. In general, it is preferred that the number of $sp^2$ allyl carbons constitute a low percentage of total carbon atoms in the poly(allylamine) polymer. For example, in one embodiment, $sp^2$

allyl carbons pendent to the poly(allylamine) polymer backbone constitute less than 1.1% of the total number of carbon atoms in the polymer. By way of further example, in one embodiment $sp^2$ allyl carbons pendent to the poly(allylamine) polymer backbone constitute less than 1.0% of the total number of carbon atoms in the polymer. By way of further example, in one embodiment $sp^2$ allyl carbons pendent to the poly(allylamine) polymer backbone constitute less than 0.9% of the total number of carbon atoms in the polymer. By way of further example, in one embodiment $sp^2$ allyl carbons pendent to the poly(allylamine) polymer backbone constitute less than 0.8% of the total number of carbon atoms in the polymer. By way of further example, in one embodiment $sp^2$ allyl carbons pendent to the poly(allylamine) polymer backbone constitute less than 0.75% of the total number of carbon atoms in the polymer. By way of further example, in one embodiment $sp^2$ allyl carbons pendent to the poly(allylamine) polymer backbone constitute less than 0.7% of the total number of carbon atoms in the polymer. By way of further example, in one embodiment $sp^2$ allyl carbons pendent to the poly(allylamine) polymer backbone constitute less than 0.6% of the total number of carbon atoms in the polymer. By way of further example, in one embodiment $sp^2$ allyl carbons pendent to the poly(allylamine) polymer backbone constitute less than 0.5% of the total number of carbon atoms in the polymer. By way of further example, in one embodiment $sp^2$ allyl carbons pendent to the poly(allylamine) polymer backbone constitute less than 0.4% of the total number of carbon atoms in the polymer. By way of further example, in one embodiment $sp^2$ allyl carbons pendent to the poly(allylamine) polymer backbone constitute less than 0.3% of the total number of carbon atoms in the polymer. By way of further example, in one embodiment $sp^2$ allyl carbons pendent to the poly(allylamine) polymer backbone constitute less than 0.25% of the total number of carbon atoms in the polymer. By way of further example, in one embodiment $sp^2$ allyl carbons pendent to the poly(allylamine) polymer backbone constitute less than 0.2% of the total number of carbon atoms in the polymer. By way of further example, in one embodiment $sp^2$ allyl carbons pendent to the poly(allylamine) polymer backbone constitute less than 0.1% of the total number of carbon atoms in the polymer. By way of further example, in one embodiment $sp^2$ allyl carbons pendent to the poly(allylamine) polymer backbone constitute less than 0.05% of the total number of carbon atoms in the polymer. By way of further example, in one embodiment $sp^2$ allyl carbons pendent to the poly(allylamine) polymer backbone are not detectible in the polymer beads. By way of further example, in one embodiment $sp^2$ allyl carbons pendent to the poly(allylamine) polymer backbone may constitute more than 0.3% of the total number of carbon atoms in the polymer, but are less than the higher limits expressed above, e.g. 1.0%. In each of the foregoing exemplary embodiments, the percentage of $sp^2$ allyl carbons pendent to the poly(allylamine) polymer backbone in the beads may be determined by quantitative $^{13}C$ solid state magic angle spinning (MAS) NMR measurement on a Bruker AVANCE III 800 MHz (18.8T) standard bore spectrometer, operating at 800.25 MHz and 201.24 MHz for $^1H$ and $^{13}C$, respectively, with a 4 mm zirconia rotor system at a spinning frequency of 16 kHz as more fully described elsewhere herein. In each of the foregoing exemplary embodiments, the percentage of $sp^2$ allyl carbons pendent to the poly(allylamine) polymer backbone in the beads may be determined by any of the methods disclosed herein.

In the second crosslinking step, the preformed poly(allylamine) polymer is crosslinked with 1,2-dichloroethane.

In general, it is preferred that the number of $sp^2$ allyl carbons constitute a low percentage of total carbon atoms in the post-polymerization crosslinked polymer. For example, in one embodiment, $sp^2$ allyl carbons pendent to the post-polymerization crosslinked polymer backbone of the crosslinked poly(allylamine) polymer constitute less than 1.0% of the total number of carbon atoms in the polymer. By way of further example, in one embodiment $sp^2$ allyl carbons pendent to the post-polymerization crosslinked polymer backbone constitute less than 0.9% of the total number of carbon atoms in the polymer. By way of further example, in one embodiment $sp^2$ allyl carbons pendent to the post-polymerization crosslinked polymer backbone constitute less than 0.8% of the total number of carbon atoms in the polymer. By way of further example, in one embodiment $sp^2$ allyl carbons pendent to the post-polymerization crosslinked polymer backbone constitute less than 0.75% of the total number of carbon atoms in the polymer. By way of further example, in one embodiment $sp^2$ allyl carbons pendent to the post-polymerization crosslinked polymer backbone constitute less than 0.7% of the total number of carbon atoms in the polymer. By way of further example, in one embodiment $sp^2$ allyl carbons pendent to the post-polymerization crosslinked polymer backbone constitute less than 0.6% of the total number of carbon atoms in the polymer. By way of further example, in one embodiment $sp^2$ allyl carbons pendent to the post-polymerization crosslinked polymer backbone constitute less than 0.5% of the total number of carbon atoms in the polymer. By way of further example, in one embodiment $sp^2$ allyl carbons pendent to the post-polymerization crosslinked polymer backbone constitute less than 0.4% of the total number of carbon atoms in the polymer. By way of further example, in one embodiment $sp^2$ allyl carbons pendent to the post-polymerization crosslinked polymer backbone constitute less than 0.3% of the total number of carbon atoms in the polymer. By way of further example, in one embodiment $sp^2$ allyl carbons pendent to the post-polymerization crosslinked polymer backbone constitute less than 0.25% of the total number of carbon atoms in the polymer. By way of further example, in one embodiment $sp^2$ allyl carbons pendent to the post-polymerization crosslinked polymer backbone constitute less than 0.2% of the total number of carbon atoms in the polymer. By way of further example, in one embodiment $sp^2$ allyl carbons pendent to the post-polymerization crosslinked polymer backbone constitute less than 0.1% of the total number of carbon atoms in the polymer. By way of further example, in one embodiment $sp^2$ allyl carbons pendent to the post-polymerization crosslinked polymer backbone constitute less than 0.05% of the total number of carbon atoms in the polymer. By way of further example, in one embodiment $sp^2$ allyl carbons pendent to the post-polymerization crosslinked polymer backbone are not detectible in the polymer beads. In each of the foregoing exemplary embodiments, the percentage of $sp^2$ allyl carbons pendent to the post-polymerization crosslinked polymer backbone in the beads may be determined by quantitative $^{13}C$ solid state magic angle spinning (MAS) NMR measurement on a Bruker AVANCE III 800 MHz (18.8T) standard bore spectrometer, operating at 800.25 MHz and 201.24 MHz for $^{1}H$ and $^{13}C$, respectively, with a 4 mm zirconia rotor system at a spinning frequency of 16 kHz as more fully described elsewhere herein. In each of the foregoing exemplary embodiments, the percentage of $sp^2$ allyl carbons pendent to the post-polymerization crosslinked polymer backbone in the beads may be determined by any of the methods disclosed herein.

The resulting post-polymerization crosslinked polymer will have a ratio of sp3 carbon to sp2 carbon that is greater than the ratio of sp3 carbon to sp2 carbon in the preformed poly(allylamine) polymer. Thus, the second crosslinking reaction with 1,2-dichloroethane will increase the amount of sp3 carbon in the post-polymerization crosslinked polymer while the amount of sp2 carbon will remain unchanged relative to the preformed poly(allylamine) polymer. In certain embodiments, for example, in which the preformed poly(allylamine) polymer is significantly crosslinked in the second crosslinking step, the incremental increase of sp3 carbon can be sufficient to degrade the signal to noise ratio for sp2 carbon, thereby decreasing measurement sensitivity. In certain embodiments, therefore, rather than directly measuring the amounts of sp2 carbon and sp3 carbon in the resulting post polymerization crosslinked polymer by NMR, it may be advantageous to measure the amount of sp2 carbon in the preformed poly(allylamine) polymer, determining how much sp3 carbon (and sp2 carbon, if any) was added to the polymer during the second crosslinking step, and then calculating the relative proportion of sp2 and sp3 carbon in the resulting post-polymerization crosslinked polymer. Examples are given in Tables 10 and 11 in which the percent of sp2 carbon were experimentally determined for both preformed poly(allylamine) polymers and corresponding crosslinked poly(allylamine) polymers. For those in which sp2 carbon could be quantified in the preformed crosslinked poly(allylamine) polymer, the average ratio of the percent of sp2 carbon in crosslinked poly(allylamine) polymers to the corresponding preformed poly(allylamine) polymer was about 0.9. This factor was applied where applicable throughout Table 11 part 2 in order to calculate the percent of sp2 carbon comprising certain examples of crosslinked poly(allylamine) polymers in Table 11.

In embodiments, the crosslinker for the step 2 crosslinking reaction is 1,2-dichloroethane (see Table B).

Table B

| Abbreviation | Common name | IUPAC name | | MW (g/mol) |
|---|---|---|---|---|
| DCE | dichloroethane | 1,2- dichloroethane | 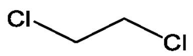 | 98.96 |
| DCP | Dichloropropane | 1,3-Dichloropropane | 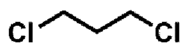 | 112.98 |

In one embodiment, the preformed poly(allylamine) polymer formed in the concurrent polymerization and crosslinking reaction is further crosslinked in a second crosslinking step in a reaction mixture comprising 1,2-dichloroethane, a swelling agent for the preformed poly(allylamine) polymer, and a dispersing solvent system. The dispersing solvent system comprises a sufficient amount of solvent to disperse the preformed poly(allylamine) polymer in the reaction mixture so as to avoid inter-polymer particle (i.e., inter-bead) crosslinking reactions and resulting aggregation. In one such embodiment, for example, the ratio of solvent comprised by the dispersing solvent system to preformed poly(allylamine) polymer in the reaction mixture is at least 2:1 (milliliters of solvent : grams of preformed poly(allylamine) polymer). By way of further example, in one such embodiment the ratio of solvent comprised by the dispersing solvent system to preformed poly(allylamine) polymer in the reaction mixture is at least 3:1 (milliliters of solvent : grams of preformed poly(allylamine) polymer). By way of further example, in one such embodiment the ratio of solvent comprised by the dispersing solvent system to preformed poly(allylamine) polymer in the reaction mixture is at least 4:1 (milliliters of solvent : grams of preformed poly(allylamine) polymer). By way of further example, in one such embodiment the ratio of solvent comprised by the dispersing solvent system to preformed poly(allylamine) polymer in the reaction mixture is at least 5:1 (milliliters of solvent: grams of preformed poly(allylamine) polymer). By way of further example, in one such embodiment the ratio of solvent comprised by the dispersing solvent system to preformed poly(allylamine) polymer in the reaction mixture is at least 7.5:1 (milliliters of solvent : grams of preformed poly(allylamine) polymer). By way of further example, in one such embodiment the ratio of solvent comprised by the dispersing solvent system to preformed poly(allylamine) polymer in the reaction mixture is at least 10:1 (milliliters of solvent : grams of preformed poly(allylamine) polymer). By way of further example, in one such embodiment the ratio of solvent comprised by the dispersing solvent system to preformed poly(allylamine) polymer in the reaction mixture is at least 20:1 (milliliters of solvent : grams of preformed poly(allylamine) polymer). In each of the foregoing embodiments, the dispersing solvent system may comprise a combination of (i) an inert solvent (relative to the preformed poly(allylamine) polymer) such as one of the non-polar solvents previously identified in connection with the organic solvent system in which the preformed poly(allylamine) polymer is formed in the first

crosslinking step and (ii) 1,2-dichloroethane (DCE) as a crosslinking solvent. Alternatively, in each of the foregoing embodiments, the dispersing solvent system may exclusively comprise as solvents 1,2-dichloroethane (DCE), but no inert solvent), thus performing a dual-purpose role, as both solvent (dispersant) and crosslinker. In a further alternative, in each of the foregoing embodiments, the dispersing solvent system may exclusively comprise as solvents neat 1,2-dichloroethane (DCE) but no inert solvent), thus performing a dual-purpose role, as both solvent (dispersant) and crosslinker.

As noted above, in certain embodiments a swelling agent for the preformed poly(allylamine) polymer is included in the second crosslinking step reaction mixture, *i.e.,* along with the 1,2-dichloroethane. In general, the swelling agent and the 1,2-dichloroethane may be miscible or immiscible and the swelling agent may be any composition or combination of compositions that have the capacity to swell the preformed poly(allylamine) polymer. Exemplary swelling agents include polar solvents such as water, methanol, ethanol, n-propanol, isopropanol, n-butanol, formic acid, acetic acid, acetonitrile, dimethylformamide, dimethylsulfoxide, nitromethane, propylene carbonate, or a combination thereof. For example, the swelling agent is water. Additionally, the amount of swelling agent included in the second crosslinking step reaction mixture will typically be less than absorption capacity of the preformed poly(allylamine) polymer for the swelling agent. For example, it is generally preferred that the weight ratio of swelling agent to preformed polymer in the second crosslinking step reaction mixture be less than 4:1. By way of further example, in some embodiments the weight ratio of swelling agent to preformed polymer in the second crosslinking step reaction mixture will be less than 3:1. By way of further example, in some embodiments the weight ratio of swelling agent to preformed polymer in the second crosslinking step reaction mixture will be less than 2:1. By way of further example, in some embodiments the weight ratio of swelling agent to preformed polymer in the second crosslinking step reaction mixture will be less than 1:1. By way of further example, in some embodiments the weight ratio of swelling agent to preformed polymer in the second crosslinking step reaction mixture will be less than 0.5:1. By way of further example, in some embodiments the weight ratio of swelling agent to preformed polymer in the second crosslinking step reaction mixture will be less than 0.4:1. By way of further example, in some embodiments the weight ratio of swelling agent to preformed polymer in the reaction mixture will be less than 0.3:1. In general, however, the weight ratio of swelling agent to preformed polymer in the second crosslinking step reaction mixture will typically be at least 0.05:1, respectively.

The second crosslinking step may be carried out at a temperature in the range of about 25 °C to about 85 °C. Typically, the second crosslinking step will be carried out at a temperature in the range of about 35 °C to about 80 °C. In some embodiments, the second crosslinking step will be carried out at a temperature in the range of about 45 °C to about 80 °C. In some embodiments, the second crosslinking step will be carried out at a temperature in the range of about 55 °C to about 75 °C. In some embodiments, the second crosslinking step will be carried out at a temperature in the range of about 60 °C to about 75 °C. In some embodiments, the second crosslinking step will be carried out at a temperature in the range of about 65 °C to about 75 °C. In general, the temperature may be held relatively constant over the course of the second crosslinking step or it may be ramped in a continuous or step-wise manner.

The second crosslinking step may be carried out for a period of about 2 to 20 hours. Typically, the second crosslinking step will be carried out for a period of about 4 to 20 h. In some embodiments, the second crosslinking step will be carried out for a period of about 5 to 20 h. In some embodiments, the second crosslinking step will be carried out for a period of about 6 to 20 h. In some embodiments, the second crosslinking stepwill be carried out for a period of about 8 to 20 h. In some embodiments, the second crosslinking step will be carried out for a period of about 10 to 20 h. In some embodiments, the second crosslinking step will be carried out for a period of about 12 to 18 h. In some embodiments, the second crosslinking step will be carried out for a period of about 14 to 18 h. In some embodiments, the second crosslinking step will be carried out for a period of about 15 to 17 h.

In one embodiment, the resulting preformed poly(allylamine) polymer is at least partially deprotonated with a base and combined with a non-protonating swelling agent to swell the free amine polymer without protonating the amine functions prior to the second crosslinking step. Thus, for example, the amount of the non-protonating swelling agent may be selected to tune the subsequent degree of crosslinking effectively forming a template that is then locked into place via the amine consuming crosslinking step.

The benefits of deprotonated preformed polymer beads in the second crosslinking step highlights the advantages of using two steps to achieve the final product. In the first crosslinking step, to form the amine polymer bead, all monomers (i.e., 2-propen-1-ylamine, or a salt thereof, and 1,3-bis(allylamino)propane, or a salt thereof,) are protonated to remain in the aqueous phase and to avoid the radical transfer reactions that severely limit the polymerization of non-protonated allylamine (and derivatives). Once the bead is formed through carbon-carbon crosslinks, the bead can then be deprotonated and further crosslinked with 1,2-dichloroethane in the second crosslinking step.

In one embodiment, selectivity for chloride over other competing ions is achieved with highly crosslinked amine polymers. For example, relatively high chloride binding capacity maybe be attained by reacting a preformed poly(allylamine) polymer bead with neat 1,2-dichloroethane in the presence of a swelling agent (water). While this "non-dispersed" reaction provides access to high selectivity for chloride over competing ions in the SIB assays, it also results in macroscopically (and microscopically) aggregated polymer beads. Accordingly, it is advantageous to include a solvent (e.g., heptane) in the second crosslinking step to disperse the preformed crosslinked polymer beads so as to avoid inter-bead reactions and resulting aggregation. The use of too much solvent (dispersant), however, can dilute the reaction solution to the point where the resulting bead is not sufficiently crosslinked to have the desired selectivity for chloride over other competing anions. By using a crosslinking agent (i.e. 1,2-dichloroethane) that also functions as a solvent (dispersant), however, sufficient solvent (dispersant) may be included in the reaction mixture to avoid inter-bead reactions and aggregation without diluting the mixture to the point where the degree of amine-consuming crosslinking is insufficient. For example, in an effort to utilize the dispersing properties of a solvent (to avoid aggregation during the reaction) while maintaining reactivity, 1,2-dichloroethane (DCE) was used neat, thus performing a dual purpose role, as both solvent (dispersant) and crosslinker. Interestingly, DCE was discovered to have excellent dispersal properties as a solvent, when compared to similar reactions with DCP and/or heptane. Additionally, less aggregation was observed when the beads were first dispersed in DCE and then in a second operation, the water is added to swell the beads. If water is added to the preformed poly(allylamine) polymer before the bead is dispersed in the DCE, aggregation may occur.

In each of the foregoing embodiments, the reaction mixture for the second crosslinking step may contain a wide range of amounts of 1,2-dichloroethane. For example, in one embodiment the 1,2-dichloroethane may be used in large excess relative to the amount of preformed poly(allylamine) polymer in the reaction mixtures. Stated differently, in such embodiments the 1,2-dichloroethane is a crosslinking solvent, *i.e.,* it is both a solvent for the reaction mixture and a crosslinking agent for the preformed poly(allylamine) polymer. In such embodiments, other solvents may optionally be included in the dispersing solvent system of the second crosslinking step reaction mixture, but are not required. Alternatively, the preformed poly(allylamine) polymer, swelling agent and 1,2-dichloroethane may be dispersed in a dispersing solvent system that is miscible with the 1,2-dichloroethane and immiscible with the swelling agent. For example, in some embodiments the swelling agent may be a polar solvent; in some such embodiments, for example, the swelling agent may comprise water, methanol, ethanol, n-propanol, isopropanol, formic acid, acetic acid, acetonitrile, N,N-dimethylformamide, dimethylsulfoxide, nitromethane, or a combination thereof. By way of further example, when the swelling agent comprises a polar solvent, the dispersing solvent system for the second crosslinking step will typically comprise a non-polar solvent such as pentane, cyclopentane, hexane, cyclohexane, benzene, toluene, 1,4-dioxane, chloroform, diethyl ether, dichloromethane, dichloroethane, dichloropropane, dichlorobutane, or a combination thereof. In certain embodiments, the 1,2-dichloroethane and the solvent comprised by the dispersing solvent system may be the same.

It is notable that in a crosslinking solvent (e.g., a DCE-dispersed reaction), there is a large excess of crosslinker regardless of the amount of crosslinking solvent (e.g., 1,2-dichloroethane (DCE)) used to disperse the bead (e.g., both 1 g:3 mL::bead:DCE and 1 g:10 mL::bead:DCE are a large excess of crosslinker, most of which is not consumed during the reaction). Despite this, the relative degree of crosslinking, and the chloride capacity and performance in the SIB assay is relatively unaffected by changes in the ratio of reactive crosslinker to polymer bead. This is possible because the reaction is limited by the acid-neutralizing capacity of the polymer bead, rather than the amount of crosslinker *(e.g.,* DCE).

To more efficiently react with 1,2-dichloroethane (DCE), the amines of the preformed polymer bead preferably have a free electron pair (neutral, deprotonated). As the free amines of the preformed polymer bead react with the 1,2-dichloroethane (DCE), HCl is produced and the amines become protonated, thus limiting the reaction. For this reason, the preformed poly(allylamine) polymer beads preferably start as the free amine in the second crosslinking step. If the preformed poly(allylamine) polymer bead is protonated after the first step of carbon-carbon crosslinking, amine-consuming crosslinking in the second crosslinking step will be limited, thus reducing the desired selectivity for chloride over other competing ions. This has been demonstrated by adding known quantities of HCl to preformed poly(allylamine) polymer beads immediately before crosslinking with DCE in the second crosslinking step. When less than 3 mol % HCl (to amine in preformed polymer amine bead) is added prior to crosslinking in the second crosslinking step, total chloride capacity (SGF) and chloride selectivity in SIB are similar to beads not treated with HCl in the second crosslinking step. When greater than 5 mol % HCl (to amine in preformed polymer amine bead) is added prior to crosslinking in the second crosslinking step, total chloride capacity (SGF) increases and chloride selectivity in SIB decreases, indicating lower incorporation of 1,2-dichloroethane.

The use of 1,2-dichloroethane ("DCE") as the crosslinking solvent also generates HCl molecules during the second crosslinking step. These HCl molecules protonate some of the free amine sites which block the reaction sites for the crosslinking reaction and thereby limit the number of binding sites available for crosslinking. Consequently, the use of DCE creates a self-limiting effect on the secondary crosslinking.

Given the large excess of 1,2-dichloroethane as a dual crosslinker/solvent, mono-incorporation of 1,2-dichloroethane can occur leading to alkyl chloride functional groups on the crosslinked polymer bead that are hydrophobic in nature and can increase non-specific interactions with undesirable solutes other than HCl that are more hydrophobic in nature. Washing with ammonium hydroxide solution converts the alkyl-chloride to alkyl-amine functions that are hydrophilic and minimize non-specific interactions with undesirable solutes. Other modifications that yield more hydrophilic groups than alkyl chloride such as -OH are suitable to quench mono-incorporated crosslinker/solvent.

As noted above, in certain embodiments a swelling agent for the preformed poly(allylamine) polymer may be included in the second crosslinking step reaction mixture for the second crosslinking step along with 1,2-dichloroethane. In

general, the swelling agent and the 1,2-dichloroethane may be miscible or immiscible and the swelling agent may be any composition or combination of compositions that have the capacity to swell the preformed poly(allylamine) polymer. Exemplary swelling agents include polar solvents such as water, methanol, ethanol, n-propanol, isopropanol, n-butanol, formic acid, acetic acid, acetonitrile, dimethylformamide, dimethylsulfoxide, nitromethane, propylene carbonate, or a combination thereof. For example, the swelling agent is water. Additionally, the amount of swelling agent included in the second crosslinking step reaction mixture will typically be less than absorption capacity of the preformed poly(allylamine) polymer for the swelling agent. For example, it is generally preferred that the weight ratio of swelling agent to preformed polymer in the second crosslinking step reaction mixture be less than 4:1. By way of further example, in some embodiments the weight ratio of swelling agent to preformed polymer in the second crosslinking step reaction mixture will be less than 3:1. By way of further example, in some embodiments the weight ratio of swelling agent to preformed polymer in the second crosslinking step reaction mixture will be less than 2:1. By way of further example, in some embodiments the weight ratio of swelling agent to preformed polymer in the second crosslinking step reaction mixture will be less than 1:1. By way of further example, in some embodiments the weight ratio of swelling agent to preformed polymer in the second crosslinking step reaction mixture will be less than 0.5:1. By way of further example, in some embodiments the weight ratio of swelling agent to preformed polymer in the second crosslinking step reaction mixture will be less than 0.4:1. By way of further example, in some embodiments the weight ratio of swelling agent to preformed polymer in the second crosslinking step reaction mixture will be less than 0.3:1. In general, however, the weight ratio of swelling agent to preformed polymer in the second crosslinking step reaction mixture will typically be at least 0.05:1, respectively.

When the swelling agent comprises water, the weight ratio of water to preformed poly(allylamine) polymer in the second crosslinking step reaction mixture will typically be less than about 4:1 (water to polymer). For example, in one such embodiment the second crosslinking step reaction mixture comprises water as a swelling agent and the weight ratio of water to preformed poly(allylamine) polymer in the second crosslinking step reaction mixture will typically be less than about 3.5:1. By way of further example, in one such embodiment the second crosslinking step reaction mixture comprises water as a swelling agent and the weight ratio of water to preformed poly(allylamine) polymer in the second crosslinking step reaction mixture will typically be less than about 3:1. By way of further example, in one such embodiment the second crosslinking step reaction mixture comprises water as a swelling agent and the weight ratio of water to preformed poly(allylamine) polymer in the second crosslinking step reaction mixture will typically be less than about 2.5:1. By way of further example, in one such embodiment the second crosslinking step reaction mixture comprises water as a swelling agent and the weight ratio of water to preformed poly(allylamine) polymer in the second crosslinking step reaction mixture will typically be less than about 2:1. By way of further example, in one such embodiment the second crosslinking step reaction mixture comprises water as a swelling agent and the weight ratio of water to preformed poly(allylamine) polymer in the second crosslinking step reaction mixture will typically be less than about 1.5:1. By way of further example, in one such embodiment the second crosslinking step reaction mixture comprises water as a swelling agent and the weight ratio of water to preformed poly(allylamine) polymer in the second crosslinking step reaction mixture will typically be less than about 1:1. By way of further example, in one such embodiment the second crosslinking step reaction mixture comprises water as a swelling agent and the weight ratio of water to preformed poly(allylamine) polymer in the second crosslinking step reaction mixture will typically be less than about 0.75:1. By way of further example, in one such embodiment the second crosslinking step reaction mixture comprises water as a swelling agent and the weight ratio of water to preformed poly(allylamine) polymer in the second crosslinking step reaction mixture will typically be less than about 0.5:1. By way of further example, in one such embodiment the second crosslinking step reaction mixture comprises water as a swelling agent and the weight ratio of water to preformed poly(allylamine) polymer in the second crosslinking step reaction mixture will typically be less than about 0.25:1. In general, however, when water is employed as a swelling agent the weight ratio of water to preformed poly(allylamine) polymer in the second crosslinking step reaction mixture will typically be at least about 0.15:1 (water to polymer) but less than the water absorption capacity of the preformed poly(allylamine) polymer. By way of further example, in one embodiment the weight ratio of water to preformed poly(allylamine) polymer in the second crosslinking step reaction mixture will typically be at least about 0.2:1 but less than the water absorption capacity of the preformed poly(allylamine) polymer. By way of further example, in one embodiment the weight ratio of water to preformed poly(allylamine) polymer in the second crosslinking step reaction mixture will typically be at least about 0.25:1 but less than the water absorption capacity of the preformed poly(allylamine) polymer. By way of further example, in one embodiment the weight ratio of water to preformed poly(allylamine) polymer in the second crosslinking step reaction mixture will typically be at least about 0.5:1 but less than the water absorption capacity of the preformed poly(allylamine) polymer. By way of further example, in one embodiment the weight ratio of water to preformed poly(allylamine) polymer in the second crosslinking step reaction mixture will typically be at least about 0.75:1 but less than the water absorption capacity of the preformed poly(allylamine) polymer. By way of further example, in one embodiment the weight ratio of water to preformed poly(allylamine) polymer in the second crosslinking step reaction mixture will typically be at least about 1:1 but less than the water absorption capacity of the preformed poly(allylamine) polymer. By way of further example, in one embodiment the weight ratio of water to preformed poly(allylamine) polymer in the second crosslinking step reaction mixture will typically be at least about 1.5:1 but less than the water absorption capacity of the preformed poly(allylamine) polymer. By way of further

example, in one embodiment the weight ratio of water to preformed poly(allylamine) polymer in the second crosslinking step reaction mixture will typically be at least about 2:1 but less than the water absorption capacity of the preformed poly(allylamine) polymer. By way of further example, in one embodiment the weight ratio of water to preformed poly(allylamine) polymer in the second crosslinking step reaction mixture will typically be at least about 2.5:1 but less than the water absorption capacity of the preformed poly(allylamine) polymer. By way of further example, in one embodiment the weight ratio of water to preformed poly(allylamine) polymer in the second crosslinking step reaction mixture will typically be at least about 3:1 but less than the water absorption capacity of the preformed poly(allylamine) polymer. By way of further example, in one embodiment the weight ratio of water to preformed poly(allylamine) polymer in the second crosslinking step reaction mixture will typically be at least about 3.5:1 but less than the water absorption capacity of the preformed poly(allylamine) polymer. Thus, in certain embodiments the weight ratio of water to preformed poly(allylamine) polymer will be in the range of about 0.15:1 to about 4:1. By way of further example, in certain embodiments the weight ratio of water to preformed poly(allylamine) polymer will be in the range of about 0.2:1 to about 3.5:1. By way of further example, in certain embodiments the weight ratio of water to preformed poly(allylamine) polymer will be in the range of about 0.2:1 to about 3:1.

The second crosslinking step reaction mixture may contain a wide range of amounts of 1,2-dichloroethane. For example, in one embodiment the 1,2-dichloroethane may be used in large excess relative to the amount of preformed poly(allylamine) polymer in the second crosslinking step reaction mixtures. Stated differently, in such embodiments the 1,2-dichloroethane is a crosslinking solvent, *i.e.,* it is both a solvent for the second crosslinking step reaction mixture and a crosslinking agent for the preformed poly(allylamine) polymer. In such embodiments, other solvents may optionally be included in the second crosslinking step reaction mixture but are not required. Alternatively, the preformed poly(allylamine) polymer, swelling agent and 1,2-dichloroethane may be dispersed in a solvent that is miscible with the 1,2-dichloroethane and immiscible with the swelling agent. For example, in some embodiments the swelling agent may be a polar solvent; in some such embodiments, for example, the swelling agent may comprise water, methanol, ethanol, n-propanol, isopropanol, formic acid, acetic acid, acetonitrile, dimethylformamide, dimethylsulfoxide, nitromethane, or a combination thereof. By way of further example, when the swelling agent comprises a polar solvent, the solvent system for the second crosslinking step reaction mixture will typically comprise a non-polar solvent such as pentane, cyclopentane, hexane, cyclohexane, benzene, toluene, 1,4-dioxane, chloroform, diethyl ether, dichloromethane, dichloroethane, dichloropropane, dichlorobutane, or a combination thereof. In certain embodiments, the crosslinker and the solvent may be the same; i.e., the solvent is 1,2-dichloroethane.

In those embodiments in which the second crosslinking step reaction mixture comprises a swelling agent, it is sometimes preferred to combine the preformed poly(allylamine) polymer with the solvent (sometimes alternatively referred to as a dispersant) before the preformed poly(allylamine) polymer is combined with the swelling agent in the second crosslinking step reaction mixture. In certain embodiments, the resulting crosslinked polymer tends to be less aggregated when the preformed poly(allylamine) polymer is combined with a solvent (dispersant) that is immiscible with the swelling agent before the preformed poly(allylamine) polymer is combined with the swelling agent. Thus, in certain embodiments less than 25% of the particles in a representative sample of a population of post polymerization crosslinked amine particles are aggregated into agglomerates. For example, in some embodiments less than 20% of the particles in a representative sample of a population of post polymerization crosslinked amine particles are aggregated into agglomerates. By way of further example, in some embodiments less than 15% of the particles in a representative sample of a population of post polymerization crosslinked amine particles are aggregated into agglomerates. By way of further example, in some embodiments less than 10% of the particles in a representative sample of a population of post polymerization crosslinked amine particles are aggregated into agglomerates. By way of further example, in some embodiments less than 5% of the particles in a representative sample of a population of post polymerization crosslinked amine particles are aggregated into agglomerates. By way of further example, in some embodiments less than 1% of the particles in a representative sample of a population of post polymerization crosslinked amine particles are aggregated into agglomerates. Aggregation can be evaluated using microscopy or other means of measuring particle size distribution. Lack of aggregation can be defined as generally separated, free-flowing beads lacking macroscopic and/or microscopic clumps. Particle size distribution (as defined elsewhere) can indicate that aggregation has occurred, for example if the average size (d(50)) and/or d(90) of the crosslinked poly(allylamine) polymer increases after the crosslinking step relative to the preformed poly(allylamine) polymer breads as previously described.

In one embodiment, a preformed poly(allylamine) polymer is formed in a first step and the preformed poly(allylamine) polymer is further crosslinked in a second crosslinking step without isolating the preformed poly(allylamine) polymer between the first and second crosslinking steps (sometimes referred to as a "one-pot synthesis"). For example, in one such embodiment a preformed poly(allylamine) polymer is formed in a first step reaction mixture (as previously described herein) and, without isolating the preformed poly(allylamine) polymer formed in the first step reaction mixture, the preformed poly(allylamine) polymer is then crosslinked using 1,2-dichloroethane). By way of further example, in one such embodiment the preformed polymer may be dispersed in any of the non-polar solvents disclosed herein (including for example, 1,2-dichloroethane as a crosslinking solvent) to form a second crosslinking step reaction mixture and a swelling

agent is added to this reaction mixture. In one such exemplary embodiment, the crosslinker and the solvent is 1,2-dichloroethane, and the swelling agent comprises water. In each of the foregoing embodiments, the preformed polymer is an amine-containing polymer containing the residues of 2-propen-1-ylamine, or a salt thereof, and 1,3-bis(allylamino) propane, or a salt thereof.

In one exemplary embodiment, a preformed polyamine polymer is crosslinked under, for example suspension conditions to generate a particle of targeted particle size and morphology. When a water immiscible crosslinker (e.g., 1,2-dichloroethane (DCE)) is used as the dispersant, high chloride binding selectivities are achieved, as demonstrated, for example, in SIB.

In one embodiment, an amine polymer can be formed and then further crosslinked in the same reaction flask and in one reaction series. A crosslinked amine polymer can be prepared under, for example, suspension conditions to generate a particle of targeted particle size and morphology. In the same reaction flask, and without isolation, the water content in the beads can be lowered by Dean Stark methods or other similar evaporative techniques. The water is adjusted to the targeted amount such that a second crosslinking reaction can be conducted to produce a final polymer with the desired properties and characteristics.

In one embodiment, the crosslinked poly(allylamine) amine polymer formed in a second crosslinking step (as previously described) is treated to reduce the concentration of any residual amine-reactive groups *(i.e.,* alkyl chloride functional groups) introduced to the crosslinked polymer by 1,2-dichloroethane. For example, in one such embodiment the crosslinked poly(allylamine) polymer is treated with a quenching agent such as a base, washed, heated, or otherwise treated to remove or quench the amine-reactive groups. By way of further example, in one such embodiment the crosslinked poly(allylamine) polymer is treated with ammonium hydroxide. The ammonium hydroxide treatment can occur immediately after the reaction, during the washing steps, or after the polymer has been washed and dried, in which case the polymer can be processed through another series of washing steps. In another such embodiment, the crosslinked poly(allylamine) polymer is heated in a conventional or in a vacuum oven at a temperature above room temperature for a period of time, for example 60°C for greater than 36 hours. The oven incubation may occur under an inert atmosphere (e.g., nitrogen or argon) to reduce the possibility of oxidation.

## Crosslinked Poly(allylamine) Polymers

As previously noted, the crosslinked poly(allylamine) polymers having the medical uses described herein possess the capacity to remove HCl.

In some embodiments, the crosslinked poly(allylamine) polymer of the present invention is a non-absorbed free-flowing powder composed of low-swelling, spherical beads, approximately 100 micrometers in diameter; each bead is a single crosslinked, high molecular weight molecule.

In general, the crosslinked poly(allylamine) polymer has a preferred particle size range that is (i) large enough to avoid passive or active absorption through the GI tract and (ii) small enough to not cause grittiness or unpleasant mouth feel when ingested as a powder, sachet and/or chewable tablet/dosage form with a mean particle size of at least 3 microns. For example, in one such embodiment the crosslinked poly(allylamine) polymer comprises a population of particles having a mean particle size (volume distribution) greater than 1 micrometer and less than 1 millimeter. For example, in one such embodiment the crosslinked poly(allylamine) polymer comprises a population of particles having a mean particle size (volume distribution) in the range of 5 to 1,000 microns. By way of further example, in one such embodiment the crosslinked poly(allylamine) polymer comprises a population of particles having a mean particle size (volume distribution) in the range of 5 to 500 microns. By way of further example, in one such embodiment the crosslinked poly(allylamine) polymer comprises a population of particles having a mean particle size (volume distribution) in the range of 10 to 400 microns. By way of further example, in one such embodiment the crosslinked poly(allylamine) polymer comprises a population of particles having a mean particle size (volume distribution) in the range of 10 to 300 microns. By way of further example, in one such embodiment the crosslinked poly(allylamine) polymer comprises a population of particles having a mean particle size (volume distribution) in the range of 20 to 250 microns. By way of further example, in one such embodiment the crosslinked poly(allylamine) polymer has a particle size range of 30 to 250 microns. By way of further example, in one such embodiment the crosslinked poly(allylamine) polymer has a particle size range of 40 to 180 microns. In certain embodiments, less than 7% of the particles in the population (on a number basis) have a diameter less than 10 microns. For example, in such embodiments less than 5% of the particles in the particles in the population (on a number basis) have a diameter less than 10 microns. By way of further example, in such embodiments less than 2.5% of the particles in the particles in the population (on a number basis) have a diameter less than 10 microns. By way of further example, in such embodiments less than 1% of the particles in the particles in the population (on a number basis) have a diameter less than 10 microns. In each of the exemplary embodiments recited in this paragraph, the particles are preferably in the form of beads.

To minimize GI side effects in patients that are often related to a large volume polymer gel moving through the GI tract, a low Swelling Ratio of the crosslinked poly(allylamine) polymer is preferred (0.5 to 10 times its own weight in water).

For example, in one such embodiment the crosslinked poly(allylamine) polymer has a Swelling Ratio of less than 2. By way of further example, in one such embodiment the crosslinked poly(allylamine) polymer has a Swelling Ratio of less than 1.9. By way of further example, in one such embodiment the crosslinked poly(allylamine) polymer has a Swelling Ratio of less than 1.8. By way of further example, in one such embodiment the crosslinked poly(allylamine) polymer has a Swelling Ratio of less than 1.7. By way of further example, in one such embodiment the crosslinked poly(allylamine) polymer has a Swelling Ratio of less than 1.6. By way of further example, in one such embodiment the crosslinked poly(allylamine) polymer has a Swelling Ratio of less than 1.5. By way of further example, in one such embodiment the crosslinked poly(allylamine) polymer has a Swelling Ratio of less than 1.4. By way of further example, in one such embodiment the crosslinked poly(allylamine) polymer has a Swelling Ratio of less than 1.3. By way of further example, in one such embodiment the crosslinked poly(allylamine) polymer has a Swelling Ratio of less than 1.2. By way of further example, in one such embodiment the crosslinked poly(allylamine) polymer has a Swelling Ratio of less than 1. By way of further example, in one such embodiment the crosslinked poly(allylamine) polymer has a Swelling Ratio of less than 0.9. By way of further example, in one such embodiment the crosslinked poly(allylamine) polymer has a Swelling Ratio of less than 0.8. By way of further example, in one such embodiment the crosslinked poly(allylamine) polymer has a Swelling Ratio of less than 0.7. By way of further example, in one such embodiment the crosslinked poly(allylamine) polymer has a Swelling Ratio of less than 0.6. By way of further example, in one such embodiment the crosslinked poly(allylamine) polymer has a Swelling Ratio of at least 0.5 and less than 2. By way of further example, in one such embodiment the crosslinked poly(allylamine) polymer has a Swelling Ratio of about 0.7 to about 1.7.

In general, the crosslinked poly(allylamine) polymer will have a theoretical binding capacity for protons of at least about 7.5 mEq/g (as determined in an SGF assay). For example, in some embodiments the crosslinked poly(allylamine) polymer will have a theoretical binding capacity for protons of at least about 8 mEq/g. By way of further example, in some embodiments the crosslinked poly(allylamine) polymer will have a theoretical binding capacity for protons of at least about 8.5 mEq/g. By way of further example, in some embodiments the crosslinked poly(allylamine) polymer will have a theoretical binding capacity for protons of at least about 9 mEq/g. By way of further example, in some embodiments the crosslinked poly(allylamine) polymer will have a theoretical binding capacity for protons of at least about 9.5 mEq/g. By way of further example, in some embodiments the crosslinked poly(allylamine) polymer will have a theoretical binding capacity for protons of at least about 10 mEq/g. By way of further example, in some embodiments the crosslinked poly(allylamine) polymer will have a theoretical binding capacity for protons of at least about 10.5 mEq/g. By way of further example, in some embodiments the crosslinked poly(allylamine) polymer will have a theoretical binding capacity for protons of at least about 11 mEq/g. In general, the crosslinked poly(allylamine) polymer will typically have a theoretical binding capacity for protons that is not in excess of about 35 mEq/g. For example, in some embodiments, the theoretical binding capacity of the crosslinked poly(allylamine) polymers for protons is not in excess of 30 mEq/g. The binding capacities recited in this paragraph are the theoretical binding capacities for protons and the theoretical binding capacities for chloride ions, independently and individually, and not the sum thereof.

Phosphate, bicarbonate, bicarbonate equivalents, the conjugate bases of bile and fatty acids are potential interfering anions for chloride or other conjugate bases of strong acids in the stomach and small intestine. Therefore, rapid and preferential binding of chloride over phosphate, bicarbonate equivalents, and the conjugate bases of bile and fatty acids in the small intestine is desirable and the SIB assay may be used to determine kinetics and preferential binding. Since the transit time of the colon is slow (2-3 days) relative to the small intestine, and since conditions in the colon will not be encountered by an orally administered crosslinked poly(allylamine) polymer until after stomach and small intestine conditions have been encountered, kinetics of chloride binding by a crosslinked poly(allylamine) polymer do not need to be as rapid in the colon or under in vitro conditions designed to mimic the late small intestine/colon. It is, however, desirable that chloride binding and selectivity over other interfering anions is high, for example, at 24 and/or 48 hours or longer.

In one embodiment, the crosslinked poly(allylamine) polymer is characterized by a chloride ion binding capacity of at least 2.5 mEq/g in a Simulated Small Intestine Inorganic ("SIB") assay. For example, in one such embodiment the crosslinked poly(allylamine) polymer is characterized by a chloride ion binding capacity of at least 3 mEq/g in a SIB assay. By way of further example, in one such embodiment the crosslinked poly(allylamine) polymer is characterized by a chloride ion binding capacity of at least 3.5 mEq/g in a SIB assay. By way of further example, in one such embodiment the crosslinked poly(allylamine) polymer is characterized by a chloride ion binding capacity of at least 4 mEq/g in a SIB assay. By way of further example, in one such embodiment the crosslinked poly(allylamine) polymer is characterized by a chloride ion binding capacity of at least 4.5 mEq/g in a SIB assay. By way of further example, in one such embodiment the crosslinked poly(allylamine) polymer is characterized by a chloride ion binding capacity of at least 5 mEq/g in a SIB assay. By way of further example, in one such embodiment the crosslinked poly(allylamine) polymer is characterized by a chloride ion binding capacity of at least 5.5 mEq/g in a SIB assay. By way of further example, in one such embodiment the crosslinked poly(allylamine) polymer is characterized by a chloride ion binding capacity of at least 6 mEq/g in a SIB assay. By way of further example, in one such embodiment the crosslinked poly(allylamine) polymer is characterized by a chloride ion binding capacity of about 4.1 mEq/g to about 5.4 mEq/g in a SIB assay.

In one embodiment, the crosslinked poly(allylamine) polymer binds a significant amount of chloride relative to

phosphate as exhibited, for example, in a SIB assay. For example, in one embodiment the ratio of the amount of bound chloride to bound phosphate in a SIB assay is at least 1:1, respectively. By way of further example, in one such embodiment the ratio of the amount of bound chloride to bound phosphate in a SIB assay is at least 2:1, respectively. By way of further example, in one such embodiment the ratio of the amount of bound chloride to bound phosphate in a SIB assay is at least 2.25:1, respectively. By way of further example, in one such embodiment the ratio of the amount of bound chloride to bound phosphate in a SIB assay is at least 2.5:1, respectively. By way of further example, in one such embodiment the ratio of the amount of bound chloride to bound phosphate in a SIB assay is at least 2.75:1, respectively. By way of further example, in one such embodiment the ratio of the amount of bound chloride to bound phosphate in a SIB assay is at least 3:1, respectively. By way of further example, in one such embodiment the ratio of the amount of bound chloride to bound phosphate in a SIB assay is at least 4:1, respectively. By way of further example, in one such embodiment the ratio of the amount of bound chloride to bound phosphate in a SIB assay is at least 5:1, respectively. By way of further example, in one such embodiment the ratio of the amount of bound chloride to bound phosphate in a SIB assay is about 2.1:1, respectively, to about 10.8:1, respectively.

In one embodiment, the crosslinked poly(allylamine) polymer is characterized by a proton-binding capacity and a chloride binding capacity in Simulated Gastric Fluid of at least 7.5 mEq/g in a SGF assay. For example, in one such embodiment the crosslinked poly(allylamine) polymer is characterized by a proton-binding capacity and a chloride binding capacity in a SGF assay of at least 8 mEq/g. By way of further example, in one such embodiment the crosslinked poly(allylamine) polymer is characterized by a proton-binding capacity and a chloride binding capacity in a SGF assay of at least 8.5 mEq/g. By way of further example, in one such embodiment the crosslinked poly(allylamine) polymer is characterized by a proton-binding capacity and a chloride binding capacity in a SGF assay of at least 9 mEq/g. By way of further example, in one such embodiment the crosslinked poly(allylamine) polymer is characterized by a proton-binding capacity and a chloride binding capacity in a SGF assay of at least 9.5 mEq/g. By way of further example, in one such embodiment the crosslinked poly(allylamine) polymer is characterized by a proton-binding capacity and a chloride binding capacity in a SGF assay of at least 10 mEq/g. By way of further example, in one such embodiment the crosslinked poly(allylamine) polymer is characterized by a proton-binding capacity and a chloride binding capacity in a SGF assay of at least 10.5 mEq/g. By way of further example, in one such embodiment the crosslinked poly(allylamine) polymer is characterized by a proton-binding capacity and a chloride binding capacity in a SGF assay of at least 11 mEq/g. By way of further example, in one such embodiment the crosslinked poly(allylamine) polymer is characterized by a proton-binding capacity and a chloride binding capacity in a SGF assay of at least 11.5 mEq/g. By way of further example, in one such embodiment the crosslinked poly(allylamine) polymer is characterized by a proton-binding capacity and a chloride binding capacity in a SGF assay of at least 12 mEq/g. By way of further example, in one such embodiment the crosslinked poly(allylamine) polymer is characterized by a proton-binding capacity and a chloride binding capacity in a SGF assay of at least 12.5 mEq/g. By way of further example, in one such embodiment the crosslinked poly(allylamine) polymer is characterized by a proton-binding capacity and a chloride binding capacity in a SGF assay of about 9.0 mEq/g to about 12.6 mEq/g.

By way of further example, in one such embodiment the crosslinked poly(allylamine) polymer is characterized by a proton-binding capacity and a chloride binding capacity after 1 hour in SGF that is at least 50% of the proton-binding capacity and the chloride binding capacity, respectively, of the crosslinked poly(allylamine) polymer at 24 hours in SGF. By way of further example, in one such embodiment the crosslinked poly(allylamine) polymer is characterized by a proton-binding capacity and a chloride binding capacity after 1 hour in SGF that is at least 60% of the proton-binding capacity and the chloride binding capacity, respectively, of the crosslinked poly(allylamine) polymer at 24 hours in SGF. By way of further example, in one such embodiment the crosslinked poly(allylamine) polymer is characterized by a proton-binding capacity and a chloride binding capacity after 1 hour in SGF that is at least 70% of the proton-binding capacity and the chloride binding capacity, respectively, of the crosslinked poly(allylamine) polymer at 24 hours in SGF. By way of further example, in one such embodiment the crosslinked poly(allylamine) polymer is characterized by a proton-binding capacity and a chloride binding capacity after 1 hour in SGF that is at least 80% of the proton-binding capacity and the chloride binding capacity, respectively, of the crosslinked poly(allylamine) polymer at 24 hours in SGF. By way of further example, in one such embodiment the crosslinked poly(allylamine) polymer is characterized by a proton-binding capacity and a chloride binding capacity after 1 hour in SGF that is at least 90% of the proton-binding capacity and the chloride binding capacity, respectively, of the crosslinked poly(allylamine) polymer at 24 hours in SGF.

In those embodiments in which the crosslinked poly(allylamine) polymer binds chloride ions, it is generally preferred that the crosslinked poly(allylamine) polymer selectively bind chloride ions relative to other counter ions such as bicarbonate equivalent anions, phosphate anions, and the conjugate bases of bile and fatty acids. Stated differently, it is generally preferred in these embodiments that the crosslinked poly(allylamine) polymer (i) remove more chloride ions than bicarbonate equivalent anions (ii) remove more chloride ions than phosphate anions, and (iii) remove more chloride ions than the conjugate bases of bile and fatty acids. Advantageously, therefore, treatment with the crosslinked poly(allylamine) polymer does not induce or exacerbate hypophosphatemia *(i.e.,* a serum phosphorous concentration of less than about 2.4 mg/dL, does not significantly elevate low density lipoproteins ("LDL"), or otherwise negatively impact serum or colon

levels of metabolically relevant anions.

In some embodiments, the carbon to nitrogen weight ratio of the crosslinked poly(allylamine) polymers of the present invention may range from about 2:1 to about 6:1, respectively. For example, in one such embodiment, the carbon to nitrogen weight ratio of the crosslinked poly(allylamine) polymers of the present invention may range from about 2.5:1 to about 5:1, respectively. By way of further example, in one such embodiment, the carbon to nitrogen weight ratio of the crosslinked poly(allylamine) polymers of the present invention may range from about 3:1 to about 4.5:1, respectively. By way of further example, in one such embodiment, the carbon to nitrogen weight ratio of the crosslinked poly(allylamine) polymers of the present invention may range from about 3.25:1 to about 4.25:1, respectively. By way of further example, in one such embodiment, the carbon to nitrogen weight ratio of the crosslinked poly(allylamine) polymers of the present invention may range from about 3.4:1 to about 4:1, respectively. By way of further example, in one such embodiment, the carbon to nitrogen weight ratio of the crosslinked poly(allylamine) polymers of the present invention may range from about 3.5:1 to about 4:1, respectively. By way of further example, in one such embodiment, the carbon to nitrogen weight ratio of the crosslinked poly(allylamine) polymers of the present invention may range from about 3.6:1 to about 3.9:1, respectively. By way of further example, in one such embodiment, the carbon to nitrogen weight ratio of the crosslinked poly(allylamine) polymers of the present invention may range from about 3.7:1 to about 3.8:1, respectively. In each of the foregoing embodiments, the carbon to nitrogen weight ratio may be determined by elemental analysis. For example, the carbon to nitrogen weight ratio may be determined by elemental analysis using a Perkin-Elmer 2400 Elemental Analyzer as more fully described elsewhere herein.

For example, in each of the foregoing exemplary embodiments recited in the above paragraphs, the residue of the 2-propen-1-ylamine, or a salt thereof, and/or the residue of the 1,3-bis(allylamino)propane, or a salt thereof, may be residue of a hydrochloric acid salt, sulfuric acid salt, phosphoric acid salt, hydrobromic acid salt or a combination thereof. By way of further example, in each of the foregoing exemplary embodiments recited in the above paragraphs, the residue of the 2-propen-1-ylamine, or a salt thereof, and/or the residue of the 1,3-bis(allylamino)propane, or a salt thereof, is a residue of a hydrochloric acid salt.

## Pharmaceutical Compositions & Administration

In general, the dosage levels of the crosslinked poly(allylamine) polymers of the present invention for therapeutic and/or prophylactic uses may range from about 3 g/day to about 9 g/day.

Optionally, the daily dose may be administered as a single dose *(i.e.,* one time a day), or divided into multiple doses (e.g., two, three or more doses) over the course of a day. Preferably, the daily dose is administered once daily.

In general, the crosslinked poly(allylamine) polymers may be administered as a fixed daily dose or titrated based on the serum bicarbonate values of the patient in need of treatment or other indicators of acidosis. The titration may occur at the onset of treatment or throughout, as required, and starting and maintenance dosage levels may differ from patient to patient based on severity of the underlying disease.

For example, in one embodiment, the recommended starting dose of the crosslinked poly(allylamine) polymer of the present invention for therapeutic and/or prophylactic uses is about 6 g/day. In one embodiment, the starting dose is adjusted in increments of about 3 g, up to about 9 g/day or down to about 3 g/day. In one embodiment, the dose adjustment is performed to achieve a desired serum bicarbonate level. In one embodiment, the dose adjustment is performed at about 2 weeks intervals.

In one embodiment, the crosslinked poly(allylamine) polymer of the present invention is administered with food. In one embodiment, the crosslinked poly(allylamine) polymer of the present invention is administered orally as a suspension in water.

The effectiveness of the crosslinked poly(allylamine) polymer may be established in animal models, or in human volunteers and patients. In addition, *in vitro, ex vivo* and *in vivo* approaches are useful to establish HCl or other target species binding. *In vitro* binding solutions can be used to measure the binding capacity for proton, chloride and other ions at different pHs. *Ex vivo* extracts, such as the gastrointestinal lumen contents from human volunteers or from model animals can be used for similar purposes. The selectivity of binding and/or retaining certain ions preferentially over others can also be demonstrated in such *in vitro* and *ex vivo* solutions. *In vivo* models of metabolic acidosis can be used to test the effectiveness of the crosslinked poly(allylamine) polymer in normalizing acid/base balance - for example 5/6 nephrectomized rats fed casein-containing chow (as described in Phisitkul S, Hacker C, Simoni J, Tran RM, Wesson DE. Dietary protein causes a decline in the glomerular filtration rate of the remnant kidney mediated by metabolic acidosis and endothelin receptors. Kidney international. 2008;73(2):192-9), or adenine-fed rats (Terai K, K Mizukami and M Okada. 2008. Comparison of chronic renal failure rats and modification of the preparation protocol as a hyperphosphatemia model. Nephrol. 13: 139-146).

Metabolic acidosis, regardless of etiology, lowers extracellular fluid bicarbonate and, thus, decreases extracellular pH. The relationship between serum pH and serum bicarbonate is described by the Henderson-Hasselbalch equation

$$pH = pK' + \log [HCO_3^-]/[(0.03X\ PaCO_2)]$$

where 0.03 is the physical solubility coefficient for $CO_2$, $[HCO_3^-]$ and $PaCO_2$ are the concentrations of bicarbonate and the partial pressure of carbon dioxide, respectively.

There are several laboratory tests that can be used to define metabolic acidosis. The tests fundamentally measure either bicarbonate ($HCO_3^-$) or proton ($H^+$) concentration in various biological samples, including venous or arterial blood. These tests can measure either bicarbonate ($HCO_3^-$) or proton ($H^+$) concentration by enzymatic methodology, by ion selective electrodes or by blood gas analysis. In both the enzymatic and ion selective electrode methods, bicarbonate is "measured." Using blood gas analysis, bicarbonate level can be calculated using the Henderson-Hasselbalch equation.

In one embodiment, the crosslinked poly(allylamine) polymers are provided (by oral administration) to an animal, including a human, in a dosing regimen of one, two or even multiple *(i.e.,* at least three) doses per day to treat an acid-base disorder (e.g., metabolic acidosis) and achieve a sustained increase of serum bicarbonate or other target species as previously described.

The crosslinked poly(allylamine) polymer disclosed herein may be provided in any form appropriate for oral administration. Such forms include powders, tablets, pills, lozenges, sachets, cachets, elixirs, suspensions, syrups, gels, soft or hard gelatin capsules, and the like. In one embodiment, the pharmaceutical composition comprises only the crosslinked poly(allylamine) polymer. Alternatively, the pharmaceutical composition may comprise a carrier, a diluent, or excipient in addition to the crosslinked poly(allylamine) polymer. Examples of carriers, excipients, and diluents that may be used in these formulations as well as others, include foods, drinks, lactose, dextrose, sucrose, sorbitol, mannitol, starches, gum acacia, alginates, tragacanth, gelatin, calcium silicate, microcrystalline cellulose, polyvinylpyrrolidone, cellulose, methyl cellulose, methylhydroxybenzoates, propylhydroxybenzoates, propylhydroxybenzoates, and talc. Pharmaceutical excipients useful in the pharmaceutical compositions further include a binder, such as microcrystalline cellulose, colloidal silica and combinations thereof (Prosolv 90), carbopol, providone and xanthan gum; a flavoring agent, such as sucrose, mannitol, xylitol, maltodextrin, fructose, or sorbitol; a lubricant, such as magnesium stearate, stearic acid, sodium stearyl fumurate and vegetable based fatty acids; and, optionally, a disintegrant, such as croscarmellose sodium, gellan gum, low-substituted hydroxypropyl ether of cellulose, sodium starch glycolate. Other additives may include plasticizers, pigments, talc, and the like. Such additives and other suitable ingredients are well-known in the art; see, e.g., Gennaro A R (ed), Remington's Pharmaceutical Sciences, 20th Edition.

In one embodiment, the crosslinked poly(allylamine) polymer may be co-administered with other active pharmaceutical agents depending on the condition being treated. This co-administration may include simultaneous administration of the two agents in the same dosage form, simultaneous administration in separate dosage forms, and separate administration. For example, for the treatment of metabolic acidosis, the crosslinked poly(allylamine) polymer may be co-administered with common treatments that are required to treat underlying co-morbidities including but not limited to hypertension, diabetes, obesity, heart failure and complications of Chronic Kidney Disease. These medications and the crosslinked poly(allylamine) polymer can be formulated together in the same dosage form and administered simultaneously as long as they do not display any clinically significant drug-drug-interactions. Alternatively, these treatments and the crosslinked poly(allylamine) polymer may be separately and sequentially administered with the administration of one being followed by the administration of the other.

In one embodiment, the daily dose of the chronic metabolic acidosis treatment is compliance enhancing (3 g/day, 6 g/day or 9 g/day of the crosslinked poly(allylamine) polymer of the present invention) and achieves an increase of serum bicarbonate of approximately 3 mEq/L at these daily doses. The non-absorbed nature of the polymer and the lack of sodium load and/or introduction of other deleterious ions for such an oral drug enable for the first time a safe, chronic treatment of metabolic acidosis without worsening blood pressure / hypertension and/or without causing increased fluid retention and fluid overload. Another benefit is further slowing of the progression of kidney disease and time to onset of lifelong renal replacement therapy (End Stage Renal Disease "ESRD" including 3 times a week dialysis) or need for kidney transplants. Both are associated with significant mortality, low quality of life and significant burden to healthcare systems around the world. In the United States alone, approximately 20 % of the 400,000 ESRD patients die and 100,000 new patients start dialysis every year.

A further aspect of the present disclosure is a pharmaceutical product comprising a sealed package and the crosslinked poly(allylamine) polymer of the present invention within the sealed package. The unit dosage forms disclosed herein could take any form consistent with one or more of the requirements disclosed. For example, the unit dosage form could include a vial, bottle, tube, jar, box, tub, blister pack, sachet (including a stick pack) or other sealed container. In one exemplary embodiment, the unit dosage form is a sachet.

As disclosed herein, the crosslinked poly(allylamine) polymer of the present invention may generate certain impurities when exposed to oxygen, e.g. $H_2C{=}CHCH_2NH_2$. The unit dosage form may include features to address or reduce such potential impurities, e.g. feature to reducing the amount of oxygen that comes into contact with the polymer

during storage.

Packaging approaches to address or reduce such potential impurities include the following: a unit dosage form with low permeability to oxygen; a unit dosage form with a low amount of gas present inside the unit dosage form; a unit dosage form containing gasses other than air inside the unit dosage form and/or a unit dosage form comprising an oxygen absorbing component, e.g. a scavenger. Each packaging approach can be used alone or in combination with any other packaging approach disclosed herein. Each packaging approach, alone or in combination, can be used with a crosslinked poly(allylamine) polymer of the present invention and all such combinations are disclosed. For example, one or more of the packaging approaches disclosed herein to address or reduce potential impurities, e.g. the approaches summarized in this paragraph, could be used to provide a pharmaceutical product comprising the crosslinked poly(allylamine) polymer of the present invention, with low levels of sp2 carbons as disclosed herein, (less than 1.0% of the total number of carbon atoms present in the crosslinked poly(allylamine) polymer are $sp^2$ allyl carbons). Such a pharmaceutical product may provide acceptable or improved shelf life as defined anywhere herein.

In some embodiments, the unit dosage form comprises a sealed package that is preferably substantially impermeable to moisture and oxygen to increase the stability of the pharmaceutical composition (e.g. the stability of veverimer). For example, the dosage unit form may comprise a sealed container (e.g., a sealed sachet) that prevents or reduces ingress of moisture and oxygen upon packaging the poly(allylamine) polymer in the container.

The container size used to form the unit dosage form, and/or the amount of pharmaceutical composition in the container can be selected to reduce the head space in the container after packing. The head space is the volume contained within the unit dosage form which is not the pharmaceutical composition. The head space comprises one or more gases, for example an inert gas such as nitrogen or a mixture of gases such as air. By minimising the volume of the head space the stability of the crosslinked poly(allylamine) polymer of the present invention, can be increased during storage, in particular when the head space comprises oxygen.

In some embodiments the unit dosage form comprises the pharmaceutical composition and the head space, wherein the head space volume is from 0 $cm^3$ to 120 $cm^3$. In some embodiments the unit dosage form comprises the pharmaceutical composition and the head space, wherein the head space volume is from 10 $cm^3$ to 110 $cm^3$. In some embodiments the unit dosage form comprises the pharmaceutical composition and the head space, wherein the head space volume is from 20 $cm^3$ to 100 $cm^3$. In some embodiments the unit dosage form comprises the pharmaceutical composition and the head space, wherein the head space volume is from 20 $cm^3$ to 40 $cm^3$. In some embodiments the unit dosage form comprises the pharmaceutical composition and the head space, wherein the head space volume is from 0 $cm^3$ to 20 $cm^3$. In some embodiments the unit dosage form comprises the pharmaceutical composition and the head space, wherein the head space volume is from 50 $cm^3$ to 70 $cm^3$.

In some embodiments the unit dosage form comprises the pharmaceutical composition and the head space, wherein the head space volume is less than 70% of the total volume of the unit dosage form. In some embodiments the unit dosage form comprises the pharmaceutical composition and the head space, wherein the head space volume is less than 65% of the total volume of the unit dosage form. In some embodiments the unit dosage form comprises the pharmaceutical composition and the head space, wherein the head space volume is less than 60% of the total volume of the unit dosage form. In some embodiments the unit dosage form comprises the pharmaceutical composition and the head space, wherein the head space volume is less than 55% of the total volume of the unit dosage form. In some embodiments the unit dosage form comprises the pharmaceutical composition and the head space, wherein the head space volume is less than 45% of the total volume of the unit dosage form. In some embodiments the unit dosage form comprises the pharmaceutical composition and the head space, wherein the head space volume is less than 35% of the total volume of the unit dosage form. In some embodiments the unit dosage form comprises the pharmaceutical composition and the head space, wherein the head space volume is less than 25% of the total volume of the unit dosage form. In some embodiments the unit dosage form comprises the pharmaceutical composition and the head space, wherein the head space volume is less than 15% of the total volume of the unit dosage form. In some embodiments the unit dosage form comprises the pharmaceutical composition and the head space, wherein the head space volume is less than 5% of the total volume of the unit dosage form.

In some embodiments the head space is minimised by compressing the unit dosage form prior to the sealing of the pharmaceutical composition inside the unit dosage form.

A unit dosage form having a relatively low head space will have a relatively high number of grams of pharmaceutical composition relative to its head space volume. In some embodiments, the unit dosage form comprises the pharmaceutical composition, wherein the pharmaceutical composition is present in an amount of at least 0.01 g per $cm^3$ of the head space volume of the unit dosage form. In some embodiments, the unit dosage form comprises the pharmaceutical composition, wherein the pharmaceutical composition is present in an amount of at least 0.02 g per $cm^3$ of the head space volume of the unit dosage form. In some embodiments, the unit dosage form comprises the pharmaceutical composition, wherein the pharmaceutical composition is present in an amount of at least 0.03 g per $cm^3$ of the head space volume of the unit dosage form. In some embodiments, the unit dosage form comprises the pharmaceutical composition, wherein the pharmaceutical composition is present in an amount of at least 0.04 g per $cm^3$ of the head space

volume of the unit dosage form. In some embodiments, the unit dosage form comprises the pharmaceutical composition, wherein the pharmaceutical composition is present in an amount of at least 0.05 g per $cm^3$ of the head space volume of the unit dosage form. In some embodiments, the unit dosage form comprises the pharmaceutical composition, wherein the pharmaceutical composition is present in an amount of from 0.01 g to 0.5 g per $cm^3$ of the head space volume of the unit dosage form. In some embodiments, the unit dosage form comprises the pharmaceutical composition, wherein the pharmaceutical composition is present in an amount of from 0.01 g to 0.2 g per $cm^3$ of the head space volume of the unit dosage form. In some embodiments, the unit dosage form comprises the pharmaceutical composition, wherein the pharmaceutical composition is present in an amount of from 0.05 g to 0.2 g per $cm^3$ of the head space volume of the unit dosage form. In some embodiments, the unit dosage form comprises the pharmaceutical composition, wherein the pharmaceutical composition is present in an amount of from 0.05 g to 0.15 g per $cm^3$ of the head space volume of the unit dosage form.

In some embodiments the unit dosage form comprises the pharmaceutical composition, wherein the pharmaceutical composition is present in an amount of 3 g and wherein the head space volume of the unit dosage form is less than 90 $cm^3$. In some embodiments the unit dosage form comprises the pharmaceutical composition, wherein the pharmaceutical composition is present in an amount of 3 g and wherein the head space volume of the unit dosage form is less than 75 $cm^3$. In some embodiments the unit dosage form comprises the pharmaceutical composition, wherein the pharmaceutical composition is present in an amount of 3 g and wherein the head space volume of the unit dosage form is less than 60 $cm^3$. In some embodiments the unit dosage form comprises the pharmaceutical composition, wherein the pharmaceutical composition is present in an amount of 3 g and wherein the head space volume of the unit dosage form is less than 45 $cm^3$.

In some embodiments the unit dosage form comprises the pharmaceutical composition, wherein the pharmaceutical composition is present in an amount of 6 g and wherein the head space volume of the unit dosage form is less than 120 $cm^3$. In some embodiments the unit dosage form comprises the pharmaceutical composition, wherein the pharmaceutical composition is present in an amount of 6 g and wherein the head space volume of the unit dosage form is less than 105 $cm^3$. In some embodiments the unit dosage form comprises the pharmaceutical composition, wherein the pharmaceutical composition is present in an amount of 6 g and wherein the head space volume of the unit dosage form is less than 90 $cm^3$. In some embodiments the unit dosage form comprises the pharmaceutical composition, wherein the pharmaceutical composition is present in an amount of 6 g and wherein the head space volume of the unit dosage form is less than 75 $cm^3$.

In some embodiments the unit dosage form comprises the pharmaceutical composition, wherein the pharmaceutical composition is present in an amount of 9 g and wherein the head space volume of the unit dosage form is less than 140 $cm^3$. In some embodiments the unit dosage form comprises the pharmaceutical composition, wherein the pharmaceutical composition is present in an amount of 9 g and wherein the head space volume of the unit dosage form is less than 125 $cm^3$. In some embodiments the unit dosage form comprises the pharmaceutical composition, wherein the pharmaceutical composition is present in an amount of 9 g and wherein the head space volume of the unit dosage form is less than 110 $cm^3$. In some embodiments the unit dosage form comprises the pharmaceutical composition, wherein the pharmaceutical composition is present in an amount of 9 g and wherein the head space volume of the unit dosage form is less than 95 $cm^3$.

A sealed unit dosage form having a relatively low head space will have a relatively high number of grams of pharmaceutical composition relative to its total volume. In some embodiments, the unit dosage form comprises the pharmaceutical composition, wherein the pharmaceutical composition is present in an amount of at least 0.02 g per $cm^3$ of the total volume of the sealed unit dosage form. In some embodiments, the unit dosage form comprises the pharmaceutical composition, wherein the pharmaceutical composition is present in an amount of at least 0.03 g per $cm^3$ of the total volume of the sealed unit dosage form. In some embodiments, the unit dosage form comprises the pharmaceutical composition, wherein the pharmaceutical composition is present in an amount of at least 0.04 g per $cm^3$ of the total volume of the sealed unit dosage form. In some embodiments, the unit dosage form comprises the pharmaceutical composition, wherein the pharmaceutical composition is present in an amount of from 0.01 g to 0.5 g per $cm^3$ of the total volume of the sealed unit dosage form. In some embodiments, the unit dosage form comprises the pharmaceutical composition, wherein the pharmaceutical composition is present in an amount of from 0.01 g to 0.25 g per $cm^3$ of the total volume of the sealed unit dosage form. In some embodiments, the unit dosage form comprises the pharmaceutical composition, wherein the pharmaceutical composition is present in an amount of from 0.01 g to 0.15 g per $cm^3$ of the total volume of the sealed unit dosage form. In some embodiments, the unit dosage form comprises the pharmaceutical composition, wherein the pharmaceutical composition is present in an amount of from 0.02 g to 0.1 g per $cm^3$ of the total volume of the sealed unit dosage form. In some embodiments, the unit dosage form comprises the pharmaceutical composition, wherein the pharmaceutical composition is present in an amount of from 0.03 g to 0.08 g per $cm^3$ of the total volume of the sealed unit dosage form. In some embodiments, the unit dosage form comprises the pharmaceutical composition, wherein the pharmaceutical composition is present in an amount of from 0.04 g to 0.07 g per $cm^3$ of the total volume of the sealed unit dosage form. In some embodiments, the unit dosage form comprises the pharmaceutical composition, wherein the

pharmaceutical composition is present in an amount of from 0.02 g to 0.2 g per $cm^3$ of the total volume of the sealed unit dosage form. In some embodiments, the unit dosage form comprises the pharmaceutical composition, wherein the pharmaceutical composition is present in an amount of from 0.04 g to 0.18 g per $cm^3$ of the total volume of the sealed unit dosage form. In some embodiments, the unit dosage form comprises the pharmaceutical composition, wherein the pharmaceutical composition is present in an amount of from 0.06 g to 0.16 g per $cm^3$ of the total volume of the sealed unit dosage form.

In some embodiments the unit dosage form comprises the pharmaceutical composition, wherein the pharmaceutical composition is present in an amount of 3 g and wherein the sealed total volume of the unit dosage form is less than 100 $cm^3$. In some embodiments the unit dosage form comprises the pharmaceutical composition, wherein the pharmaceutical composition is present in an amount of 3 g and wherein the sealed total volume of the unit dosage form is less than 90 $cm^3$. In some embodiments the unit dosage form comprises the pharmaceutical composition, wherein the pharmaceutical composition is present in an amount of 3 g and wherein the sealed total volume of the unit dosage form is less than 80 $cm^3$. In some embodiments the unit dosage form comprises the pharmaceutical composition, wherein the pharmaceutical composition is present in an amount of 3 g and wherein the sealed total volume of the unit dosage form is less than 70 $cm^3$.

In some embodiments the unit dosage form comprises the pharmaceutical composition, wherein the pharmaceutical composition is present in an amount of 6 g and wherein the total volume of the sealed unit dosage form is less than 160 $cm^3$. In some embodiments the unit dosage form comprises the pharmaceutical composition, wherein the pharmaceutical composition is present in an amount of 6 g and wherein the total volume of the sealed unit dosage form is less than 150 $cm^3$. In some embodiments the unit dosage form comprises the pharmaceutical composition, wherein the pharmaceutical composition is present in an amount of 6 g and wherein the total volume of the sealed unit dosage form is less than 140 $cm^3$. In some embodiments the unit dosage form comprises the pharmaceutical composition, wherein the pharmaceutical composition is present in an amount of 6 g and wherein the total volume of the sealed unit dosage form is less than 130 $cm^3$.

In some embodiments the unit dosage form comprises the pharmaceutical composition, wherein the pharmaceutical composition is present in an amount of 9 g and wherein the total volume of the sealed unit dosage form is less than 200 $cm^3$. In some embodiments the unit dosage form comprises the pharmaceutical composition, wherein the pharmaceutical composition is present in an amount of 9 g and wherein the total volume of the sealed unit dosage form is less than 190 $cm^3$. In some embodiments the unit dosage form comprises the pharmaceutical composition, wherein the pharmaceutical composition is present in an amount of 9 g and wherein the total volume of the sealed unit dosage form is less than 180 $cm^3$. In some embodiments the unit dosage form comprises the pharmaceutical composition, wherein the pharmaceutical composition is present in an amount of 9 g and wherein the sealed total volume of the unit dosage form is less than 170 $cm^3$.

In some embodiments the unit dosage form is a sachet. In such embodiments the sachet has a height (h) and a width (w) and the walls of the sachet can bend but not stretch, the total volume of the sealed sachet can be estimated according to Formula X:

$$V = w^3\left(\frac{h}{\pi w} - 0.142\left(1 - 10^{(-h/w)}\right)\right) \qquad \text{Formula X.}$$

Formula X can be used to define a total volume of the sealed unit dosage form, when the dosage form is a sachet. For example, when the unit dosage form is a sachet, any description of an amount of drug in grams per $cm^3$ of the total volume refers to the amount of drug in grams per $cm^3$ of the sealed total volume as estimated by Formula X.

In some embodiments, the unit dosage form is a sachet comprising the pharmaceutical composition in an amount of 3 g, 6 g, or 9 g and wherein the width of the sachet is less than 25 cm and the height of the sachet is less than 25 cm. In some embodiments, the unit dosage form is a sachet comprising the pharmaceutical composition in an amount of 3 g, 6 g, or 9 g and wherein the width of the sachet is less than 20 cm and the height of the sachet is less than 25 cm. In some embodiments, the unit dosage form is a sachet comprising the pharmaceutical composition in an amount of 3 g, 6 g, or 9 g and wherein the width of the sachet is less than 15 cm and the height of the sachet is less than 20 cm. In some embodiments, the unit dosage form is a sachet comprising the pharmaceutical composition in an amount of 3 g, 6 g, or 9 g and wherein the width of the sachet is less than 10 cm and the height of the sachet is less than 15 cm.

In some embodiments, the unit dosage form is a sachet comprising the pharmaceutical composition in an amount of 9 g and wherein the width of the sachet is less than 25 cm and the height of the sachet is less than 25 cm. In some embodiments, the unit dosage form is a sachet comprising the pharmaceutical composition in an amount of 9 g and wherein the width of the sachet is less than 20 cm and the height of the sachet is less than 25 cm. In some embodiments, the unit dosage form is a sachet comprising the pharmaceutical composition in an amount of 9 g and wherein the width of the sachet is less than 15 cm and the height of the sachet is less than 20 cm. In some embodiments, the unit dosage form is a

sachet comprising the pharmaceutical composition in an amount of 9 g and wherein the width of the sachet is less than 10 cm and the height of the sachet is less than 15 cm. In some embodiments, the unit dosage form is a sachet comprising the pharmaceutical composition in an amount of 9 g and wherein the width of the sachet is less than 9 cm and the height of the sachet is less than 12 cm.

In some embodiments, the unit dosage form is a sachet comprising the pharmaceutical composition in an amount of 6 g and wherein the width of the sachet is less than 25 cm and the height of the sachet is less than 25 cm. In some embodiments, the unit dosage form is a sachet comprising the pharmaceutical composition in an amount of 6 g and wherein the width of the sachet is less than 20 cm and the height of the sachet is less than 25 cm. In some embodiments, the unit dosage form is a sachet comprising the pharmaceutical composition in an amount of 6 g and wherein the width of the sachet is less than 15 cm and the height of the sachet is less than 20 cm. In some embodiments, the unit dosage form is a sachet comprising the pharmaceutical composition in an amount of 6 g and wherein the width of the sachet is less than 10 cm and the height of the sachet is less than 15 cm. In some embodiments, the unit dosage form is a sachet comprising the pharmaceutical composition in an amount of 6 g and wherein the width of the sachet is less than 8 cm and the height of the sachet is less than 11 cm.

In some embodiments, the unit dosage form is a sachet comprising the pharmaceutical composition in an amount of 3 g and wherein the width of the sachet is less than 25 cm and the height of the sachet is less than 25 cm. In some embodiments, the unit dosage form is a sachet comprising the pharmaceutical composition in an amount of 3 g and wherein the width of the sachet is less than 20 cm and the height of the sachet is less than 25 cm. In some embodiments, the unit dosage form is a sachet comprising the pharmaceutical composition in an amount of 3 g and wherein the width of the sachet is less than 15 cm and the height of the sachet is less than 20 cm. In some embodiments, the unit dosage form is a sachet comprising the pharmaceutical composition in an amount of 3 g and wherein the width of the sachet is less than 10 cm and the height of the sachet is less than 15 cm. In some embodiments, the unit dosage form is a sachet comprising the pharmaceutical composition in an amount of 3 g and wherein the width of the sachet is less than 9 cm and the height of the sachet is less than 10 cm. In some embodiments, the unit dosage form is a sachet comprising the pharmaceutical composition in an amount of 3 g and wherein the width of the sachet is less than 8 cm and the height of the sachet is less than 9 cm.

Any head space present in any unit dosage form disclosed herein may be filled with an inert gas such as nitrogen, or may otherwise include reduced levels of oxygen gas. The head space present in the unit dosage form can contain any gas, and herein the gas present in the head space is referred to as the head space gas. In some embodiments, the head space gas is nitrogen. In some embodiments, the head space gas is argon. In some embodiments, the head space gas is helium. In some embodiments, the head space gas is neon. In some embodiments, the head space gas is carbon dioxide. In some embodiments, the head space gas is nitrogen. In some embodiments the head space gas is a mixture of gases mentioned herein. For example, in some embodiments, the head space gas is a mixture of inert gases, e.g. a mixture of nitrogen and carbon dioxide. In some embodiments, a reference to the head space gas being a particular gas means that the head space gas consists essentially of or consists of the recited gas.

In some embodiments, the head space gas includes other gasses, such as oxygen, that are not inert. In some embodiments, the percentage of oxygen present in the head space gas is less than 21%. In some embodiments, the percentage of oxygen present in the head space gas is less than 20%. In some embodiments, the percentage of oxygen present in the head space gas is less than 18%. In some embodiments, the percentage of oxygen present in the head space gas is less than 16%. In some embodiments, the percentage of oxygen present in the head space gas is less than 14%. In some embodiments, the percentage of oxygen present in the head space gas is less than 12%. In some embodiments, the percentage of oxygen present in the head space gas is less than 10%. In some embodiments, the percentage of oxygen present in the head space gas is less than 8%. In some embodiments, the percentage of oxygen present in the head space gas is less than 6%. In some embodiments, the percentage of oxygen present in the head space gas is less than 4%. In some embodiments, the percentage of oxygen present in the head space gas is less than 2%. In some embodiments, the percentage of oxygen present in the head space gas is less than 1%. In some embodiments, the percentage of oxygen present in the head space gas is less than 0.5%.

In some embodiments, the the head space gas is a mixture of oxygen and a second gas. In some embodiments the head space gas is ≤20% oxygen ($O_2$) and ≥80% nitrogen ($N_2$). In some embodiments the head space gas is ≤18% $O_2$ and ≥82% $N_2$. In some embodiments the head space gas is ≤16% $O_2$ and ≥84% $N_2$. In some embodiments the head space gas is ≤14% $O_2$ and ≥86% $N_2$. In some embodiments the head space gas is ≤12% $O_2$ and ≥88% $N_2$. In some embodiments the head space gas is ≤10% $O_2$ and ≥90% $N_2$. In some embodiments the head space gas is ≤8% $O_2$ and ≥92% $N_2$. In some embodiments the head space gas is ≤6% $O_2$ and ≥94% $N_2$. In some embodiments the head space gas is ≤4% $O_2$ and ≥96% $N_2$. In some embodiments the head space gas is <2% $O_2$ and ≥98% $N_2$. In some embodiments the head space gas is ≤1% $O_2$ and ≥99% $N_2$. In some embodiments the head space gas is ≤0.5% $O_2$ and ≥99.5% $N_2$. In such embodiments, the head space gas may consist essentially of or consist of the recited gasses.

In other embodiments, the unit dosage forms disclosed herein are packaged in air and/or the head space in the unit dosage form is air. The head space gas can be achieved by packaging in the presence of that gas. The head space gas

could also be varied after the packaging step, e.g. by adding or changing the gas inside the pharmaceutical dosage form after manufacture, e.g. via a valve in the packaging.

Container material of construction used for the unit dosage forms disclosed herein can be chosen to minimize the moisture and oxygen ingress inside the container after packaging. For example, poly(allylamine) polymer of the present invention may be packaged in a multilayer sachet containing at least one or more layer that serves as a barrier layer to moisture and oxygen ingress. In another example, the poly(allylamine) polymer may be packaged in a single layer or multilayer plastic, metal or glass container that has at least one or more barrier layers incorporated in the structure that limits oxygen and/or moisture ingress after packaging. For example, in one such embodiment the sachet (or other container or package) may comprise a multi-layer laminate of an inner contact layer, an outer layer; and a barrier layer disposed between the contact layer and outer layer. In such embodiments, the multi-layer laminate may also comprise one or more adhesive layers and/or a print layer. For example, the laminate may comprise the following layers in the following order: an inner contact layer, an adhesive, a barrier layer, an adhesive, a print layer and an outer layer. In some embodiments, one or more of the layers of any multi-laminate disclosed herein may be attached to each other via an adhesive and/or lamination (e.g. lamination by extrusion). In some embodiments, the layers of any multi-laminate disclosed herein are attached to each other via an adhesive and/or lamination (e.g. lamination by extrusion). In some embodiments, the layers of any multi-laminate disclosed herein are be attached to each other via an adhesive. In some embodiments, the layers of any multi-laminate disclosed herein are attached to each other via lamination (e.g. lamination by extrusion).

In one embodiment, the unit dosage form is made from, or comprises, a plastic. For example, the plastic may be polyethylene (e.g. linear low-density polyethylene or low-density polyethylene), polypropylene, poly(ethylene terephthalate), polyester, nylon or polyvinyl chloride. In such embodiments, the plastic may be in the form of a film or sheet. For example, when the unit dosage form is a sachet, the sachet may be made out of any one of polyethylene, polypropylene, poly(ethylene terephthalate), polyester, nylon or polyvinyl chloride, optionally as part of a multilayer laminate. In one embodiment, the sachet (or other container or package) comprises a multi-layer laminate that comprises an inner contact layer, an outer layer; and a barrier layer disposed between the contact layer and outer layer. In such embodiments, any of the layers may be made from any of the recited plastics. More specifically, the inner contact layer may comprise any one of polyethylene, polypropylene, poly(ethylene terephthalate), polyester, nylon or polyvinyl chloride. In one embodiment, the inner contact layer comprises polyethylene. In one embodiment, the inner contact layer is linear low-density polyethylene. In one embodiment, the inner contact layer is low-density polyethylene. More specifically, the outer layer may comprise any one of polyethylene, polypropylene, poly(ethylene terephthalate), polyester, nylon, polyvinyl chloride or paper. In one embodiment, the outer layer is poly(ethylene terephthalate). In one embodiment, the barrier layer may be any aluminum layer disclosed herein. In one embodiment the barrier layer may include any plastic layer disclosed herein and any aluminum layer disclosed herein. For example, the barrier layer may comprise low-density polyethylene and aluminium film. In one exemplary embodiment, the unit dosage form disclosed herein is a sachet made from a laminate of the following components in the following order: an inner contact layer made from low-density polyethylene, a barrier layer made from aluminium film followed by low-density polyethylene, a print layer made from ink and an outer layer made from poly(ethylene terephthalate), optionally with adhesives between any of these layers, optionally wherein the aluminium film is about 18um thick. In one exemplary embodiment, the unit dosage form disclosed herein is a sachet made from a laminate of the following components in the following order: an inner contact layer made from low-density polyethylene, a barrier layer made from aluminum film followed by low-density polyethylene, a print layer made from ink and an outer layer made from poly(ethylene terephthalate), optionally where those layers are laminated together by extrusion, optionally wherein the aluminum film is about 18um thick.

In one aspect, the unit dosage form has an exterior and interior, the interior comprising the crosslinked poly(allylamine) polymer of the present invention wherein the oxygen transfer rate between the exterior and interior of the unit dosage form is less than about 0.050 cubic centimeters per meter squared per day (CC/$m^2$/day).

In one aspect, the unit dosage form comprises a sealed enclosure containing the crosslinked poly(allylamine) polymer of the present invention the sealed enclosure comprising a multi-layer laminate of an inner contact layer, an outer layer; and a barrier layer disposed between the contact layer and outer layer, wherein the oxygen transfer rate between the multi-layer laminate is less than about 0.050 cubic centimetes per meter squared per day (CC/$m^2$/day).

In any aspect, the oxygen transfer rate specified is, in some embodiments, less than about 0.030 CC/$m^2$/day. In any aspect, the oxygen transfer rate specified is, in some embodiments, less than about 0.010 CC/$m^2$/day. In any aspect, the oxygen transfer rate specified is, in some embodiments, less than about 0.009 CC/$m^2$/day. In any aspect, the oxygen transfer rate specified is, in some embodiments, less than about 0.007 CC/$m^2$/day. In any aspect, the oxygen transfer rate specified is, in some embodiments, less than about 0.005 CC/$m^2$/day. In any aspect, the oxygen transfer rate specified is, in some embodiments, less than about 0.003 CC/$m^2$/day.

Where the oxygen transfer rate is specified for a unit dosage form disclosed herein, that transfer rate can be controlled by selecting appropriate materials, and an appropriate thicknesses of those materials, when constructing the unit dosage form. Materials can that be used to control the oxygen transfer rate of the unit dosage form include aluminum,

ethylene vinyl alcohol, glass, polyester (e.g. polyethylene terephthalate) and polyamides (e.g. nylon). One or more of these materials may be used to achieve the specified oxygen transfer rate.

For example, one or more layers of aluminum can be used to control oxygen transfer rates. Therefore, in one embodiment, the unit dosage form disclosed herein comprises at least one aluminum layer. For example, the layer comprising aluminum may be part of the barrier layer as described herein. More specifically, the layer comprising aluminum may be the barrier layer. For example, the barrier layer may comprise any of the aluminum layers disclosed herein. For example, the barrier layer may consist essentially of any of the aluminum layers disclosed herein. For example, the barrier layer may consist of any of the aluminum layers disclosed herein. In some embodiments, barrier layer and/or the aluminum layer has any one of the oxygen transfer rates disclosed herein when measured alone.

In one embodiment, the thickness of the aluminum layer disclosed herein is greater than 5 μm. In one embodiment, the thickness of the aluminum layer disclosed herein is greater than 8 μm. In one embodiment, the thickness of the aluminum layer disclosed herein is greater than 10 μm. In one embodiment, the thickness of the aluminum layer disclosed herein is greater than 12 μm. In one embodiment, the thickness of the aluminum layer disclosed herein is greater than 15 μm. In one embodiment, the thickness of the aluminum layer disclosed herein is greater than 18 μm. In one embodiment, the thickness of the aluminum layer disclosed herein is greater than 20 μm. In one embodiment, the thickness of the aluminum layer disclosed herein is greater than 25 μm. In one embodiment, the thickness of the aluminium layer is 9 - 20 μm. In one embodiment, the thickness of the aluminium layer is 15 - 20 μm. In one embodiment, the thickness of the aluminium layer is about 18 μm.

In any aspect, the unit dosage forms are made from one consistent material, for example a sachet made from a single type of sheet or multi laminate. In some embodiments, the unit dosage form may comprise different materials at different parts (e.g. ends) of the unit dosage form. In such scenarios, the requirements specified herein for the unit dosage form may apply to one or more of such parts. Or, in such scenarios, the requirements specified herein for the unit dosage form may apply to substantially all of such parts. Or, in such scenarios the requirements specified herein for the unit dosage form may apply to all of such parts. Therefore, for example, unit dosage forms disclosed herein include sealed vials (e.g. made from plastic or glass) sealed by a lid, where both parts (the lid and the vial) have an oxygen transfer rate as specified anywhere herein, e.g. less than 0.005 $CC/m^2$/day. In such an example, the closed vial may have an oxygen transfer rate between the exterior and interior of the unit dosage form that is as specified anywhere herein, e.g. less than 0.005 $CC/m^2$/day.

In any aspect, the unit dosage form may comprise layers that form the dosage form, for example the inner contact layer, the outer layer and the barrier layer and potentially further layers. Any of these layers may alone, or together, be responsible for the specified oxygen transfer rate. The layers may be attached together using adhesives, heat sealing, extrusion lamination, or any other fastening method.

In one embodiment, the unit dosage form comprises an oxygen scavenger. In one exemplary embodiment, the unit dosage form includes one or more oxygen-scavenging layers comprising an oxygen scavenger. In one embodiment, the unit dosage form comprises an oxygen scavenger that is in contact with the crosslinked poly(allylamine) polymer, e.g. as an excipient. In one embodiment the oxygen scavenger is present in the interior of the unit dosage form, e.g. in a separate insert. In one embodiment, the oxygen scavenger is in communication with the interior of the unit dosage form, e.g. formed into the lid of the unit dosage form.

The oxygen scavenger as disclosed herein could be any material that absorbs oxygen. In one embodiment, the oxygen scavenger is any material that can be oxidized under ambient conditions. In one embodiment, the oxygen scavenger is one or more of the following: iron (e.g. iron powder or activated iron), ferrous oxide, iron oxide powders, ferrous salts such as ferrous sulfate or ferrous chloride, sulfites, bisulfites, reducing sulfur compounds such as dithionite, ascorbic acid and/or their salts, Pd, Cu, ZN, Mg, Mn, Co(II), Zn, ascorbic acid, ascorbic acid salts, isoascorbic acid, tocopherol, hydroquinone, catechol, rongalit, sorbose, lignin, gallic acid, gallic acid and potassium carbonate, erythorbic acid, quinones, catechol, butylated hydroxytoluene (BHT), butylated hydroxyanisole (BHA), polyunsaturated fatty acids, glucose oxidase, laccase, and ethanol oxidase. Further oxygen scavengers are available, e.g. U.S. Patent Publication Nos. 20060076536,20070084144 and 20060260967. Oxygen scavengers are commercially available e.g., StabilOx® (MultisorbTechnologies), cyclohexene methyl acrylated (EMCM) polymer (Chevron-Phillips Chemical Company) or Ciba's Specialty Chemical's SHELFPLUS.TM.). In one exemplary embodiment the scavenger is iron.

In one embodiment, the oxygen scavenger the unit dosage form includes one or more oxygen-scavenging layers comprising an oxygen scavenger. Such layers are known in the art, for example see Gaikwad et al., Environmental Chemistry Letters volume 16, pages 523-538(2018). In one embodiment, the unit dosage forms disclosed herein comprise a layer (film) as disclosed in Gaikwad et al., specifically any one of those disclosed in Table 2 of Gaikwad et al., or combinations thereof. In one embodiment, the unit dosage forms disclosed herein comprise a layer (film) as disclosed in Table 3 of Gaikwad et al., or combinations thereof. In one embodiment, the unit dosage forms disclosed herein comprise a layer (film) as disclosed in Gaikwad et al., specifically any one of those disclosed in the "multilayer active films" section, or combinations thereof. Indeed, such layers are already commercially available, for example the unit dosage form disclosed herein may comprise Oxy Vanish-Dry Film (Mitsubishi), Ageless Omac (Mitsubishi), Activ-FilmsTM (Sorbead India),

Oxbar (Crown, Cork and Seal), Amosorb 3000 (AMOCO), Self Plus (Ciba Specialty Chemicals), ZERO2 (Visy Industries) and OS1000 (Cryovac Sealed Air). In one exemplary embodiment, the unit dosage form disclosed herein comprises Oxy Vanish-Dry Film (Mitsubishi).

A further aspect of the present disclosure is a product comprising a plurality of unit dosage forms disclosed herein. The product may additionally comprise one or more oxygen scavengers as defined herein. For example, the product may include a separate container comprising an oxygen scavenger in addition to the plurality of unit dosage forms.

In one embodiment, the product and/or the unit dosage form includes a statement that the product has a shelf life of at least one year. In one embodiment, the product and/or the unit dosage form includes a statement that the product has a shelf life of one year. In one embodiment, the product and/or the unit dosage form includes a statement that the product has a shelf life of at least two years. In one embodiment, the product and/or the unit dosage form includes a statement that the product has a shelf life of two years. In one embodiment, the product and/or the unit dosage form includes a statement that the product has a shelf life of at least three years. In one embodiment, the product and/or the unit dosage form includes a statement that the product has a shelf life of three years. In one embodiment, the product and/or the unit dosage form includes a statement that the product has a shelf life of at least four years. In one embodiment, the product and/or the unit dosage form includes a statement that the product has a shelf life of four years. In one embodiment, the product and/or the unit dosage form includes a statement that the product has a shelf life of at least five years. In one embodiment, the product and/or the unit dosage form includes a statement that the product has a shelf life of at five years.

In any such embodiment, the statement regarding the shelf life may specify that the shelf life starts from the date of manufacture (e.g. the statement may specify that the product should be used within two years of manufacture). And/or in any such embodiment, the statement regarding the shelf life may be expressed by an expiry date (or similar), in which case the shelf life is the difference between that expiry date and the date on which the expiry date is being read (e.g. the expiry date could be more than two years in the future, relative to the date on which it is read, in which case the shelf life would be more than two years).

The unit dosage forms disclosed herein may exhibit a high degree of stability. For example, the crosslinked poly(allylamine) polymer of the present invention will not contain a significant amount of impurities even after storage for an extended period of time, e.g. at least one, two, three, four or five years.

In one embodiment, the unit dosage form disclosed herein protects the crosslinked poly(allylamine) polymer of the present invention such that the polymer will have less than 20 ppm $CH_2CHCH_2NH_2$ at least five years after being placed into the unit dosage form. In one embodiment, the unit dosage form disclosed herein protects the crosslinked poly(allylamine) polymer of the present invention such that the polymer will have less than 15 ppm $CH_2CHCH_2NH_2$ at least five years after being placed into the unit dosage form. In one embodiment, the unit dosage form disclosed herein protects the crosslinked poly(allylamine) polymer of the present invention such that the polymer will have less than 20 ppm $CH_2CHCH_2NH_2$ at least four years after being placed into the unit dosage form. In one embodiment, the unit dosage form disclosed herein protects the crosslinked poly(allylamine) polymer of the present invention such that the polymer will have less than 15 ppm $CH_2CHCH_2NH_2$ at least four years after being placed into the unit dosage form. In one embodiment, the unit dosage form disclosed herein protects the crosslinked poly(allylamine) polymer of the present invention such that the polymer will have less than 20 ppm $CH_2CHCH_2NH_2$ at least three years after being placed into the unit dosage form. In one embodiment, the unit dosage form disclosed herein protects the crosslinked poly(allylamine) polymer of the present invention such that the polymer will have less than 15 ppm $CH_2CHCH_2NH_2$ at least three years after being placed into the unit dosage form. In one embodiment, the unit dosage form disclosed herein protects the crosslinked poly(allylamine) polymer of the present invention such that the polymer will have less than 20 ppm $CH_2CHCH_2NH_2$ at least two years after being placed into the unit dosage form. In one embodiment, the unit dosage form disclosed herein protects the crosslinked poly(allylamine) polymer of the present invention such that the polymer will have less than 15 ppm $CH_2CHCH_2NH_2$ at least two years after being placed into the unit dosage form. In one embodiment, the unit dosage form disclosed herein protects the crosslinked poly(allylamine) polymer of the present invention such that the polymer will have less than 20 ppm $CH_2CHCH_2NH_2$ at least one year after being placed into the unit dosage form. In one embodiment, the unit dosage form disclosed herein protects the crosslinked poly(allylamine) polymer of the present invention such that the polymer will have less than 15 ppm $CH_2CHCH_2NH_2$ at least one year after being placed into the unit dosage form. In all such embodiments, the purity requirement referred to using the term "at least X year(s) after being placed into the unit dosage form" also discloses the option that the purity requirement is met at year X.

## Methods of treatment and medical uses of the crosslinked poly(allylamine) polymers

Methods of treatment and medical uses of the crosslinked poly(allylamine) polymers of the present invention are described in WO2014/197725 A1, WO2016/094685 A1, WO2017/193050 A1, WO2017/193064 A1, WO2017/193024 A1, WO2019/090176 A1, WO2019/090177 A1, WO2019/236639 A1, WO2019/236636 A1 and WO2019/236124 A1.

In accordance with one aspect of the present disclosure, the crosslinked poly(allylamine) polymers of the present invention are for use in any of the methods of treatment or medical uses described in any of WO2014/197725 A1,

WO2016/094685 A1, WO2017/193050 A1, WO2017/193064 A1, WO2017/193024 A1, WO2019/090176 A1, WO2019/090177 A1, WO2019/236639 A1, WO2019/236636 A1 and WO2019/236124 A1.

For example, the crosslinked poly(allylamine) polymers of the present invention, are for use in any of the methods of treating an acid-base disorder, such as metabolic acidosis, described in any of WO2014/197725 A1, WO2016/094685 A1, WO2017/193050 A1, WO2017/193064 A1, WO2017/193024 A1, WO2019/090176 A1, WO2019/090177 A1, WO2019/236639 A1, WO2019/236636 A1 and WO2019/236124 A1.

The baseline serum bicarbonate value may be the serum bicarbonate concentration determined at a single time point or may be the mean or median value of two or more serum bicarbonate concentrations determined at two or more time-points. For example, in one embodiment the baseline serum bicarbonate value may be the value of the serum bicarbonate concentration determined at a single time point and the baseline serum bicarbonate value is used as a basis to determine an acute acidic condition requiring immediate treatment. In another embodiment, the baseline serum bicarbonate treatment value is the mean value of the serum bicarbonate concentration for serum samples drawn at different time points (e.g., different days). By way of further example, in one such embodiment the baseline serum bicarbonate treatment value is the mean value of the serum bicarbonate concentration for serum samples drawn on different days (e.g., at least 2, 3, 4, 5 or more days, that may be consecutive or separated by one or more days or even weeks). By way of further example, in one such embodiment the baseline serum bicarbonate treatment value is the mean value of the serum bicarbonate concentration for serum samples drawn on two consecutive days preceding the initiation of treatment.

In one embodiment, the baseline serum bicarbonate value is the value of the serum bicarbonate concentration determined at a single time point. In another embodiment, the baseline serum bicarbonate value is the mean value of at least two serum bicarbonate concentrations determined at different time-points. For example, in one such embodiment the baseline serum bicarbonate value is the mean value of at least two serum bicarbonate concentrations for serum samples drawn on different days. By way of further example, the baseline serum bicarbonate value is the mean or median value of at least two serum bicarbonate concentrations for serum samples drawn on non-consecutive days. By way of further example, in one such method the non-consecutive days are separated by at least two days. By way of further example, in one such method the non-consecutive days are separated by at least one week. By way of further example, in one such method the non-consecutive days are separated by at least two weeks. By way of further example, in one such method the non-consecutive days are separated by at least three weeks.

In certain embodiments, the individual is treated with the daily dose for a period of at least one day. For example, in one such embodiment the individual is treated with the daily dose for a period of at least one week. By way of further example, in one such embodiment the individual is treated with the daily dose for a period of at least one month. By way of further example, in one such embodiment the individual is treated with the daily dose for a period of at least two months. By way of further example, in one such embodiment the individual is treated with the daily dose for a period of at least three months. By way of further example, in one such embodiment the individual is treated with the daily dose for a period of at least several months. By way of further example, in one such embodiment the individual is treated with the daily dose for a period of at least six months. By way of further example, in one such embodiment the individual is treated with the daily dose for a period of at least one year.

**Treating individuals afflicted with cancer and diabetes**

Gillies et al. (BBA - Reviews on Cancer 1871 (2019) 273-280) describes the effect of acidosis on cancer progression and diabetes. The acidification of the surrounding microenvironment of tumours results in important sequela for cancer progression including increased invasion and metastasis. Acidification also affects diabetes by preventing insulin from binding to its receptor, leading to peripheral resistance and an exacerbation of symptoms.

Gillies *et al.* proposes acidosis as a relevant thereapeutic target for the treatment of cancer and describes three approaches for targeting: buffers, nanomedicine, and proton pump inhibitors. Gillies *et al.* concludes that targeting the extracellular acidity directly has been shown to provide preclinical or clinical benefit in cancer and diabetes. A number of therapeutic agents are proposed, including a commercially available mix of bicarbonate and carbonate salts to control melanoma growth.

Veverimer (TRC101) is mentioned in Gillies *et al.* as an alternative agent that can be used to directly raise pH. A further aspect of the present disclosure is a composition comprising a crosslinked poly(allylamine) polymer of the present invention for use in treating an individual afflicted with cancer.

In aspects, the cancer is associated with acidosis. In aspects the acidosis is metabolic acidosis. In aspects the acidosis is lactic acidosis.

A further aspect of the present disclosure is a composition comprising a crosslinked poly(allylamine) polymer of the present invention for use in treating diabetes.

In aspects, the diabetes is type 1 diabetes. In aspects, the diabetes is type 2 diabetes.

Having described the invention in detail, it will be apparent that modifications and variations are possible without

departing the scope of the invention defined in the appended claims. Furthermore, it should be appreciated that all examples in the present disclosure are provided as non-limiting examples.

## EXAMPLES

The following non-limiting examples are provided to further illustrate the present invention. It should be appreciated by those of skill in the art that the techniques disclosed in the examples that follow represent approaches the inventors have found function well in the practice of the invention, and thus can be considered to constitute examples of modes for its practice. However, those of skill in the art should, in light of the present disclosure, appreciate that many changes can be made in the specific embodiments that are disclosed and still obtain a like or similar result without departing from the scope of the invention.

The examples of crosslinked poly(allylamine) polymers, wherein greater than 1.0% of the total number of carbon atoms present in the crosslinked poly(allylamine) polymers are $sp^2$ allyl carbons, are comparative examples and so are not polymers of the present invention.

### Example 1 - chemistry examples

The following chemistry examples are presented in six categories based on the process parameter manipulated:

(a) water content (Table 10_1 polymers 1-6, 12-16, 56-57; Table 10_2 polymers 35-39)

(b) reaction time (Table 10_1 polymers 7; Table 10_2 polymers 29-34, 40-43)

(c) reaction temperature (Table 10_1 polymers 8-11 Table 10_2 polymers 24-28)

(d) initiator content in the radical polymerization (Table 10_1 polymers 17-23, 58)

(e) ratio of DCE to poly(allylamine) polymer (Table 10_2 polymers 44-51)

(f) Ratio of AAH to DAPDA (Table 10_1 polymers 53 - 55)

(g) container closure (Table 10_2 polymer 52).

A general polymerization procedure is described and in each case specific examples are called out with reference to tables of synthesis parameters that were varied within the general procedure. Tables of the physicochemical performance characteristics of the resulting polymers are also provided.

### General polymerization procedure for beads formed by radical polymerization (addition/chain growth) and post-polymerization crosslinking of poly(allylamine) beads

An aqueous stock solution was prepared by dissolving 2-propen-1-ylamine, or a salt thereof, and 1,3-bis(allylamino)propane, or a salt thereof, in water. A reactor equipped with a stirrer was charged with aqueous stock solution and surfactant dissolved in a hydrophobic organic suspending solvent. A solution of radical initiator was prepared. The two mixtures were independently sparged with nitrogen. The initiator solution was added to the reaction mixture while stirring, and subsequently heated for up to 48 hours. A second portion of initiator may be added to the reaction mixture if necessary depending on the polymerization kinetics. After cooling the vessel to room temperature, the organic phase was removed and the beads were purified. The beads were dried. Examples of conditions that are suitable for the synthesis of polymers described in this example include, but are not limited to, the combinations shown in Table 10 part 1. These polymers were then subjected to post-polymerization crosslinking procedures as described below and in Table 10 part 2.

The resulting dried polyamine described in the procedure above was placed into a reactor and 1,2-dichloroethane, which could optionally act as the solvent, was added. A swelling agent was added to the resulting slurry. The mixture was heated with stirring for a required amount of time to reach completion. The reaction mixture was cooled and the beads were purified by washing and filtration and dried until no further water was removed and the weight remained constant. Examples of post-polymerization crosslinking described in this example include, but are not limited to, the polymers shown in Table 10, Part 2. Table 11 describes key physicochemical properties of the polymer examples shown in Table 10..

**Example 1 (a-1) Specific procedure for polymer prepared with variable water contents**

An aqueous stock solution was prepared by dissolving allylamine hydrochloride (2-Propen-1-ylamine, "AAH") (371.1 g) and 1,3-Bis(allylamino)propane dihydrochloride ("DAPDA") (386.0 g) in water (757.1 g). A reactor equipped with an overhead stirrer and nitrogen inlet, was charged with aqueous stock solution and surfactant (branched dodecylbenzene sulfonate, 36.0 g) dissolved in heptane (3264 g, "Solvent System 1"). In a separate vessel, a solution of 2,2'-azobis(2-methylpropionamidine)dihydrochloride (V-50) (76.8 g) in water (435.3 g) was prepared. The two mixtures were independently sparged with nitrogen. Under inert atmosphere, the initiator solution was added to the reaction mixture while stirring, and subsequently heated to 67°C for 16 hours. After cooling the vessel to room temperature, the organic phase was removed by decanting, and the beads were purified by washing and filtration with 1 M NaOH (1:1 water:MeOH, v/v), water until filtrate approached neutral pH, IPA, and finally heptane, and then dried for 48 hours. This polymer is shown in Table 10_1 and Table 11_1 as polymer 12.

1,2-Dichlorethane ("DCE") (750 g) was added to a reactor charged with the above resulting polyamine beads (100 g) and equipped with overhead stirring. The beads were dispersed before adding water (25 g) and heated to 65°C for 16 hours. The cooled beads were purified by washing and filtration with 1 M NaOH (1:1 water:MeOH, v/v), water until filtrate approached neutral pH, and finally methanol, and then dried for 24 hours. This polymer is shown in Table 10_Part 2 and Table 11 as polymer number 14.

**Example 1 (a-2) Specific procedure for post-crosslinked polymer prepared with variable water contents**

An aqueous stock solution of AAH was prepared by dissolving allylamine hydrochloride (2-Propen-1-ylamine, "AAH") (402.8 g) in water (207.5 g). An aqueous stock solution of DAPDA was prepared by dissolving 1,3-Bis(allylamino) propane dihydrochloride ("DAPDA", 419.0 g) in water (419.0 g). Into a 6 L, glass jacketed reactor equipped with an overhead stirrer, glass anchor stir paddle, nitrogen inlet and addition funnel, was charged surfactant (branched dodecylbenzene sulfonate, 60.0 g) dissolved in heptane (3284 g) and both aqueous stock solutions. In a separate vessel, a solution of V-50 (83.4 g) in water (472.6 g) was prepared. The two mixtures were independently sparged with nitrogen. Under inert atmosphere, the initiator solution was added to the reaction mixture while stirring, and subsequently heated to 67°C for 16 hours. Six hours after the first addition of V-50, an additional solution of V-50 (41.7 g in 236.3 g $H_2O$) is prepared, sparged with nitrogen, and added to the reaction mixture. After cooling the vessel to room temperature, the organic phase was removed by decanting, and the beads were purified by washing and filtration once with methanol, 4 times with 1 M NaOH (1:1 water:MeOH, v/v), water until the pH of the solution after washing was 9, and twice with methanol. The beads were dried in a vacuum oven at 60°C for 48 hours. This polymer is shown in Table 10_1 and Table 11_1 as polymer 37.

The following procedure was performed using different amounts of water (i.e. 6, 8, 10, 12, or 14 g, respectively).

1,2-Dichlorethane ("DCE") (200 g) was added to a jacketed reactor charged with the above resulting polyamine beads (40 g) and equipped with overhead stirring. The beads were dispersed before adding water (10 g) and heated to 65°C for 16 hours. The slurry was drained, and the crude product was isolated by filtration. The beads were purified by washing and filtration (4 times with 1N NaOH (1:1 water:MeOH, v/v), water until the pH of solution after washing was 9, and once with methanol). The purified beads were dried in a vacuum oven at 60°C for 24 hours. The dried polymer is packaged in a hermetically sealed container which provides a barrier to oxygen and moisture. This polymer is shown in Table 10_2 and Table 11_2 as polymer 37.

**Example 1 (b-1) Specific procedure for post-crosslinked polymer prepared with variable reaction time**

An aqueous stock solution was prepared by dissolving allylamine hydrochloride (2-Propen-1-ylamine, "AAH") (402.8 g) and 1,3-Bis(allylamino)propane dihydrochloride ("DAPDA") (419.0 g) in water (727.4 g). A reactor equipped with an overhead stirrer and nitrogen inlet, was charged with aqueous stock solution and surfactant (branched dodecylbenzene sulfonate, 60.0 g) dissolved in heptane (3261.6 g, "Solvent System 1"). In a separate vessel, a solution of V-50 (83.4 g) in water (472.6 g) was prepared. The two mixtures were independently sparged with nitrogen. Under inert atmosphere, the initiator solution was added to the reaction mixture while stirring, and subsequently heated to 67°C for 16 hours. After cooling the vessel to room temperature, the organic phase was removed by decanting, and the beads were purified by washing and filtration with 1 M NaOH (1:1 water:MeOH, v/v), water until filtrate approached neutral pH, IPA, finally heptane, and dried for 48 hours. This polymer is shown in Table 10_1 and Table 11_1 as polymer 28.

1,2-Dichlorethane ("DCE") (799.8 g) was added to a reactor charged with the above resulting polyamine beads (133.3 g) and equipped with overhead stirring. The beads were dispersed before adding water (33.3 g) and heated to 65°C for 10 hours. The cooled beads were purified by washing and filtration with 1 M NaOH (1:1 water:MeOH, v/v), water until filtrate approached neutral pH, and finally methanol, and dried for 24 hours. This polymer is shown in Table 10_Part 2 and Table 11 as polymer number 28.

**Example 1 (b-2) Specific procedure for polymer prepared with variable reaction time**

To prepare a 56 wt% aqueous allylamine hydrochloride (2-Propen-1-ylamine, "AAH") solution, allylamine (2-Propen-1-ylamine, "AA", 614 kg was slowly added to a cooled 34 wt% hydrochloric acid solution (1002 L) so that the temperature was between -10°C and 30°C. The solution was stirred for a minimum of 30 minutes. An aqueous stock solution of DAPDA was prepared by dissolving 1,3-Bis(allylamino)propane dihydrochloride ("DAPDA", 925 kg) in water (925 kg). Into a glass jacketed reactor equipped with an overhead stirrer, glass anchor stir paddle, and nitrogen inlet, was charged surfactant (branched dodecylbenzene sulfonate, 43 kg) dissolved in heptane (2346 kg), 596 kg DAPDA solution, and 514 kg of AAH solution. In a separate vessel, a solution of V-50 (60 kg) in water (291 kg) was prepared. The two mixtures were independently sparged with nitrogen. Under inert atmosphere, the initiator solution was added to the reaction mixture while stirring, and subsequently heated to 67°C for 16 hours. Six hours after the first addition of V-50, an additional solution of V-50 (30 kg in 145 kg $H_2O$) is prepared, sparged with nitrogen, and added to the reaction mixture. After cooling the vessel to room temperature, the organic phase was removed by decanting, and the beads were purified by washing with 1 M NaOH (1:1 water:MeOH, v/v), water until the pH of the solution after washing was 11, and with methanol. The beads were dried in a vacuum oven at 60°C for 48 hours. This polymer is shown in Table 10_1 and Table 11_1 as polymer 41.

The following procedure was performed using different reaction times. Reactions were heated to 65°C for either 12, 16, or 20 hours.

1,2-Dichlorethane ("DCE") (175 g) was added to a jacketed reactor charged with the resulting polyamine beads (35 g) and equipped with overhead stirring. The beads were dispersed before adding water (8.75 g) and heated to 65°C for 16 hours (see note above regarding reaction time). The slurry was drained, and the crude product was isolated by filtration. The beads were purified by washing with 1 M NaOH (1:1 water:MeOH, v/v), water until the pH of the solution after washing was 11, and with methanol. The purified beads were dried in a vacuum oven at 60°C for 24 hours. The dried polymer is packaged in a hermetically sealed container which provides a barrier to oxygen and moisture. This polymer is shown in Table 10_2 and Table 11_2 as polymer 41.

**Example 1 (c-1) Specific procedure for polymer prepared with variable reaction temperature in the radical polymerization step**

An aqueous stock solution was prepared by dissolving allylamine hydrochloride (2-Propen-1-ylamine, "AAH") (67.1 g) and 1,3-Bis(allylamino)propane dihydrochloride ("DAPDA") (69.8 g) in water (121.2 g). A reactor equipped with an overhead stirrer and nitrogen inlet, was charged with aqueous stock solution and surfactant (branched dodecylbenzene sulfonate, 10.0 g) dissolved in heptane (543.6 g, "Solvent System 1"). In a separate vessel, a solution of V-50 (13.9 g) in water (78.8 g) was prepared. The two mixtures were independently sparged with nitrogen. Under inert atmosphere, the initiator solution was added to the reaction mixture while stirring, and subsequently heated to 75°C for 16 hours. After cooling the vessel to room temperature, the organic phase was removed by decanting, and the beads were purified by washing and filtration with 1 M NaOH solution (1:1 water:MeOH, v/v), water until filtrate approached neutral pH, IPA, and finally heptane, and dried for 48 hours. This polymer is shown in Table 10_1 and Table 11_1 as polymer 11.

1,2-Dichlorethane ("DCE") (500.3 g) was added to a reactor charged with the above resulting polyamine beads (66.7 g) and equipped with overhead stirring. The beads were dispersed before adding water (16.7 g) and heated to 65°C for 16 hours. The cooled beads were purified by washing and filtration with 1 M NaOH (1:1 water:MeOH, v/v), water until filtrate approached neutral pH, and finally methanol, and dried for 24 hours. This polymer is shown in Table 10_Part 2 and Table 11 as polymer number 11.

**Example 1 (c-2) Specific procedure for post-crosslinked polymer prepared with variable reaction temperature**

An aqueous stock solution was prepared by dissolving allylamine hydrochloride (2-Propen-1-ylamine, "AAH") (402.8 g) and 1,3-Bis(allylamino)propane dihydrochloride ("DAPDA") (419.0 g) in water (727.4 g). A reactor equipped with an overhead stirrer and nitrogen inlet, was charged with aqueous stock solution and surfactant (branched dodecylbenzene sulfonate, 60.0 g) dissolved in heptane (3261.6 g, "Solvent System 1"). In a separate vessel, a solution of V-50 (83.4 g) in water (472.6 g) was prepared. The two mixtures were independently sparged with nitrogen. Under inert atmosphere, the initiator solution was added to the reaction mixture while stirring, and subsequently heated to 67°C for 16 hours. After cooling the vessel to room temperature, the organic phase was removed by decanting, and the beads were purified by washing and filtration with 1 M NaOH solution (1:1 water:MeOH, v/v), water until filtrate approached neutral pH, IPA, and finally heptane, and dried for 48 hours. This polymer is shown in Table 10_1 and Table 11_1 as polymer 28.

1,2-Dichlorethane ("DCE") (750 g) was added to a reactor charged the above resulting polyamine beads (100 g) and equipped with overhead stirring. The beads were dispersed before adding water (25 g) and heated to 75°C for 16 hours. The cooled beads were purified by washing and filtration with 1 M NaOH (1:1 water:MeOH, v/v), water until filtrate

approached neutral pH, and finally methanol, and dried for 24 hours. This polymer is shown in Table 10_Part 2 and Table 11 as polymer number 28.

**Example 1 (d-1) Specific procedure for polymer prepared with variable initiator content**

An aqueous stock solution was prepared by dissolving allylamine hydrochloride (2-Propen-1-ylamine, "AAH") (64.3 g) and 1,3-Bis(allylamino)propane dihydrochloride ("DAPDA") (66.9 g) in water (242.3). A 3-neck round bottom flask with four side baffles equipped with an overhead stirrer, Dean Stark apparatus and condenser, and nitrogen inlet, was charged with aqueous stock solution and surfactant (branched dodecylbenzene sulfonate, 19.6 g) dissolved in a 74:26 chlorobenzene/heptane solution (1120 g, "Solvent System 2"). In a separate vessel, a solution of V-50 (6.7 g) in water (37.7 g) was prepared. The two mixtures were independently sparged with nitrogen. Under inert atmosphere, the initiator solution was added to the reaction mixture while stirring, and subsequently heated to 67°C for 16 hours. After cooling the vessel to room temperature, the organic phase was removed by decanting, and the beads were purified by washing and filtration twice with methanol, water, twice with 1 M HCl, water, 4 times with 1 M NaOH (1:1 water:MeOH, v/v), and water until the pH of the solution after washing was 7. The beads were dried in a lyophilizer for 48 hours. This polymer is shown in Table 10_1 and Table 11_1 as polymer 21.

1,2-Dichlorethane ("DCE") (75.0 g) was added to a reactor charged with the above resulting polyamine beads (10.0 g) and equipped with overhead stirring. The beads were dispersed before adding water (2.5 g) and heated to 65°C for 16 hours. The cooled beads were purified by washing and filtration twice with methanol, water, twice with 1 M HCl, water, 4 times with 1 M NaOH (1:1 water:MeOH, v/v), and water until the pH of solution after washing was 7. The polymer was dried in a lyophilizer for 48 hours. This polymer is shown in Table 10_Part 2 and Table 11 as polymer 21.

**Example 1 (d-2) Specific procedure for polymer prepared with variable initiator content**

An aqueous stock solution was prepared by dissolving allylamine hydrochloride (2-Propen-1-ylamine, "AAH") (11.0 g) and 1,3-Bis(allylamino)propane dihydrochloride ("DAPDA") (11.4 g) in water (37.1 g). A 3-neck round bottom flask with four side baffles equipped with an overhead stirrer, Dean Stark apparatus and condenser, and nitrogen inlet, was charged with aqueous stock solution and surfactant (branched dodecylbenzene sulfonate, 3.0 g) dissolved in a 74:26 chlorobenzene/heptane solution (300 g, "Solvent System 2"). In a separate vessel, a solution of V-50 (2.4 g) in water (13.6 g) was prepared. The two mixtures were independently sparged with nitrogen. Under inert atmosphere, the initiator solution was added to the reaction mixture while stirring, and subsequently heated to 67°C for 16 hours. A second portion of initiator solution (16.0 g) and the reaction mixture were degassed and combined before increasing the temperature to 115°C for a final dehydration step. After cooling the vessel to room temperature, the organic phase was removed by decanting, and the beads were purified by washing and filtration twice with methanol, water, twice with 1 M HCl, water, 4 times with 1 M NaOH (1:1 water:MeOH, v/v), and water until the pH of the solution after washing was 7. The beads were dried in a lyophilizer for 48 hours. This polymer is shown in Table 10_1 and Table 11_1 as polymer 23.

1,2-Dichlorethane ("DCE") (75.0 g) is added to a reactor charged with the above resulting polyamine beads (10.0 g) and equipped with overhead stirring. The beads are dispersed before adding water (2.5 g) and heated to 65°C for 16 hours. The cooled beads are purified by washing and filtration twice with methanol, water, twice with 1 M HCl, water, 4 times with 1 M NaOH (1:1 water:MeOH, v/v), and water until the pH of solution after washing was 7. The polymer is dried in a lyophilizer for 48 hours. This polymer is shown in Table 10_Part 2 and Table 11 as polymer 23.

**Example 1 (d-3) Specific procedure for polymer prepared with variable initiator content**

The following procedure was performed using different amounts of V-50 in the first addition (i.e. 4.6, 7.4, and 9.1 g, respectively). A 15 weight % solution of V-50 is always used, and water addition is adjusted accordingly to maintain same total amount of water.

An aqueous stock solution of AAH was prepared by dissolving allylamine hydrochloride (2-Propen-1-ylamine, "AAH") (46.2 g) in water (23.8 g). An aqueous stock solution of DAPDA was prepared by dissolving 1,3-Bis(allylamino) propane dihydrochloride ("DAPDA", 48.0 g) in water (48.0 g). Into a 1 L, glass jacketed reactor equipped with an overhead stirrer, glass anchor stir paddle, nitrogen inlet and addition funnel, was charged surfactant (branched dodecylbenzene sulfonate, 6.6 g) dissolved in heptane (359 g), both aqueous stock solutions, and water (19.3 g). In a separate vessel, a solution of V-50 (9.1 g) in water (51.6 g) was prepared. The two mixtures were independently sparged with nitrogen. Under inert atmosphere, the initiator solution was added to the reaction mixture while stirring, and subsequently heated to 67°C for 16 hours. Six hours after the first addition of V-50, an additional solution of V-50 (4.6 g in 26.3 g $H_2O$) is prepared, sparged with nitrogen, and added to the reaction mixture. After cooling the vessel to room temperature, the organic phase was removed by decanting, and the beads were purified by washing and filtration once with methanol, 4 times with 1 M NaOH (1:1 water:MeOH, v/v), water until the pH of the solution after washing was 9, and twice with methanol. The beads

were dried in a vacuum oven at 60°C for 48 hours. This polymer is shown in Table 10_1 and Table 11_1 as polymer 19.

1,2-Dichlorethane ("DCE") (200 g) was added to a jacketed reactor charged with the resulting polyamine beads (40 g) and equipped with overhead stirring. The beads were dispersed before adding water (10 g) and heated to 65°C for 16 hours. The slurry was drained, and the crude product was isolated by filtration. The beads were purified by washing and filtration (4 times with 1N NaOH (1:1 water:MeOH, v/v), water until the pH of solution after washing was 11, and once with methanol). The purified beads were dried in a vacuum oven at 60°C for 24 hours. The dried polymer is packaged in a hermetically sealed container which provides a barrier to oxygen and moisture. This polymer is shown in Table 10_2 and Table 11_2 as polymer 19.

**Example 1 (e-1) Specific procedure for polymer prepared with variable DCE to poly(allylamine) polymer ratios**

An aqueous stock solution of AAH was prepared by dissolving allylamine hydrochloride (2-Propen-1-ylamine, "AAH") (263.0 g) in water (263.0 g). An aqueous stock solution of DAPDA was prepared by dissolving 1,3-Bis(allylamino) propane dihydrochloride ("DAPDA", 273.6 g) in water (273.6 g). Into a 6 L, glass jacketed reactor equipped with an overhead stirrer, glass anchor stir paddle, nitrogen inlet and addition funnel, was charged surfactant (branched dodecylbenzene sulfonate, 84.0 g) dissolved in heptane (3284 g) and both aqueous stock solutions. In a separate vessel, a solution of V-50 (54.5 g) in water (308.6 g) was prepared. The two mixtures were independently sparged with nitrogen. Under inert atmosphere, the initiator solution was added to the reaction mixture while stirring, and subsequently heated to 67°C for 16 hours. After cooling the vessel to room temperature, the organic phase was removed by decanting, and the beads were purified by washing and filtration twice with methanol, water, twice with 1 M HCl, water, 3 times with 1 M NaOH (1:1 water:MeOH, v/v), water until the pH of the solution after washing was 9, twice with isopropanol, and twice with heptane. The purified beads were dried in a vacuum oven at 60°C for 48 hours. This polymer is shown in Table 10_1 and Table 11_1 as polymer 49.

The following procedure was performed using different amounts of polyamine and water, to adjust the DCE: polymer ratio without affecting the overall volume of the reaction or the water: polymer ratio. Reactions were performed with the following amounts of polyamine and water (90 g polyamine, 22.5 g water; 60 g, 15 g; 36 g, 9 g; 18 g, 4.5 g).

1,2-Dichlorethane ("DCE") (450 g) was added to a jacketed reactor charged with the resulting polyamine beads (60 g) and equipped with overhead stirring. The beads were dispersed before adding water (15 g) and heated to 65°C for 16 hours. The slurry was drained, and the crude product was isolated by filtration. The beads were purified by washing and filtration (twice with methanol, water, twice with 1 M HCl, water, 4 times with 1 M NaOH (aq), water until the pH of the solution after washing was 9, and once with methanol). The purified beads were dried in a vacuum oven at 40°C for 48 hours. The dried polymer is packaged in a hermetically sealed container which provides a barrier to oxygen and moisture. This polymer is shown in Table 10_2 and Table 11_2 as polymer 49.

**Example 1 (e-2) Specific procedure for polymer prepared with variable DCE to poly(allylamine) polymer ratios**

An aqueous stock solution of AAH was prepared by dissolving allylamine hydrochloride (2-Propen-1-ylamine, "AAH") (402.8 g) in water (207.5 g). An aqueous stock solution of DAPDA was prepared by dissolving 1,3-Bis(allylamino) propane dihydrochloride ("DAPDA", 419.0 g) in water (419.0 g). Into a 6 L, glass jacketed reactor equipped with an overhead stirrer, glass anchor stir paddle, nitrogen inlet and addition funnel, was charged surfactant (branched dodecylbenzene sulfonate, 60.0 g) dissolved in heptane (3284 g) and both aqueous stock solutions. In a separate vessel, a solution of V-50 (83.4 g) in water (472.6 g) was prepared. The two mixtures were independently sparged with nitrogen. Under inert atmosphere, the initiator solution was added to the reaction mixture while stirring, and subsequently heated to 67°C for 16 hours. After cooling the vessel to room temperature, the organic phase was removed by decanting, and the beads were purified by washing and filtration (3 times with 1 M NaOH (1:1 water:MeOH, v/v), water until the pH of the solution after washing was 7, twice with isopropanol and twice with heptane. The beads were dried in a vacuum oven at 60°C for 48 hours. This polymer is shown in Table 10_1 and Table 11_1 as polymer 45.

The following procedure was performed using different amounts of polyamine and water, to adjust the DCE: polymer ratio without affecting the overall volume of the reaction or the water: polymer ratio. Reactions were performed with the following amounts of polyamine and water (90 g polyamine, 22.5 g water; 60 g, 15 g; 36 g, 9 g; 18 g, 4.5 g).

1,2-Dichlorethane ("DCE") (450 g) was added to a jacketed reactor charged with the above resulting polyamine beads (60 g) and equipped with overhead stirring. The beads were dispersed before adding water (15 g) and heated to 65°C for 16 hours. The slurry was drained, and the crude product was isolated by filtration. The beads were purified by washing and filtration (4 times with 1N NaOH (1:1 water:MeOH, v/v), water until the pH of solution after washing was 9, and once with methanol). The purified beads were dried in a vacuum oven at 40°C for 48 hours. The dried polymer is packaged in a hermetically sealed container which provides a barrier to oxygen and moisture. This polymer is shown in Table 10_2 and Table 11_2 as polymer 45.

**Example 1 (f-1) Specific procedure for polymer stored with variable container closure**

To prepare a 56 wt% aqueous allylamine (2-Propen-1-ylamine, AAH) hydrochloride solution, allylamine (2-Propen-1-ylamine, AA, 305 kg) was slowly added to a cooled 34 wt% hydrochloric acid solution (585 kg) so that the temperature was between -10°C and 30°C. The solution was stirred for a minimum of 30 minutes. An aqueous stock solution of DAPDA was prepared by dissolving 1,3-Bis(allylamino)propane dihydrochloride ("DAPDA", 56.2 kg) in water (56.2 kg). Into a glass jacketed reactor equipped with an overhead stirrer, glass anchor stir paddle, and nitrogen inlet, was charged surfactant (branched dodecylbenzene sulfonate, 8.0 kg) dissolved in heptane (437 kg), the DAPDA solution, and 96 kg of AAH solution. In a separate vessel, a solution of V-50 (11.2 kg) in water (63.5 kg) was prepared. The two mixtures were independently sparged with nitrogen. Under inert atmosphere, the initiator solution was added to the reaction mixture while stirring, and subsequently heated to 67°C for 16 hours. Six hours after the first addition of V-50, an additional solution of V-50 (5.6 kg in 31.7 kg $H_2O$) is prepared, sparged with nitrogen, and added to the reaction mixture. After cooling the vessel to room temperature, the organic phase was removed by decanting, and the beads were purified by washing with 1 M NaOH (1:1 water:MeOH, v/v), water until the pH of the solution after washing was 11, and with methanol. The beads were dried in a vacuum oven at 60°C for 48 hours. This polymer is shown in Table 10_1 and Table 11_1 as polymer 52.

1,2-Dichlorethane ("DCE") (322 kg) was added to a jacketed reactor charged with the resulting polyamine beads (65.7 kg) and equipped with overhead stirring. The beads were dispersed before adding water (15.6 kg) and heated to 65°C for 16 hours (see note above regarding reaction time). The slurry was drained, and the crude product was isolated by filtration. The beads were purified by washing with 1 M NaOH (1:1 water:MeOH, v/v), water until the pH of the solution after washing was 11, and with methanol. The purified beads were dried in a vacuum oven at 60°C for 72 hours. The dried polymer is packaged in a hermetically sealed container which provides a barrier to oxygen and moisture. This polymer is shown in Table 10_2 and Table 11_2 as polymer 52.

More frequently, the sp2 carbon content of poly(allylamine) polymers comprised of allylamine hydrochloride and 1,3-Bis(allylamino)propane hydrochloride was measured prior to crosslinking with 1,2-dichloroethane. As discussed elsewhere, it is advantageous to determine the sp2 carbon content of such preformed poly(allylamine) polymers because a higher signal to noise may be obtained for sp2 carbon by 13C ssNMR at this stage. All values of percent sp2 allyl carbon given in Table 11 part 1 are directly measured. In some cases the percent sp2 allyl carbon in crosslinked poly(allylamine) polymers given in the examples was measured directly by NMR, and these are indicated in Table 11 part 2. Alternatively, the percent sp2 allyl carbon present in the crosslinked poly(allylamine) polymers given in the example was calculated by multiplying the percent sp2 allyl carbon in the corresponding preformed poly(allylamine) polymer by a factor of 0.9. The factor is derived from the addition of sp3 carbon during crosslinking of the preformed poly(allylamine) polymer with 1,2-dichloroethane.

**Example 1 (g-1) Specific procedure for variable ratio of DAPDA to AAH**

The following procedure was performed using different relative amounts of the AAH and DAPDA. Reactions were performed with the following amounts of AAH and DAPDA (38.9 g AAH, 50.9 g DAPDA; 44.0 g AAH, 45.8g DAPDA; 49.7 g AAH, 40.2 g DAPDA).

To prepare a 56 wt% aqueous allylamine hydrochloride (2-Propen-1-ylamine, AAH) solution, allylamine (2-Propen-1-ylamine, AA, 614 kg) was slowly added to a cooled 34 wt% hydrochloric acid solution (1002 L) so that the temperature was between -10°C and 30°C. The solution was stirred for a minimum of 30 minutes. An aqueous stock solution of DAPDA was prepared by dissolving 1,3-Bis(allylamino)propane dihydrochloride ("DAPDA", 925 kg) in water (925 kg). Into a 1 L, glass jacketed reactor equipped with an overhead stirrer, glass anchor stir paddle, nitrogen inlet and addition funnel, was charged surfactant (branched dodecylbenzene sulfonate, 6.6 g) dissolved in heptane (357 g), AAH stock solution (38.9 g), and DAPDA stock solution (50.9 g). In a separate vessel, a solution of V-50 (9.1 g) in water (51.6 g) was prepared. The two mixtures were independently sparged with nitrogen. Under inert atmosphere, the initiator solution was added to the reaction mixture while stirring, and subsequently heated to 67°C for 16 hours. Six hours after the first addition of V-50, an additional solution of V-50 (4.5 g in 25.8 g $H_2O$) is prepared, sparged with nitrogen, and added to the reaction mixture. After cooling the vessel to room temperature, the organic phase was removed by decanting, and the beads were purified by washing with 1 M NaOH (1:1 water:MeOH, v/v), water until the pH of the solution after washing was 11, and with methanol. The beads were dried in a vacuum oven at 60°C for 48 hours. This polymer is shown in Table 10_1 and Table 11_1 as polymer 53.

1,2-Dichlorethane ("DCE") (200 g) was added to a jacketed reactor charged with the resulting polyamine beads (40 g) and equipped with overhead stirring. The beads were dispersed before adding water (10 g) and heated to 65°C for 16 hours. The slurry was drained, and the crude product was isolated by filtration. The beads were purified by washing with 1 M NaOH (1:1 water:MeOH, v/v), water until the pH of the solution after washing was 11, and with methanol. The purified beads were dried in a vacuum oven at 60°C for 24 hours. The dried polymer is packaged in a hermetically sealed container which provides a barrier to oxygen and moisture. This polymer is shown in Table 10_2 and Table 11_2 as polymer 53.

**Example 2 - comparative polymers made by a process disclosed in WO2016/094685 A1:**

Three comparative poly(allylamine) polymers and three comparative crosslinked poly(allylamine) polymers (polymers 62, 63 and 64 in the tables below) were tested for $sp^2$ carbon % and the amount (ppm) of allylamine formed per day in the Heated Stability Assay (Stability Assay 2).

The comparative poly(allylamine) polymers and crosslinked poly(allylamine) polymers were prepared using the process disclosed in paragraphs [0385], [0386], and [0388] of WO2016/094685A1.

The $sp^2$ carbon % of the poly(allylamine) polymers 62, 63 and 64 was measured using the $^{13}C$ CPMAS ssNMR (quantitative $^{13}C$ solid state cross-polarization magic angle spinning (CPMAS) NMR) method and the $sp^2$ carbon % of the crosslinked poly(allylamine) polymers 62, 63 and 64 was measured using the $^{13}C$ MAS ssNMR (quantitative $^{13}C$ solid state magic angle spinning (MAS) NMR) method.

The crosslinked poly(allylamine) polymers 62, 63 and 64 were also subjected to the Heated Stability Assay (Stability Assay 2).

Table 10_Part 1: Synthesis of radical polymerization poly(allylamine) polymer beads

| Polymer number | Solvent (g) | Amine (2-Propen-1-ylamine, "AAH") (g) | Water (g) | (1,3-Bis(allylamino) propane "DAPDA") (g) | Surfactant (g) | V-50 (g) | Solvent System | Aqueous solid content (ASC) (wt%) | Temp (°C) | Time (h) |
|---|---|---|---|---|---|---|---|---|---|---|
| 1 | 3261.6 | 402.8 | 1200 | 419 | 60 | 83.4 | 1 | 43 | 67 | 16 |
| 2 | 761 | 61.4 | 280 | 63.8 | 14 | 12.7 | 1 | 33 | 67 | 16 |
| 3 | 544 | 47.9 | 200 | 49.9 | 14 | 9.9 | 1 | 35 | 67 | 16 |
| 4 | 544 | 52.3 | 200 | 54.4 | 14 | 10.8 | 1 | 37 | 67 | 16 |
| 5 | 544 | 61.8 | 200 | 64.3 | 6 | 12.8 | 1 | 41 | 67 | 16 |
| 6 | 3261.6 | 402.8 | 1200 | 419 | 60 | 83.4 | 1 | 43 | 67 | 16 |
| 7 | 356.6 | 44 | 131.2 | 45.8 | 6.6 | 9.1 | 1 | 43 | 67 | 6 |
| 8 | 356.6 | 44 | 131.2 | 45.8 | 6.6 | 9.1 | 1 | 43 | 47 | 48 |
| 9 | 356.6 | 44 | 131.2 | 45.8 | 6.6 | 9.1 | 1 | 43 | 57 | 28 |
| 10 | 356.6 | 28.8 | 131.2 | 29.9 | 6.6 | 6 | 1 | 33 | 57 | 16 |
| 11 | 543.6 | 67.1 | 200 | 69.8 | 10 | 13.9 | 1 | 43 | 75 | 16 |
| 12 | 1120 | 61.4 | 280 | 63.8 | 19.6 | 12.7 | 2 | 33 | 67 | 16 |
| 13 | 3261.6 | 263 | 1200 | 273.6 | 84 | 54.5 | 1 | 33 | 67 | 16 |
| 14 | 3264 | 371.1 | 1200 | 386 | 36 | 76.8 | 1 | 41 | 67 | 16 |
| 15 | 544 | 61.8 | 200 | 64.3 | 4 | 12.8 | 1 | 43 | 67 | 16 |
| 16 | 544 | 72.8 | 200 | 75.7 | 10 | 15.1 | 1 | 45 | 67 | 16 |
| 17 | 356.6 | 46.2 | 159.6 | 48 | 6.6 | 9.1 | 1 | 43 | 67 | 16 |
| 18 | 356.6 | 46.2 | 159.6 | 48 | 6.6 | 12 | 1 | 43 | 67 | 16 |
| 19 | 356.6 | 46.2 | 159.6 | 48 | 6.6 | 13.7 | 1 | 43 | 67 | 16 |
| 20 | 356.6 | 44 | 131.2 | 45.8 | 6.6 | 9.1 | 1 | 43 | 67 | 16 |
| 21 | 1120 | 64.3 | 280 | 66.9 | 19.6 | 6.7 | 2 | 33 | 67 | 20 |
| 22 | 2400 | 131.5 | 600 | 136.8 | 36 | 27.2 | 2 | 33 | 67 | 16 |
| 23 | 300 | 11 | 75.5 | 11.4 | 3 | 4.8 | 2 | 33 | 67 | 20 |
| 24 | 3261.6 | 402.8 | 1200 | 419 | 60 | 83.4 | 1 | 43 | 67 | 16 |
| 25 | 3261.6 | 402.8 | 1200 | 419 | 60 | 83.4 | 1 | 43 | 67 | 16 |

(continued)

| Polymer number | Solvent (g) | Amine (2-Propen-1-ylamine, "AAH") (g) | Water (g) | (1,3-Bis(allylamino) propane "DAPDA") (g) | Surfactant (g) | V-50 (g) | Solvent System | Aqueous solid content (ASC) (wt%) | Temp (°C) | Time (h) |
|---|---|---|---|---|---|---|---|---|---|---|
| 26 | 3261.6 | 402.8 | 1200 | 419 | 60 | 83.4 | 1 | 43 | 67 | 16 |
| 27 | 3261.6 | 402.8 | 1200 | 419 | 60 | 83.4 | 1 | 43 | 67 | 16 |
| 28 | 3261.6 | 402.8 | 1200 | 419 | 60 | 83.4 | 1 | 43 | 67 | 16 |
| 29 | 3261.6 | 402.8 | 1200 | 419 | 60 | 83.4 | 1 | 43 | 67 | 16 |
| 30 | 3261.6 | 402.8 | 1200 | 419 | 60 | 83.4 | 1 | 43 | 67 | 16 |
| 31 | 3261.6 | 402.8 | 1200 | 419 | 60 | 83.4 | 1 | 43 | 67 | 16 |
| 32 | 3261.6 | 402.8 | 1200 | 419 | 60 | 83.4 | 1 | 43 | 67 | 16 |
| 33 | 3261.6 | 402.8 | 1200 | 419 | 60 | 83.4 | 1 | 43 | 67 | 16 |
| 34 | 3261.6 | 402.8 | 1200 | 419 | 60 | 83.4 | 1 | 43 | 67 | 16 |
| 35 | 3284 | 402.8 | 1436.3 | 419 | 60 | 125.1 | 1 | 43 | 67 | 16 |
| 36 | 3284 | 402.8 | 1436.3 | 419 | 60 | 125.1 | 1 | 43 | 67 | 16 |
| 37 | 3284 | 402.8 | 1436.3 | 419 | 60 | 125.1 | 1 | 43 | 67 | 16 |
| 38 | 3284 | 402.8 | 1436.3 | 419 | 60 | 125.1 | 1 | 43 | 67 | 16 |
| 39 | 3284 | 402.8 | 1436.3 | 419 | 60 | 125.1 | 1 | 43 | 67 | 16 |
| 40 | 2346000 | 287800 | 870200 | 298000 | 43000 | 90000 | 1 | 43 | 67 | 16 |
| 41 | 2346000 | 287800 | 870200 | 298000 | 43000 | 90000 | 1 | 43 | 67 | 16 |
| 42 | 2346000 | 287800 | 870200 | 298000 | 43000 | 90000 | 1 | 43 | 67 | 16 |
| 43 | 2346000 | 287800 | 870200 | 298000 | 43000 | 90000 | 1 | 43 | 67 | 16 |
| 44 | 3284 | 402.8 | 1394.4 | 419 | 60 | 83.4 | 1 | 43 | 67 | 16 |
| 45 | 3284 | 402.8 | 1394.4 | 419 | 60 | 83.4 | 1 | 43 | 67 | 16 |
| 46 | 3284 | 402.8 | 1394.4 | 419 | 60 | 83.4 | 1 | 43 | 67 | 16 |
| 47 | 3284 | 402.8 | 1394.4 | 419 | 60 | 83.4 | 1 | 43 | 67 | 16 |
| 48 | 3284 | 263 | 1200 | 273.6 | 84 | 54.5 | 1 | 33 | 67 | 16 |
| 49 | 3284 | 263 | 1200 | 273.6 | 84 | 54.5 | 1 | 33 | 67 | 16 |
| 50 | 3284 | 263 | 1200 | 273.6 | 84 | 54.5 | 1 | 33 | 67 | 16 |

(continued)

| Polymer number | Solvent (g) | Amine (2-Propen-1-ylamine, "AAH") (g) | Water (g) | (1,3-Bis(allylamino) propane "DAPDA") (g) | Surfactant (g) | V-50 (g) | Solvent System | Aqueous solid content (ASC) (wt%) | Temp (°C) | Time (h) |
|---|---|---|---|---|---|---|---|---|---|---|
| 51 | 3284 | 263 | 1200 | 273.6 | 84 | 54.5 | 1 | 33 | 67 | 16 |
| 52 | 437000 | 54000 | 188700 | 56400 | 8000 | 16800 | 1 | 43 | 67 | 16 |
| 53 | 356.6 | 38.9 | 131.2 | 50.9 | 6.6 | 13.65 | 1 | 43 | 67 | 16 |
| 54 | 356.6 | 44 | 131.2 | 45.8 | 6.6 | 13.65 | 1 | 43 | 67 | 16 |
| 55 | 356.6 | 49.7 | 131.2 | 40.2 | 6.6 | 13.65 | 1 | 43 | 67 | 16 |
| 56 | 61600 | 3400 | 15490 | 3520 | 1110 | 710 | 2 | 33 | 67 | 16 |
| 57 | 437000 | 53760 | 163060 | 56000 | 8000 | 11100 | 1 | 43 | 67 | 16 |
| 58 | 2346000 | 288400 | 872600 | 300000 | 44000 | 90000 | 1 | 43 | 67 | 16 |
| 59 | 4391000 | 555000 | 1638000 | 542000 | 83200 | 169200 | 1 | 42 | 67 | 16 |
| 60 | 437000 | 55000 | 160300 | 56200 | 8000 | 16800 | 1 | 43 | 67 | 16 |
| 61 | 4377000 | 543000 | 1648000 | 556000 | 81300 | 171000 | 1 | 42 | 67 | 16 |
| | Polymers made using the process in WO2016/094685 A1 | | | | | | | | | |
| 62 | 1200 | 43.83 | 59.14 | 45.60 | 12 | 9.08 | 2 | 33 | 67 | 16 |
| 63 | 1200 | 43.83 | 59.14 | 45.60 | 12 | 9.08 | 2 | 33 | 67 | 16 |
| 64 | 1200 | 43.83 | 59.14 | 45.60 | 12 | 9.08 | 2 | 33 | 67 | 16 |
| | NP = not performed | | | | | | | | | |

Table 10_Part 2: Synthesis of post-crosslinked radical polymerization crosslinked poly(allylamine) polymer beads (Contd.)

| Post-crosslinked polymer number | Polymer (g) | DCE (g) | Water (g) | Temp (°C) | Time (h) |
|---|---|---|---|---|---|
| 1 | 133.3 | 1000 | 33.3 | 65 | 16 |
| 2 | 45 | 337.5 | 11.3 | 65 | 16 |
| 3 | 45 | 337.5 | 11.3 | 65 | 16 |
| 4 | 45 | 337.5 | 11.3 | 65 | 16 |
| 5 | 45 | 337.5 | 11.3 | 65 | 16 |
| 6 | NP | NP | NP | NP | NP |
| 7 | 45 | 337.5 | 11.3 | 65 | 16 |
| 8 | 40 | 300 | 10 | 65 | 16 |
| 9 | 40 | 300 | 10 | 65 | 16 |
| 10 | 20 | 150 | 5 | 65 | 16 |
| 11 | 66.7 | 500.3 | 16.7 | 65 | 16 |
| 12 | 50 | 375 | 12.5 | 65 | 16 |
| 13 | 60 | 450 | 15 | 65 | 16 |
| 14 | 100 | 750 | 25 | 65 | 16 |
| 15 | 45 | 337.5 | 11.3 | 65 | 16 |
| 16 | 45 | 337.5 | 11.3 | 65 | 16 |
| 17 | NP | NP | NP | NP | NP |
| 18 | NP | NP | NP | NP | NP |
| 19 | 40 | 200 | 10 | 65 | 16 |
| 20 | 40 | 300 | 10 | 65 | 16 |
| 21 | 10 | 75 | 2.5 | 65 | 16 |
| 22 | 133 | 997.5 | 33.3 | 65 | 16 |
| 23 | NP | NP | NP | NP | NP |
| 24 | 133.3 | 1000 | 33.3 | 65 | 16 |
| 25 | 66.7 | 500 | 16.7 | 55 | 16 |
| 26 | 66.7 | 500 | 16.7 | 60 | 16 |
| 27 | 45 | 337.5 | 11.3 | 70 | 16 |
| 28 | 66.7 | 500 | 16.7 | 75 | 16 |
| 29 | 133.3 | 799.8 | 33.3 | 65 | 10 |
| 30 | 66.7 | 500 | 16.7 | 65 | 20 |
| 31 | 100 | 750 | 25 | 65 | 10 |
| 32 | 66 | 495 | 16.5 | 65 | 10 |
| 33 | 266.7 | 2000 | 66.7 | 65 | 16 |
| 34 | 266.7 | 2000 | 66.7 | 65 | 16 |
| 35 | 40 | 200 | 6 | 65 | 16 |
| 36 | 40 | 200 | 8 | 65 | 16 |
| 37 | 40 | 200 | 10 | 65 | 16 |
| 38 | 40 | 200 | 12 | 65 | 16 |
| 39 | 40 | 200 | 14 | 65 | 16 |

(continued)

| Post-crosslinked polymer number | Polymer (g) | DCE (g) | Water (g) | Temp (°C) | Time (h) |
|---|---|---|---|---|---|
| 40 | 35 | 175 | 8.75 | 65 | 12 |
| 41 | 35 | 175 | 8.75 | 65 | 16 |
| 42 | 35 | 175 | 8.75 | 65 | 16 |
| 43 | 35 | 175 | 8.75 | 65 | 20 |
| 44 | 90 | 450 | 22.5 | 65 | 16 |
| 45 | 60 | 450 | 15 | 65 | 16 |
| 46 | 36 | 450 | 9 | 65 | 16 |
| 47 | 18 | 450 | 4.5 | 65 | 16 |
| 48 | 90 | 450 | 22.5 | 65 | 16 |
| 49 | 60 | 450 | 15 | 65 | 16 |
| 50 | 36 | 450 | 9 | 65 | 16 |
| 51 | 18 | 450 | 4.5 | 65 | 16 |
| 52 | 65700 | 322000 | 15600 | 65 | 16 |
| 53 | 40 | 200 | 10 | 65 | 16 |
| 54 | 40 | 200 | 10 | 65 | 16 |
| 55 | 40 | 200 | 10 | 65 | 16 |
| 56 | 3800 | 28600 | 950 | 65 | 16 |
| 57 | 63200 | 450000 | 15800 | 65 | 16 |
| 58 | 64600 | 300000 | 16200 | 65 | 16 |
| 59 | 638400 | 3204000 | 160000 | 64 | 16 |
| 60 | 61000 | 320000 | 15400 | 65 | 16 |
| 61 | 641300 | 3202000 | 160000 | 65 | 16 |
| Polymers made using the process in WO2016/094685 A1 | | | | | |
| 62 | 15.00 | 112.5 | 3.75 | 70 | 16 |
| 63 | 15.00 | 112.5 | 3.75 | 70 | 16 |
| 64 | 15.00 | 112.5 | 3.75 | 70 | 16 |
| NP = not performed | | | | | |

Table 11_Part 1: Properties of radical polymerization poly(allylamine) polymer beads

| Polymer number | swelling (g/g) | SGF (mmol/g) | % sp2 carbons |
|---|---|---|---|
| 1 | 3.4 | 14.4 | NP |
| 2 | 4.2 | 13.8 | 1.6% |
| 3 | 4.9 | 13.8 | 1.4% |
| 4 | 4.2 | 13.8 | 1.2% |
| 5 | 4.3 | 14.1 | 1.2% |
| 6 | 3.5 | 14 | 1.2% |
| 7 | 3.5 | 14.2 | NP |
| 8 | 3.3 | 14.6 | NP |
| 9 | 3.2 | 14.1 | NP |

(continued)

| Polymer number | swelling (g/g) | SGF (mmol/g) | % sp2 carbons |
|---|---|---|---|
| 10 | 3.3 | 14.6 | 2.3% |
| 11 | 3.6 | 14.8 | 2.7% |
| 12 | NP | 14.1 | NP |
| 13 | 4.9 | 13.4 | NP |
| 14 | 3.8 | 14.1 | NP |
| 15 | 3.1 | 14.2 | NP |
| 16 | 2.7 | 14 | 0.7% |
| 17 | 3.7 | 14.4 | 1.4% |
| 18 | 3.8 | 14.1 | 0.8% |
| 19 | 4.1 | 13.5 | 0.6% |
| 20 | 4.4 | 14.4 | 3.2% |
| 21 | NP | 14.1 | 3.4% |
| 22 | NP | 14.1 | 1.8% |
| 23 | NP | 13.5 | 1.2% |
| 24 | 3.5 | 14.1 | NP |
| 25 | 3.5 | 14.1 | NP |
| 26 | 3.5 | 14.1 | NP |
| 27 | 3.5 | 14.1 | NP |
| 28 | 3.5 | 14.1 | NP |
| 29 | 3.9 | 13.6 | NP |
| 30 | 3.6 | 13.4 | NP |
| 31 | 3.9 | 13.6 | NP |
| 32 | 3.9 | 13.6 | NP |
| 33 | 3.5 | 14 | 1.2% |
| 34 | 3.5 | 14 | 1.2% |
| 35 | 3.7 | 14.0 | 0.8% |
| 36 | 3.7 | 14.0 | 0.8% |
| 37 | 3.7 | 14.0 | 0.8% |
| 38 | 3.7 | 14.0 | 0.8% |
| 39 | 3.7 | 14.0 | 0.8% |
| 40 | 3.6 | 14.0 | 0.7% |
| 41 | 3.6 | 14.0 | 0.7% |
| 42 | 3.6 | 14.0 | 0.7% |
| 43 | 3.6 | 14.0 | 0.7% |
| 44 | 3.9 | 13.6 | NP |
| 45 | 3.9 | 13.6 | NP |
| 46 | 3.9 | 13.6 | NP |
| 47 | 3.9 | 13.6 | NP |
| 48 | NP | 13.7 | NP |

(continued)

| Polymer number | swelling (g/g) | SGF (mmol/g) | % sp2 carbons |
|---|---|---|---|
| 49 | NP | 13.7 | NP |
| 50 | NP | 13.7 | NP |
| 51 | NP | 13.7 | NP |
| 52 | 3.8 | 13.4 | 0.9% |
| 53 | 4.1 | 13.7 | 0.8% |
| 54 | 4.2 | 13.7 | 0.8% |
| 55 | 4.2 | 14.2 | 0.6% |
| 56 | 4.6 | 13.5 | 1.5% |
| 57 | 4.1 | 13.7 | 1.9% |
| 58 | 3.7 | 13.6 | 0.9% |
| 59 | 3.8 | 14.0 | 0.6% |
| 60 | 3.7 | 13.9 | 0.7% |
| 61 | 3.6 | 13.7 | 0.5% |
| Polymers made using the process in WO2016/094685 A1 | | | |
| 62 | NP | NP | 1.2% |
| 63 | NP | NP | 1.2% |
| 64 | NP | NP | 1.2% |
| NP = not performed | | | |

Table 11_Part 2: Properties of post-crosslinked radical polymerization crosslinked poly(allylamine) polymer beads

| Post-crosslinked polymer number | swelling (g/g) | SGF (mmol/g) | SIB Cl (mmol/g) | SIB P (mmol/g) | % sp2 Carbons | Calculated or Measured[a] | Wt Ratio of carbon to nitrogen (C/N) | AA at release (ppm) | AME at release (ppm) | AOH at release (ppm) | AA stability assay number | AA stability (ppm/d) |
|---|---|---|---|---|---|---|---|---|---|---|---|---|
| 1 | NP | 11.1 | 5.2 | 1.3 | NP | NP | NP | 0.9 | < 25 | < 5 | 1 | 0.4 |
| 2 | 1.1 | 11.4 | 4.8 | 1.3 | 1.4% | C | NP | 3.1 | 494 | < 5 | 2 | 11.3 |
| 3 | 1.2 | 11.4 | 4.9 | 1.5 | 1.3% | C | NP | 2.4 | 513 | < 5 | 2 | 9.3 |
| 4 | 1.0 | 11.1 | 5.3 | 1 | 1.1% | C | NP | 1.6 | 475 | < 5 | 2 | 6.3 |
| 5 | NP | 11.4 | 5.3 | 1 | 1.1% | C | NP | 0.8 | 493 | < 5 | 1 | 0.8 |
| 6 | NP | NP | NP | NP | 1.1% | C | NP | NP | NP | NP | NP | NP |
| 7 | NP | 10.8 | 4.3 | 0.8 | NP | NP | NP | 0.7 | < 25 | < 5 | 1 | 0.3 |
| 8 | NP | 11.3 | 4.0 | 2.3 | NP | NP | NP | 0.6 | < 25 | < 5 | 1 | 0.6 |
| 9 | NP | 10.6 | 4.4 | 1.3 | NP | NP | NP | 0.5 | < 25 | < 5 | 1 | 0.4 |
| 10 | NP | 10.8 | 4.1 | 1.1 | 2.1% | C | NP | 0.5 | < 25 | < 5 | 1 | 1.2 |
| 11 | NP | 11.5 | 4.9 | 1.2 | 2.4% | C | NP | 0.6 | < 25 | < 5 | 1 | 0.9 |
| 12 | NP | 11.3 | 4.8 | 1.6 | NP | NP | NP | 2.6 | 593 | < 5 | 1 | 1.2 |
| 13 | NP | 11.3 | 5 | 1.7 | NP | NP | NP | 3.0 | 522 | < 5 | 1 | 1.0 |
| 14 | 1.3 | 11.7 | 5 | 1.5 | NP | NP | NP | 1.4 | 534 | < 5 | 1 | 0.6 |
| 15 | 1.1 | 10.8 | 4.9 | 0.9 | NP | NP | NP | 0.7 | 131 | < 5 | 1 | 0.6 |
| 16 | 1.3 | 11.4 | 5.5 | 0.9 | 0.6% | C | NP | 1.0 | 398 | < 5 | 1 | 0.1 |
| 17 | NP | NP | NP | NP | 1.3% | C | NP | NP | NP | NP | NP | NP |
| 18 | NP | NP | NP | NP | 0.7% | C | NP | NP | NP | NP | NP | NP |
| 19 | NP | 11.1 | 5.0 | 0.8 | 0.5% | C | NP | < 0.3 | 70 | 1.6 | 1 | 0.2 |
| 20 | 1.3 | 12.8 | 2.4 | 4.4 | 2.9% | C | NP | 3.6 | < 25 | < 5 | 1 | 2.5 |
| 21 | NP | 10.9 | 2.7 | 3.5 | 3.1% | C | NP | NP | NP | NP | NP | NP |
| 22 | NP | 11.3 | 4.5 | 2 | 1.6% | C | NP | NP | NP | NP | NP | NP |
| 23 | NP | NP | NP | NP | 1.1% | C | NP | NP | NP | NP | NP | NP |
| 24 | NP | 11.2 | 4.9 | 1.4 | NP | NP | NP | 0.5 | < 25 | < 5.5 | 1 | 0.6 |

(continued)

| Post-crosslinked polymer number | swelling (g/g) | SGF (mmol/g) | SIB Cl (mmol/g) | SIB P (mmol/g) | % sp2 Carbons | Calculated or Measured[a] | Wt Ratio of carbon to nitrogen (C/N) | AA at release (ppm) | AME at release (ppm) | AOH at release (ppm) | AA stability assay number | AA stability (ppm/d) |
|---|---|---|---|---|---|---|---|---|---|---|---|---|
| 25 | NP | 10.2 | 4.5 | 2.2 | NP | NP | NP | 0.5 | < 25 | < 5.5 | 1 | 0.6 |
| 26 | NP | 11.5 | 4.8 | 1.6 | NP | NP | NP | 0.9 | 149 | < 5.5 | 1 | 0.6 |
| 27 | NP | 11.1 | 4.8 | 0.9 | NP | NP | NP | < 0.8 | < 25 | < 5.5 | 1 | 0.3 |
| 28 | NP | 9.9 | 4.5 | 0.8 | NP | NP | NP | < 0.8 | < 25 | < 5.5 | 1 | 0.2 |
| 29 | NP | 11.8 | 5.2 | 1.3 | NP | NP | NP | 0.9 | NP | NP | NP | NP |
| 30 | NP | 10.8 | 4.4 | 1.3 | NP | NP | NP | 0.6 | < 25 | < 5.5 | 1 | 0.9 |
| 31 | NP | 11.2 | 5.3 | 1.1 | NP | NP | NP | 0.7 | < 25 | < 5.5 | 1 | 0.6 |
| 32 | NP | 11.0 | 5.5 | 1.1 | NP | NP | NP | 0.9 | < 25 | < 5.5 | 1 | 0.6 |
| 33 | NP | 11.4 | 5.4 | 1.5 | 0.9% | M | NP | 1.5 | 120 | < 5.5 | 2 | 1.8 |
| 34 | NP | 11.4 | 5.4 | 1.5 | 0.9% | M | NP | 1.5 | 120 | < 5.5 | 1 | 0.5 |
| 35 | NP | 11.1 | 5.0 | 1.2 | 0.7% | C | NP | 0.3 | 246 | 1.1 | 1 | 0.2 |
| 36 | NP | 11.0 | 5.4 | 0.9 | 0.7% | C | NP | < 0.3 | 185 | 1.1 | 1 | 0.1 |
| 37 | NP | 11.2 | 5.3 | 0.8 | 0.7% | C | NP | 0.3 | 213 | < 1.0 | 1 | 0.2 |
| 38 | NP | 11.5 | 5.2 | 1.0 | 0.7% | C | NP | 0.6 | 216 | 1.1 | 1 | 0.3 |
| 39 | NP | 11.4 | 5.0 | 1.4 | 0.7% | C | NP | 1.2 | 199 | 1.0 | 1 | 0.3 |
| 40 | 1.3 | 10.8 | 4.9 | 1.3 | 0.6% | C | NP | 1.8 | 202 | < 1 | NP | NP |
| 41 | 1.2 | 10.3 | 4.9 | 1.0 | 0.6% | C | NP | < 1.25 | 78 | 1.4 | NP | NP |
| 42 | 1.3 | 10.7 | 4.8 | 1.2 | 0.6% | C | NP | 1.7 | 235 | < 1 | NP | NP |
| 43 | 1.2 | 10.4 | 4.9 | 0.9 | 0.6% | C | NP | < 1.25 | < 50 | < 1 | NP | NP |
| 44 | 1.2 | 11.2 | 5.3 | 1.0 | NP | NP | NP | < 0.8 | < 25 | < 5.5 | NP | NP |
| 45 | 1.3 | 11.8 | 5.3 | 1.0 | NP | NP | NP | < 0.8 | < 25 | < 5.5 | NP | NP |
| 46 | 1.3 | 11.6 | 5.7 | 0.9 | NP | NP | NP | < 0.8 | < 25 | < 5.5 | NP | NP |
| 47 | 1.3 | 11.4 | 5.5 | 0.9 | NP | NP | NP | < 0.8 | < 25 | < 5.5 | NP | NP |
| 48 | 1.2 | 11.4 | 5.1 | 1.6 | NP | NP | NP | 4.0 | 461 | < 5.5 | NP | NP |

(continued)

| Post-crosslinked polymer number | swelling (g/g) | SGF (mmol/g) | SIB Cl (mmol/g) | SIB P (mmol/g) | % sp2 Carbons | Calculated or Measured[a] | Wt Ratio of carbon to nitrogen (C/N) | AA at release (ppm) | AME at release (ppm) | AOH at release (ppm) | AA stability assay number | AA stability (ppm/d) |
|---|---|---|---|---|---|---|---|---|---|---|---|---|
| 49 | 1.2 | 11.3 | 5.0 | 1.6 | NP | NP | NP | 3.6 | 477 | < 5.5 | NP | NP |
| 50 | 1.2 | 11.3 | 5.2 | 1.3 | NP | NP | NP | 3.0 | 430 | < 5.5 | NP | NP |
| 51 | 1.2 | 11.0 | 5.2 | 1.1 | NP | NP | NP | 3.1 | 463 | < 5.5 | NP | NP |
| 52 | 1.1 | 10.3 | 4.7 | 1.0 | 0.8% | C | 3.7 | 1.5 | 321 | 4.0 | 3 | NA |
| 53 | 1.5 | 11.1 | 4.4 | 1.7 | 0.7% | C | NP | 1.5 | 324 | 1.2 | NP | NP |
| 54 | 1.5 | 10.8 | 5.3 | 1.1 | 0.7% | C | NP | < 1.25 | 179 | 1.4 | NP | NP |
| 55 | 1.5 | 11.3 | 5.9 | 0.8 | 0.5% | C | NP | < 1.25 | 154 | 1.5 | NP | NP |
| 56 | 1.1 | 11.0 | 4.7 | 1.5 | 1.3% | M | 3.7 | 4.3 | 503 | 2.8 | NP | NP |
| 57 | 1.2 | 11.1 | 4.5 | 1.3 | 2.0% | M | 3.8 | 3.2 | 454 | 12.8 | NP | NP |
| 58 | 1.2 | 11.2 | 4.4 | 0.9 | < 0.7% | M | 3.7 | < 1.25 | 107 | 2.0 | NP | NP |
| 59 | 1.1 | 10.3 | 4.4 | 0.7 | 0.88% | M | 3.8 | 1.4 | 120 | 2 | NP | NP |
| 60 | 1.1 | 10.3 | 4.7 | 1.3 | 0.63% | C | 3.8 | 1.8 | 269 | 4 | 2 | 2.0 |
| 61 | 1.2 | 10.6 | 4.9 | 1.1 | 0.45% | C | 3.8 | 1.1 | 134 | 2 | 2 | 1.5 |
| | Polymers made using the process in WO2016/094685 A1 | | | | | | | | | | | |
| 62 | NP | NP | NP | NP | 1.1% | M | NP | NP | NP | NP | 2 | 2.7 |
| 63 | NP | NP | NP | NP | 1.3% | M | NP | NP | NP | NP | 2 | 2.8 |
| 64 | NP | NP | NP | NP | 1.3% | M | NP | NP | NP | NP | 2 | 3.4 |
| | NA = not applicable; NP = not performed | | | | | | | | | | | |
| | a) M indicates that % sp2 carbons was measured directly by NMR spectroscopy. C indicates that the % sp2 carbons was calculated from the corresponding example from Table 11 part 1 as described above. | | | | | | | | | | | |

Table 12: Properties of post-crosslinked radical polymerization beads from Stability Assay 3

| | AA (ppm) | |
|---|---|---|
| **Time (Months)** | **25°C/60%RH** | **40°C/75%RH** |
| 0 | 4 | 4 |
| 1 | 4 | 4 |
| 3 | 3 | 3 |
| 6 | 5 | 4 |
| RH = relative humidity | | |

**Example 3** - **examples relating to the stability of the crosslinked poly(allylamine) polymer with respect to oxygen with different packaging approaches**

The following examples demonstrate several approaches to reducing the exposure of the crosslinked poly(allylamine) polymer disclosed herein to oxygen, thereby reducing or eliminating degradation, by measuring the allylamine concentration. Thus, these approaches also relate to increasing the stability of the polymer disclosed herein.

**General**

The following examples are involve unit dosage forms produced from loading a crosslinked poly(allylamine) polymer disclosed herein in a packet material. Packet materials are known and Table A0 provides details of commercially available packet materials. The packet materials can comprise aluminum foil laminate films. An example packet material, can be formed from a 4-layer laminate web stock (composed of polyethylene terephthalate (PET)/low density polyethylene (LDPE)/foil/LDPE). The packet material can be heat-sealed on four sides to form a packet. The outer layer/surface of the packet can be PET, and the inner layer or surface in contact with crosslinked poly(allylamine) polymer disclosed herein can be LDPE. Packet materals may not contain adhesive layers if produced by extrusion lamination. Other packet materials can be produced from a 4-layer laminate web stock and adhesive layers.

For the packet materials described herein, oxygen transfer rate can be measured by the method defined in standard: ASTM D3985 (e.g. DOI: 10.1520/D3985-17). For the packet materials described herein, water vapor transfer rate can be measured by the method defined in standard: ASTM F1249 (e.g. DOI: 10.1520/F1249-20).

Table A0: example packet materials suitable for unit dosage forms disclosed herein.

| Packet material | Supplier |
|---|---|
| RFE-005 | Amcor Flexibles Europe & Americas |
| RFE-006 | Amcor Flexibles Europe & Americas |
| RFE-011 | Amcor Flexibles Europe & Americas |
| RFE-013 | Amcor Flexibles Europe & Americas |
| RFE-015 | Amcor Flexibles Europe & Americas |
| RFE-016 | Amcor Flexibles Europe & Americas |
| RFE-022 | Amcor Flexibles Europe & Americas |
| RFE-024 | Amcor Flexibles Europe & Americas |
| RFE-040 | Amcor Flexibles Europe & Americas |
| RFE-070 | Amcor Flexibles Europe & Americas |
| RFE-071 | Amcor Flexibles Europe & Americas |
| RFE-074 | Amcor Flexibles Europe & Americas |
| RFE-083 | Amcor Flexibles Europe & Americas |
| RFE-084 | Amcor Flexibles Europe & Americas |
| RFE-207 | Amcor Flexibles Europe & Americas |

(continued)

| Packet material | Supplier |
|---|---|
| EFO 181-001 | Glenroy |
| 48603X | FPC Flexible Packging Corp. |
| NeoCarta | Gualapack Group |
| Sterifoil | Gualapack Group |
| FLEXA | Constantia Flexibles |
| DIVIDUA | Constantia Flexibles |
| VELAR | Constantia Flexibles |
| FRUGI | Constantia Flexibles |
| ALUTA | Constantia Flexibles |
| OxyVanish-Dry Film | Mitsubishi Gas Chemicals America Inc. |
| PAKVF4 | Impak Corporation |

**Example 3 (a) Impact of packet material on stability**

**Introduction**

As mentioned above, a crosslinked poly(allylamine) polymer disclosed herein is veverimer. Veverimer can be provided in 3 g, 6 g and 9 g dosage strengths which require packaging in a packet material to form the unit dosage form. It was postulated that if the packet material minimized the exposure of veverimer to atmospheric oxygen, then it would increase the stability (e.g. the shelf life/long term storage) of veverimer inside the packet material.

To investigate this, three different packet materials containing 1.5 g of veverimer were subjected to a stability study in order to determine the effect of the packet material on minimizing the degradation of veverimer by oxygen under various conditions (e.g. temperature and/or relative humidity over time).

**Packet materials for testing**

Packet materials which had barrier properties for oxygen and water ingress were selected for testing. Three commercially-available packet materials comprised of aluminum foil laminate films were sourced (details in Table A1).

Further technical information about the packet materials for testing, e.g. structure, oxygen transfer rate ($O_2TR$), and water vapor transfer rate (WVTR) is also presented in Table A1.

Table A1: Information on the aluminum foil laminate films evaluated as a packet material

| **Aluminum foil laminate films (Packet materials)** | **Construction (from outer surface to product contact surface)** | **Oxygen Transfer Rate ($CC/m^2/day$)** | **Water Vapor Transfer Rate ($g/m^2/day$)** |
|---|---|---|---|
| A | Paper (32.5 $g/m^2$)<br>Adhesive (4.0 $g/m^2$)<br>Polyester (32.7 $g/m^2$)<br>PE Resin (12.5 $g/m^2$)<br>Aluminum Foil (24.3 $g/m^2$) = 9 um<br>PE Resin (24.5 $g/m^2$) | N/A | 0.02a |
| C | PET (32.4 $g/m^2$)<br>Ink[b] (1.5 $g/m^2$)<br>Adhesive (3.5 $g/m^2$)<br>Aluminum Foil (48.0 $g/m^2$) = 18 um<br>Adhesive (2.5 $g/m^2$)<br>LLDPE (23.1 $g/m^2$) | <0.005[c] | 0.01[d] |

(continued)

| Aluminum foil laminate films (Packet materials) | Construction (from outer surface to product contact surface) | Oxygen Transfer Rate (CC/m$^2$/day) | Water Vapor Transfer Rate (g/m$^2$/day) |
|---|---|---|---|
| B | PET (23.0 μm)<br>Ink[b]<br>White LDPE (12.0 g/m$^2$)<br>Aluminum Foil (18.0 μm)<br>LDPE (34.0 g/m$^2$) | <0.045[e] | 0.10[f] |
| N/A = Not available; PE = polyester; LLDPE = linear low density polyethylene; CC = cubic centimeters [a] Measured at 38°C, 100% RH<br>[b] Aluminum foil laminate films used in this study do not contain an ink layer<br>[c] Measured at 23°C, 50% RH<br>[d] Measured at 38°C, 90% RH<br>[e] Measured at 23°C, 0% RH<br>[f] Measured at 38°C, 100% RH | | | |

**Manufacture of veverimer packets**

Veverimer (1.5 g) was packaged in 7.62 cm x 10.16 cm (3" x 4") packets prepared from the aluminum foil laminate films described in Table A1, which yielded the unit dose forms for testing. For aluminum foil laminate films B and C, 3-side sealed packets of size 7.62 cm x 10.16 cm (3" x 4") were loaded with veverimer and the remaining (top) side was manually heat sealed with portable heat sealer at Tricida. For A, a 3-side sealed packet of size ~7.62 cm x ~10.16 cm (~3" x ~4") were prepared at Tricida by heat sealing and the remaining (top) side was heat sealed after adding veverimer. Thus, unit dosage form forms made from the A, B and C packet materials were produced.

**Stability Study With Different Aluminum Foil Laminate Films**

Having manufactured the unit dosage forms for testing, a stability study was initiated. The study measured the concentration of allylamine (a degradation product) over a period of six weeks in unit dosage forms A, B and C at 25°C/60% relative humidity (RH) and 40°C/75% RH. The concentration (ppm) of allylamine (AA) in the unit dosage form was determined by cation exchange ion chromatography (as per the protocol described herein under the "Cation IC Extraction Procedure").

The cumulative concentration of allylamine in unit dosage forms A, B and C over six weeks at 25°C/60% RH is displayed in Table A2. The cumulative concentration of allylamine in unit dosage forms A, B and C over six weeks at 40°C/75% RH is displayed in Table A3. Lower limit of detection (LOD) data is also included for the studies at 25°C/60% RH and 40°C/75% RH in Tables A4 and A5 respectively.

Table A2: Concentration (ppm) of AA in veverimer unit dosage forms at 25°C/60% RH

| Unit dosage form | Time (Weeks) | | | | |
|---|---|---|---|---|---|
| | 0 | 1 | 2 | 4 | 6 |
| | Concentration of AA (ppm) | | | | |
| A | <1.3 | 4.0 | 7.2 | 13.3 | 13.5 |
| B | <1.3 | 3.4 | 4.4 | 5.7 | 4.6 |
| C | <1.3 | 3.4 | 4.1 | 5.3 | 6.1 |

Note: Quantitation Limit (QLs) for AA measurement is 1.3 ppm. The AA value at T0 was estimated to be 0.9 ppm for calculation/graphing purposes.

Table A3: Concentration (ppm) of AA in veverimer unit dosage forms at 40°C/75% RH

| Unit dosage form | Time (Weeks) | | | | |
|---|---|---|---|---|---|
| | 0 | 1 | 2 | 4 | 6 |
| | Concentration of AA (ppm) | | | | |
| A | <1.3 | 7.5 | 12.0 | 18.1 | 17.1 |
| B | <1.3 | 4.9 | 4.8 | 5.0 | 4.0 |
| C | <1.3 | 4.3 | 4.5 | 4.8 | 3.5 |

Note: Quantitation Limit (QLs) for AA measurement is 1.3 ppm. The AA value at T0 was estimated to be 0.9 ppm for calculation/graphing purposes.

Table A4: LOD data for veverimer unit dosage forms at 25°C/60% RH

| Unit dosage form | Time (Weeks) | | | | |
|---|---|---|---|---|---|
| | 0 | 1 | 2 | 4 | 6 |
| | Concentration of AA (ppm) | | | | |
| A | 1.9 | 3.4 | 4.2 | 4.5 | 4.7 |
| B | 1.9 | 3.2 | 2.4 | 4.0 | 2.5 |
| C | 1.9 | 2.2 | 2.1 | 2.4 | 2.4 |

Table A5: LOD data for veverimer unit dosage forms at 40°C/75% RH

| Unit dosage form | Time (Weeks) | | | | |
|---|---|---|---|---|---|
| | 0 | 1 | 2 | 4 | 6 |
| | Concentration of AA (ppm) | | | | |
| A | 1.9 | 4.7 | 4.6 | 5.6 | 6.3 |
| B | 1.9 | 2.6 | 2.2 | 3.4 | 3.0 |
| C | 1.9 | 2.0 | 2.0 | 1.6 | 2.5 |

The data from Table 2A is presented in Figure 1A. The data from Table A3 is presented in Figure 1B.

**Discussion of results**

The data in Table 2A and Figure 1A shows that the concentration of allylamine is increasing over time for unit dosage form A. This is suggests that oxygen, the form of air, is entering the unit dosage form and causing veverimer to degrade and form allylamine. The data in Table 2A and Figure 1A shows that the concentration of allylamine is substantially constant after one week for unit dosage forms B and C (c.f. the LOD data from Table A4). This suggests that minimal or no oxygen is entering the unit dosage form. Similar trends are observed for the data in Table A3 and Figure 1B. Any slight increase in AA concentration could be attributable due to oxygen already present in the unit dosage form, e.g. because the unit dosage form was produced in air so air was present in the head space.

In view of the data for unit dosage forms B and C from Table A3 and Figure 1B being obtained under more stressful conditions (40°C/75% RH) than the data in Table A2 and Figure 1A (25°C/60% RH), it's notable that the results are substantially the same, e.g. a constant ~5 ppm of allylamine. This suggests that unit dosage forms B and C are effective at preventing oxygen from entering the unit dosage form. The B and C packets (e.g. aluminum foil laminate films) have an Oxygen Transfer Rate (CC/$m^2$/day) of <0.045 and <0.005 respectively. These data would suggest that a crosslinked poly(allylamine) polymer disclosed herein (e.g. veverimer) in a packet material with an Oxygen Transfer Rate (CC/$m^2$/day) of 0.05 or less may be suitable for producing a stable unit dosage form.

**Example 3 (b) Impact of packet head space on stability**

**Introduction**

Example 3 (a) demonstrates that unit dosage forms with B or C as the package material (or a material with an Oxygen Transfer Rate (CC/$m^2$/day) of 0.05 or less) prevents oxygen from entering the unit dosage form. Consequently, the crosslinked poly(allylamine) polymer (e.g. veverimer) does not appreciably degrade and is stable over long periods of time.

The unit dosage form has a fixed volume. Different amounts (e.g. 3 g, 6 g or 9 g dosages) of the crosslinked poly(allylamine) polymer (e.g. veverimer) can be loaded into the packet material to produce the unit dosage form. The different amounts (or mass) of the crosslinked poly(allylamine) polymer (e.g. veverimer) will have different volumes. Therefore, a unit dosage form containing less drug substance (e.g. the crosslinked poly(allylamine) polymer such as veverimer) will have a larger head space. It follows that a unit dosage form containing more drug substance will have a smaller head space. In this context, the head space can be considered the volume of the sealed packet material minus the volume of the drug substance inside the packet. If the unit dosage form is produced in air, the amount of oxygen in the unit dosage form will vary depending on the amount of drug substance.

Thus, this example investigates the effect of different amounts of oxygen in the head space of the unit dosage form on the stability of the drug substance (e.g. the crosslinked poly(allylamine) polymer such as veverimer).

**Manufacture of veverimer packets (unit dosage forms)**

The procedure described in Example 3 (a) was followed to produce the unit dosage forms for this Example. Veverimer in 1.5, 3.0 and 4.5 g amounts was packaged in 6.35 cm x 7.94 cm (2.5" × 3.125") packets of packet material B to produce 1.5, 3.0 and 4.5 g unit dosage forms. As mentioned above, as the amount of veverimer in the packet increases, the head space volume:veverimer mass ratio decreases since the dimensions and therefore the volume of the packet is fixed.

For reference, the head space of a sealed packet can be estimated according to known methods, e.g. Formula X described elsewhere herein. For example, 3 g of veverimer packaged in a 6.35 cm x 7.94 cm (2.5" × 3.125") packet, the estimated head space volume is 29 $cm^3$, and the ratio of head space volume:veverimer mass is 10 $cm^3$/g.

**Stability study with different head space volumes**

Having manufactured the unit dosage forms for testing, a stability study was initiated. The study measured the concentration of allylamine (a degradation product) over a period of three months in the 1.5, 3.0 and 4.5 g unit dosage forms at 25°C/60% relative humidity (RH) and 40°C/75% RH. The concentration (ppm) of allylamine (AA) in the unit dosage form was determined by cation exchange ion chromatography (as per the protocol described herein under the "Cation IC Extraction Procedure").

The cumulative concentration of allylamine in the 1.5, 3.0 and 4.5 g unit dosage forms over three months at 25°C/60% RH is displayed in Table A6. The cumulative concentration of allylamine in the 1.5, 3.0 and 4.5 g unit dosage forms over three months at 40°C/75% RH is displayed in Table A7.

Table A6: Concentration (ppm) of AA in veverimer unit dosage forms at 25°C/60% RH

| **Amount (g) of veverimer in the unit dosage form (packet material B)** | **Time (Months)** | | | |
|---|---|---|---|---|
| | **0** | **1** | **2** | **3** |
| | **Concentration of AA (ppm)** | | | |
| 1.5 | 0.8 | 2.6 | 2.9 | 3.4 |
| 3 | 0.8 | 2.0 | 2.8 | 3.0 |
| 4.5 | 0.8 | 1.7 | 2.2 | 2.3 |

Table A7: Concentration (ppm) of AA in veverimer unit dosage forms at 40°C/75% RH

| **Amount (g) of veverimer in the unit dosage form (packet material B)** | **Time (Months)** | | | |
|---|---|---|---|---|
| | **0** | **1** | **2** | **3** |
| | **Concentration of AA (ppm)** | | | |
| 1.5 | 0.8 | 2.4 | 3.1 | 3.2 |
| 3 | 0.8 | 1.7 | 2.5 | 2.8 |

(continued)

| Amount (g) of veverimer in the unit dosage form (packet material B) | Time (Months) | | | |
|---|---|---|---|---|
| | 0 | 1 | 2 | 3 |
| | Concentration of AA (ppm) | | | |
| 4.5 | 0.8 | 1.5 | 1.9 | 2.5 |

The data from Table A6 is presented in Figure 2. The data from Table A7 is presented in Figure 3.

**Discussion of results**

The data in Table A6 and Figure 2 shows that the concentration of allylamine is steadily increasing from about 1 ppm to about 2-3 ppm over time the 1.5, 3.0 and 4.5 g unit dosage forms. This suggests that a small amount of the polymer within the unit dosage form is being degraded by oxygen to form allylamine. For the 1.5, 3.0 and 4.5 g unit dosage forms, the rate of formation of allylamine decreases over the three month study. This suggests that the oxygen in the unit dosage form is being consumed and not replaced, e.g. from air outside the unit dosage form, which would agree with the results from Example 3(a) that unit dosage form B prevents oxygen from entering the unit dosage form.

As discussed above, the head space in the packet of the unit dosage form decreases with increasing amounts of drug substance in the packet of the unit dosage form. Therefore the amount of oxygen in the 1.5, 3.0 and 4.5 g unit dosage forms should be the most for the 1.5 g unit dosage form and the least for the 4.5 g unit dosage form. The data in Table A6 and Figure 2 shows that highest concentration of AA was measured in the 1.5 g unit dosage form. By contrast, the lowest concentration of AA was measured in the 4.5 g unit dosage form. These results show that less oxygen in the unit dosage form results in less formation of AA and therefore less degradation by oxygen.

Similar trends are observed for the data in Table A7 and Figure 3.

Therefore, the data shows that minimal polymer degradation due to oxygen already present in the unit dosage form occurs in the 1.5, 3.0 and 4.5 g unit dosage forms. The most degradation, as measured by the concentration of AA, occurs in the 4.5 g unit dosage form which is due to this unit dosage form having the largest head space, and therefore, the most oxygen present.

To conclude, this example demonstrates that an approach to increase the stability of the crosslinked poly(allylamine) polymer (e.g. veverimer) is reducing the volume of the head space in the unit dosage form.

**Example 3 (c) Impact of an oxygen scavenger in the unit dosage form**

**Introduction**

Example 3 (b) demonstrates that a residual amount oxygen in the unit dosage form, e.g. due to the unit dosage form being produced in air, results in some degradation of the crosslinked poly(allylamine) polymer disclosed herein (e.g. veverimer). Example 3(b) also demonstrates that if the amount of oxygen is reduced, e.g. by reducing the head space in the unit dosage form, degradation of the polymer is further minimized.

It was postulated that if there was no oxygen present in the unit dosage form then no degradation of the crosslinked poly(allylamine) polymer disclosed herein (e.g. veverimer) would be observed. It was further postulated, that an oxygen scavenger would reduce the amount of oxygen in the unit dosage form such that no oxygen mediated degradation (as measured by allylamine concentration) of the polymer occurred.

Thus, this example investigates the effect of an oxygen scavenger in the unit dosage form on the stability of the drug substance (e.g. the crosslinked poly(allylamine) polymer such as veverimer).

**Manufacture of veverimer packets (unit dosage forms)**

The procedure described in Example 3(a) was followed to produce the unit dosage forms for this Example. Veverimer in a 1.5 g amount was packaged in 6.35 cm x 7.94 cm (2.5" × 3.125") packets of paket material D. Packet material D comprisies an oxygen scavenger. Packet material D has a composition of PET (12 μm)/Aluminum foil (12 μm)/ Oxygen scavenger layer (70 μm)/adhesive. Packet material D has an oxygen absorption volume of greater than or equal to 0.01 mL/cm$^2$ at 25°C/0%RH for 7 days (0%RH can be achieved under dry conditions using a desiccant).

**Stability study with oxygen scavenger**

Having manufactured the unit dosage form for testing, a stability study was initiated. The study measured the

concentration of allylamine over a period of six months in the 1.5 g unit dosage form with an oxygen scavenger at 25°C/60% relative humidity (RH) and 40°C/75% RH. The concentration (ppm) of allylamine (AA) in the unit dosage form was determined by cation exchange ion chromatography (as per the protocol described herein under the "Cation IC Extraction Procedure").

The cumulative concentration of allylamine in the 1.5 g unit dosage form with an oxygen scavenger over six months at 25°C/60% RH and 40°C/75% RH is displayed in Table A8. The data from Table A8 is presented in Figure 4.

Table A8: Concentration (ppm) of AA in veverimer unit dosage form at 25°C/60% RH and 40°C/75% RH

| 1.5 g unit dosage form with an oxygen scavenger | Time (Months) | | | | | |
|---|---|---|---|---|---|---|
| | 0 | 0.5 | 1 | 2 | 4 | 6 |
| | Concentration of AA (ppm) | | | | | |
| 25C/60% RH | 1.3 | 1.4 | 1.3 | 1.2 | 1.3 | 0.9 |
| 40C/75% RH | 1.3 | 1.2 | 1.1 | 0.9 | 1.2 | 0.8 |

**Discussion of results**

The data in Table A8 and Figure 4 shows that the concentration of allylamine is constant over six months for the 1.5 g unit dosage form with an oxygen scavenger at 25°C/60% RH and 40°C/75% RH. This suggests that no significant degradation has occurred because the concentration of allylamine has not increased.

To conclude, this example demonstrates that an approach to increase the stability of the crosslinked poly(allylamine) polymer disclosed herein (e.g. veverimer) can be removing oxygen from the unit dosage form, which can be achieved with an oxygen scavenger.

**Example 3 (d) Impact of unit dosage form environment on stability**

**Introduction**

Example 3 (c) demonstrates that if oxygen in the unit dosage form is removed, e.g. by an oxygen scavenger, degradation of the crosslinked poly(allylamine) polymer disclosed herein (e.g. veverimer) is minimized. In Example c), the unit dosage form was produced in air which explains why some oxygen is present in the unit dosage form.

It was postulated that if there was no oxygen present in the unit dosage form when it was produced, then no degradation of the crosslinked poly(allylamine) polymer disclosed herein (e.g. veverimer) would be observed. It was further postulated, that producing the unit dosage form in low oxygen environments or an environment with no oxygen, would result in less or no degradation (as measured by allylamine concentration) of the polymer by exposure to oxygen.

Thus, this example investigates the effect of no or low oxygen environments in the unit dosage form on the stability of the drug substance (e.g. the crosslinked poly(allylamine) polymer such as veverimer).

**Manufacture of veverimer packets (unit dosage forms)**

The procedure described in Example 3 (a) was followed to produce the unit dosage forms for this Example. Veverimer in a 1.5 g amount was packaged in 2.54 cm x 7.62 cm (1" × 3") packets prepared from packet material E under three different environments. The environments were air, 8% oxygen and 92% nitrogen, and 99+% nitrogen. Thus, three 1.5 g unit dosage forms were produced, each with an internal environment of air, 8% oxygen and 92% nitrogen, or 99+% nitrogen. Packet material E is a lamination of film foil and polyethylene. In particular, Packet material E is a PET/adhesive/foil/adhesive/LLDPE laminate having an oxygen transfer rate of 0.001 CC/$m^2$/day and a water vapor transfer rate of < 0.0005 g/100$in^2$/day.

**Stability study with different environments**

Having manufactured the unit dosage form for testing, a stability study was initiated. The study measured the concentration of allylamine over a period of six months in the 1.5 unit dosage forms at 25°C/60% relative humidity (RH) and 40°C/75% RH. The concentration (ppm) of allylamine (AA) in the unit dosage form was determined by cation exchange ion chromatography (as per the protocol described herein under the "Cation IC Extraction Procedure").

The cumulative concentration of allylamine in the 1.5 g unit dosage forms with different environments over six months at 25°C/60% RH is displayed in Table A9. The cumulative concentration of allylamine in the 1.5 g unit dosage forms

with different environments over six months at 40°C/75% RH is displayed in Table A10.

Table A9: Concentration (ppm) of AA in veverimer unit dosage form at 25°C/60% RH

| Internal environment of 1.5 g unit dosage form | Time (Months) | | | | | |
|---|---|---|---|---|---|---|
| | 0 | 0.5 | 1 | 2 | 3 | 6 |
| | Concentration of AA (ppm) | | | | | |
| Air | 0.4 | 3.2 | 3.0 | 2.6 | 2.8 | 2.5 |
| 8% Oxygen and 92% nitrogen | 0.4 | 2.5 | 2.3 | 1.9 | 2.3 | 1.9 |
| 99+% Nitrogen | 0.4 | 1.0 | 1.7 | 0.9 | 1.3 | 0.6 |

Table A10: Concentration (ppm) of AA in veverimer unit dosage form at 40°C/75% RH

| Internal environment of 1.5 g unit dosage form | Time (Months) | | | | | |
|---|---|---|---|---|---|---|
| | 0 | 0.5 | 1 | 2 | 3 | 6 |
| | Concentration of AA (ppm) | | | | | |
| Air | 0.4 | 3.9 | 3.4 | 3.5 | 5.2 | 2.7 |
| 8% Oxygen and 92% nitrogen | 0.4 | 2.3 | 2.3 | 1.9 | 2.3 | 1.4 |
| 99+% Nitrogen | 0.4 | 1.1 | 1.1 | 1.1 | 1.4 | 0.6 |

The data from Table A9 is presented in Figure 5. The data from Table A10 is presented in Figure 6.

**Discussion of results**

This example demonstrates that an approach to ensure the stability of the crosslinked poly(allylamine) polymer disclosed herein (e.g. veverimer) can be achieved by removing oxygen from the unit dosage form or by decreasing the amount of oxygen in the unit dosage form. Oxygen can be removed the unit dosage form by producing the unit dosage form in an inert environment. A nitrogen environment is an example of an inert environment.

## Claims

1. A crosslinked poly(allylamine) polymer wherein the crosslinked poly(allylamine) polymer is veverimer, and less than 1.0% of the total number of carbon atoms present in the crosslinked poly(allylamine) polymer are $sp^2$ allyl carbons, wherein

   the percentage of $sp^2$ allyl carbons in the crosslinked poly(allylamine) polymer is determined by $^{13}C$ NMR, and the integration of the $sp^2$ allyl carbon peaks between 110 - 150 ppm and alkyl carbon peaks between 0 - 80 ppm is used to quantitate the percentage of $sp^2$ allyl carbons using the formula

$$\%\ \mathrm{sp2}\ allyl\ carbons = \frac{peak\ integration\ (110\text{–}150\ ppm)}{peak\ integration\ (0\text{–}80\ ppm) + peak\ integration\ (110\text{–}150\ ppm)} \times 100.$$

2. The crosslinked poly(allylamine) polymer of claim 1 wherein less than 0.9% of the total number of carbon atoms present in the crosslinked poly(allylamine) polymer are $sp^2$ allyl carbons.

3. The crosslinked poly(allylamine) polymer of any preceding claim wherein less than 0.8% of the total number of carbon atoms present in the crosslinked poly(allylamine) polymer are $sp^2$ allyl carbons.

4. The crosslinked poly(allylamine) polymer of any preceding claim wherein less than 0.7% of the total number of carbon atoms present in the crosslinked poly(allylamine) polymer are $sp^2$ allyl carbons.

5. The crosslinked poly(allylamine) polymer of any preceding claim wherein less than 0.6% of the total number of carbon

atoms present in the crosslinked poly(allylamine) polymer are $sp^2$ allyl carbons.

6. The crosslinked poly(allylamine) polymer of any preceding claim wherein more than 0.3% of the total number of carbon atoms present in the crosslinked poly(allylamine) polymer are $sp^2$ allyl carbons.

7. The crosslinked poly(allylamine) polymer of any preceding claim wherein the crosslinked poly(allylamine) polymer contains less than 20 ppm allylamine ($H_2C=CHCH_2NH_2$) as an impurity, wherein the allylamine content is determined by the Cation IC Extraction Procedure.

8. The crosslinked poly(allylamine) polymer of any preceding claim wherein when tested with the Heated Stability Assay as carried out in the specification (Stability Assay 2) the allylamine content of the crosslinked poly(allylamine) polymer increases by less than 2.5 ppm/day allylamine.

9. The crosslinked poly(allylamine) polymer of any preceding claim wherein when tested with the Heated Stability Assay (Stability Assay 2) the allylamine content of the crosslinked poly(allylamine) polymer increases by less than 2.0 ppm/day allylamine.

10. The crosslinked poly(allylamine) polymer of any preceding claim wherein when tested with the Heated Stability Assay (Stability Assay 2) the allylamine content of the crosslinked poly(allylamine) polymer increases by less than 1.5 ppm/day allylamine.

11. The crosslinked poly(allylamine) polymer of any preceding claim for use in a method of treating metabolic acidosis.

## Patentansprüche

1. Vernetztes Poly(allylamin)polymer, wobei das vernetzte Poly(allylamin)polymer Veverimer ist und weniger als 1,0 % der Gesamtzahl von in dem vernetzten Poly(allylamin)polymer vorhandenen Kohlenstoffatomen $sp^2$-Allylkohlenstoffe sind, wobei

   der prozentuelle Anteil an $sp^2$-Allylkohlenstoffen in dem vernetzten Poly(allylamin)polymer durch $^{13}C$-NMR bestimmt wird und
   das Integral der $sp^2$-Allylkohlenstoffpeaks zwischen 110 und 150 ppm und der Alkylkohlenstoffpeaks zwischen 0 und 80 ppm verwendet wird, um den prozentuellen Anteil von $sp^2$-Allylkohlenstoffen unter Verwendung der folgenden Formel zu quantifizieren

$$\% sp2 - Allylkohlenstoffe = \frac{Peakintegral\ (110-150\ ppm)}{Peakintegral\ (0-80\ ppm) + Peakintegral\ (110-150\ ppm)}\ x\ 100.$$

2. Vernetztes Poly(allylamin)polymer nach Anspruch 1, wobei weniger als 0,9 % der Gesamtzahl von in dem vernetzten Poly(allylamin)polymer vorhandenen Kohlenstoffatomen $sp^2$-Allylkohlenstoffe sind.

3. Vernetztes Poly(allylamin)polymer nach einem der vorstehenden Ansprüche, wobei weniger als 0,8 % der Gesamtzahl der in dem vernetzten Poly(allylamin)polymer vorhandenen Kohlenstoffatome $sp^2$-Allylkohlenstoffe sind.

4. Vernetztes Poly(allylamin)polymer nach einem der vorstehenden Ansprüche, wobei weniger als 0,7 % der Gesamtzahl der in dem vernetzten Poly(allylamin)polymer vorhandenen Kohlenstoffatome $sp^2$-Allylkohlenstoffe sind.

5. Vernetztes Poly(allylamin)polymer nach einem der vorstehenden Ansprüche, wobei weniger als 0,6 % der Gesamtzahl der in dem vernetzten Poly(allylamin)polymer vorhandenen Kohlenstoffatome $sp^2$-Allylkohlenstoffe sind.

6. Vernetztes Poly(allylamin)polymer nach einem der vorstehenden Ansprüche, wobei mehr als 0,3 % der Gesamtzahl der in dem vernetzten Poly(allylamin)polymer vorhandenen Kohlenstoffatome $sp^2$-Allylkohlenstoffe sind.

7. Vernetztes Poly(allylamin)polymer nach einem der vorstehenden Ansprüche, wobei das vernetzte Poly(allylamin) polymer weniger als 20 ppm Allylamin ($H_2C=CHCH_2NH_2$) als Verunreinigung enthält, wobei der Allylamingehalt durch das Kationen-IC-Extraktionsverfahren bestimmt wird.

**8.** Vernetztes Poly(allylamin)polymer nach einem der vorstehenden Ansprüche, wobei, wenn mit der Heizstabilitätsprüfung (Stabilitätsprüfung 2) wie in der Beschreibung beschrieben geprüft, der Allylamingehalt des vernetzten Poly (allylamin) polymers um weniger als 2,5 ppm/Tag Allylamin zunimmt.

**9.** Vernetztes Poly(allylamin)polymer nach einem der vorstehenden Ansprüche, wobei, wenn mit der Heizstabilitätsprüfung (Stabilitätsprüfung 2) geprüft, der Allylamingehalt des vernetzten Poly(allylamin)polymers um weniger als 2,0 ppm/Tag Allylamin zunimmt.

**10.** Vernetztes Poly(allylamin)polymer nach einem der vorstehenden Ansprüche, wobei, wenn mit der Heizstabilitätsprüfung (Stabilitätsprüfung 2) geprüft, der Allylamingehalt des vernetzten Poly(allylamin)polymers um weniger als 1,5 ppm/Tag Allylamin zunimmt.

**11.** Vernetztes Poly(allylamin)polymer nach einem der vorstehenden Ansprüche zur Verwendung bei einem Verfahren zur Behandlung von metabolischer Azidose.

**Revendications**

**1.** Polymère de poly(allylamine) réticulé, dans lequel le polymère de poly(allylamine) réticulé est un veverimer, et moins de 1,0 % du nombre total d'atomes de carbone présents dans le polymère de poly(allylamine) réticulé sont des carbones allyliques $sp^2$, dans lequel le pourcentage de carbones allyliques $sp^2$ dans le polymère poly(allylamine) réticulé est déterminé par RMN $^{13}C$, et l'intégration des pics de carbone allylique $sp^2$ entre 110-150 ppm et des pics de carbone alkylique entre 0-80 ppm est utilisée pour quantifier le pourcentage de carbones allyliques $sp^2$ selon la formule

$$\%\,carbones\ allyliques\ \ sp2\ = \frac{intégration\ des\ pics\ (110-150\ ppm)}{intégration\ des\ pics\ (0-80\ ppm)+intégration\ des\ pics\ (110-150\ ppm)} \times 100.$$

**2.** Polymère poly(allylamine) réticulé selon la revendication 1, dans lequel moins de 0,9 % du nombre total d'atomes de carbone présents dans le polymère de poly(allylamine) réticulé sont des carbones allyliques $sp^2$.

**3.** Polymère poly(allylamine) réticulé selon l'une quelconque des revendications précédentes, dans lequel moins de 0,8 % du nombre total d'atomes de carbone présents dans le polymère poly(allylamine) réticulé sont des carbones allyliques $sp^2$.

**4.** Polymère poly(allylamine) réticulé selon l'une quelconque des revendications précédentes, dans lequel moins de 0,7 % du nombre total d'atomes de carbone présents dans le polymère poly(allylamine) réticulé sont des carbones allyliques sp2.

**5.** Polymère poly(allylamine) réticulé selon l'une quelconque des revendications précédentes, dans lequel moins de 0,6 % du nombre total d'atomes de carbone présents dans le polymère poly(allylamine) réticulé sont des carbones allyliques sp2.

**6.** Polymère poly(allylamine) réticulé selon l'une quelconque des revendications précédentes, dans lequel plus de 0,3 % du nombre total d'atomes de carbone présents dans le polymère poly(allylamine) réticulé sont des carbones allyliques $sp^2$.

**7.** Polymère poly(allylamine) réticulé selon l'une quelconque des revendications précédentes, dans lequel le polymère poly(allylamine) réticulé contient moins de 20 ppm d'allylamine ($H_2C{=}CHCH_2NH_2$) comme impureté, dans lequel la teneur en allylamine est déterminée par la procédure d'extraction par IC de cations.

**8.** Polymère poly(allylamine) réticulé selon l'une quelconque des revendications précédentes, dans lequel, lorsqu'il est testé avec le test de stabilité à chaud tel qu'effectué dans la spécification (test de stabilité 2), la teneur en allylamine du polymère poly(allylamine) réticulé augmente de moins de 2,5 ppm/jour d'allylamine.

**9.** Polymère poly(allylamine) réticulé selon l'une quelconque des revendications précédentes, dans lequel, lorsqu'il est

testé avec le test de stabilité à chaud (test de stabilité 2), la teneur en allylamine du polymère poly(allylamine) réticulé augmente de moins de 2,0 ppm/jour d'allylamine.

**10.** Polymère poly(allylamine) réticulé selon l'une quelconque des revendications précédentes, dans lequel, lorsqu'il est testé avec le test de stabilité à chaud (test de stabilité 2), la teneur en allylamine du polymère poly(allylamine) réticulé augmente de moins de 1,5 ppm/jour d'allylamine.

**11.** Polymère poly(allylamine) réticulé selon l'une quelconque des revendications précédentes, destiné à être utilisé dans un procédé de traitement de l'acidose métabolique.

**a.**

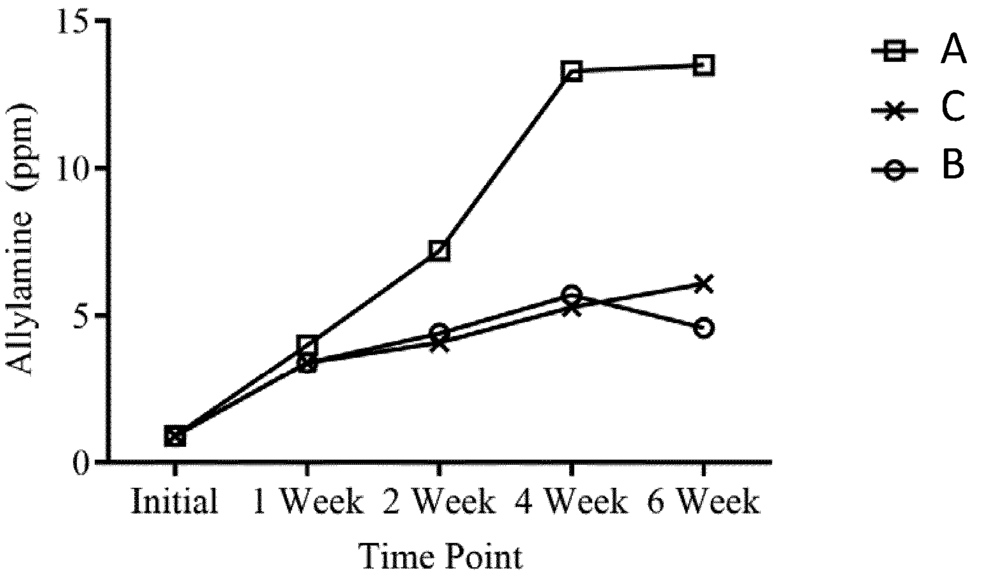


Figure 1A

**b.**

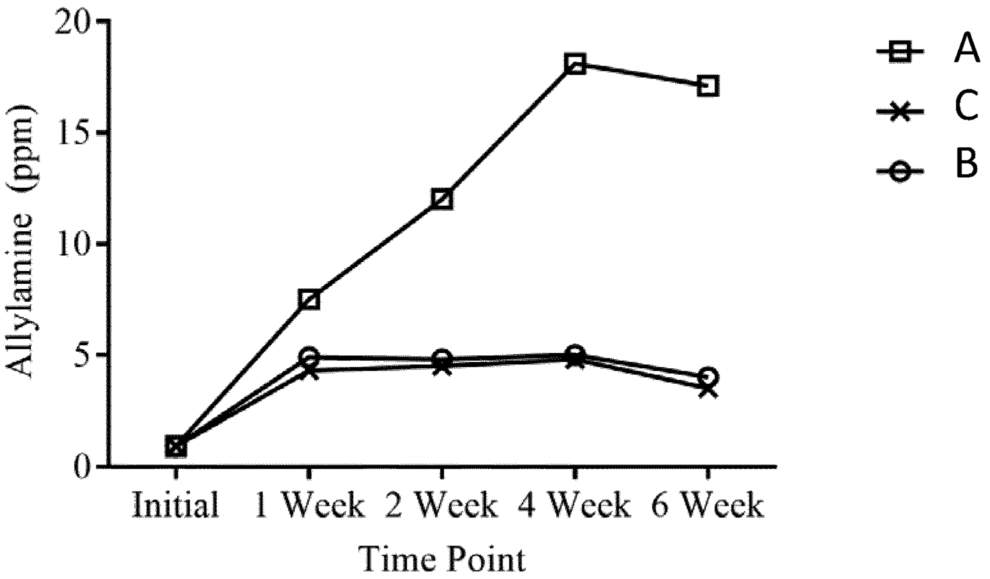


Figure 1B

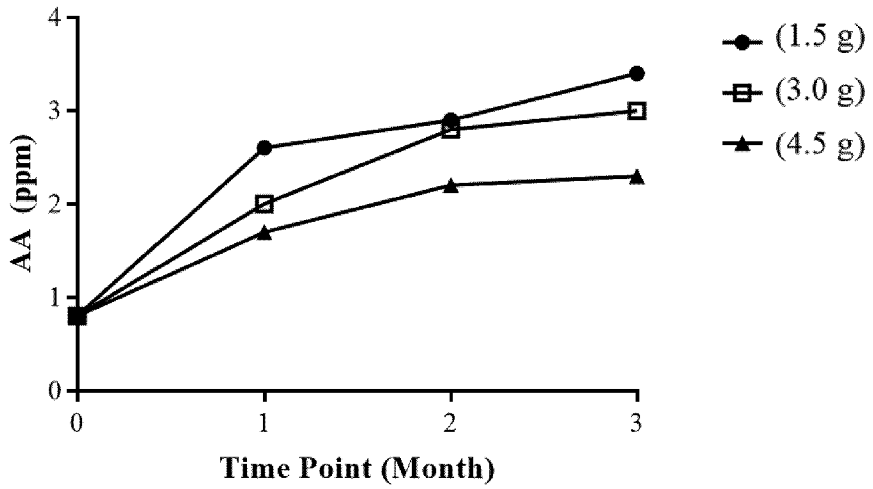


Figure 2

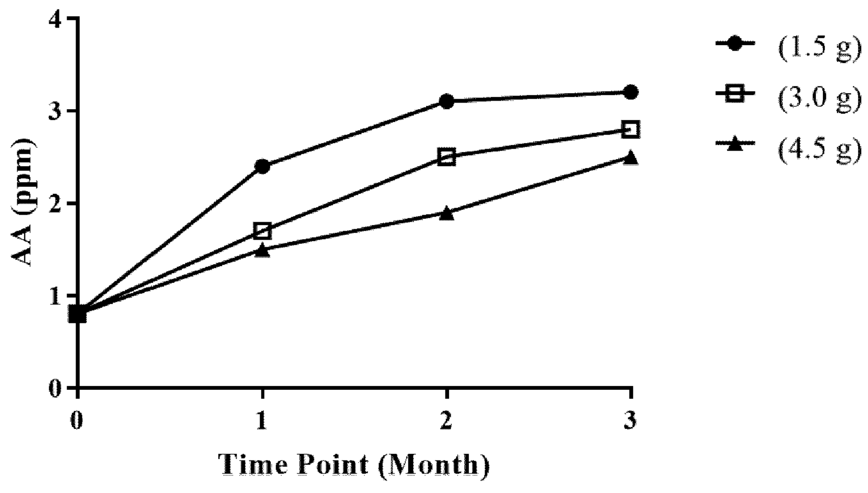


Figure 3

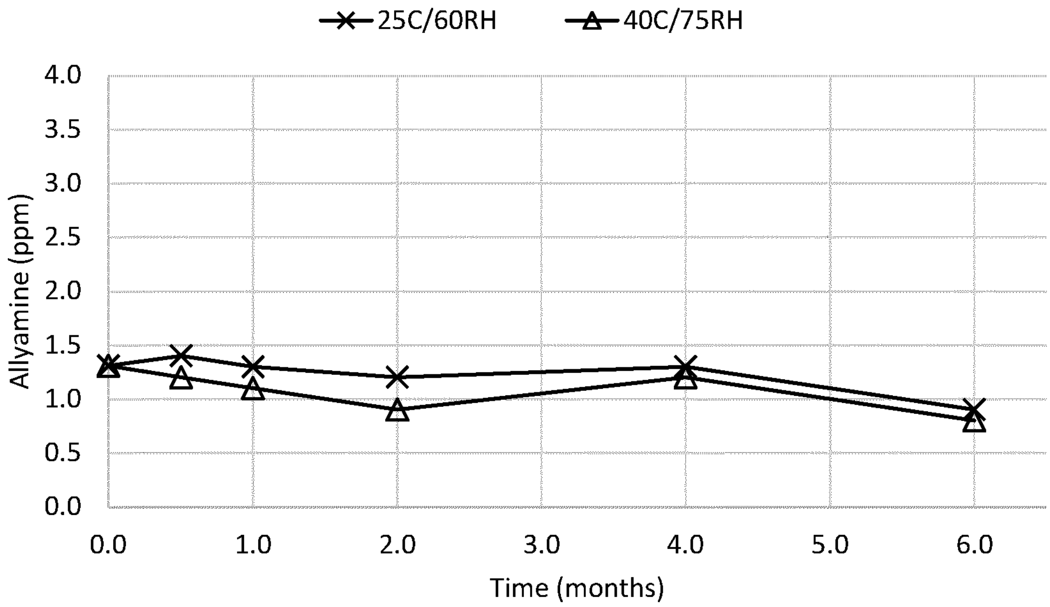


Figure 4

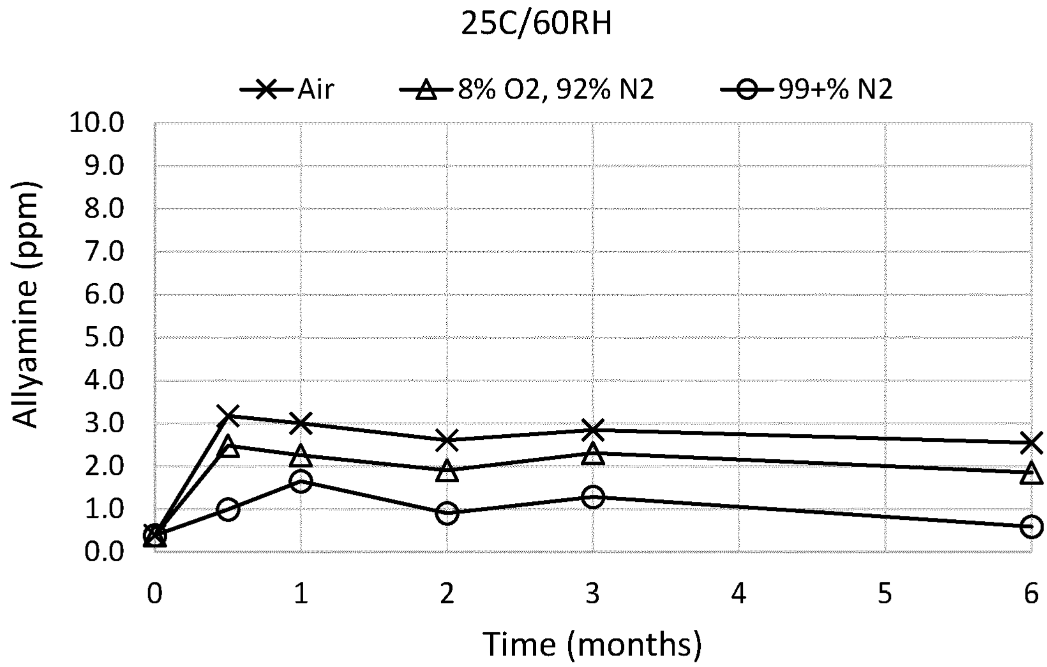


Figure 5

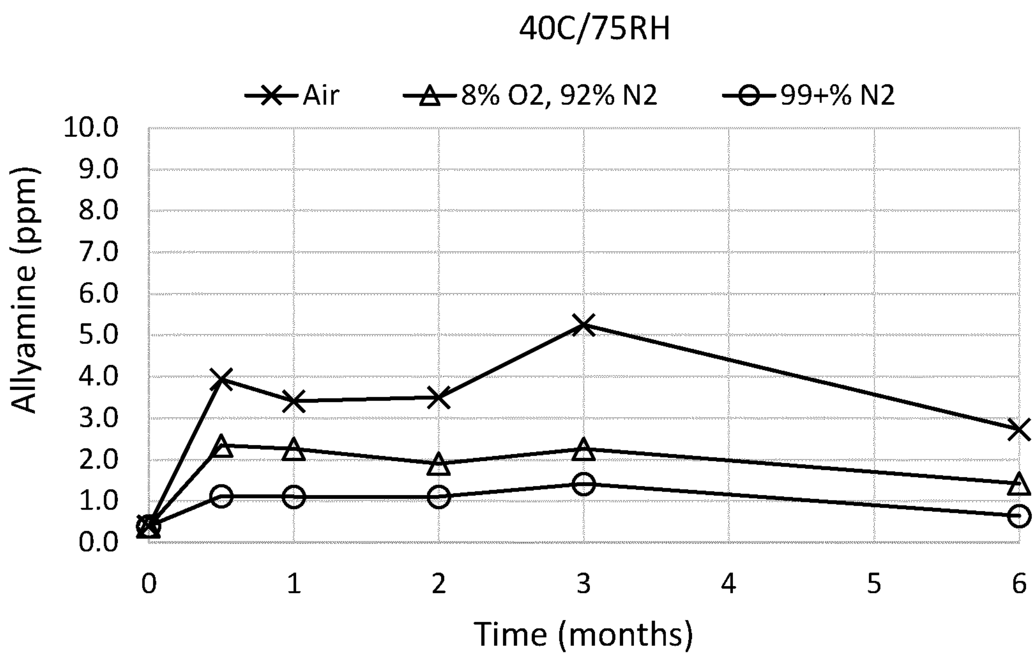
40C/75RH
Air
8% O2, 92% N2
99+% N2
10.0
9.0
8.0
7.0
6.0
5.0
4.0
3.0
2.0
1.0
0.0
Allyamine (ppm)
0
1
2
3
4
5
6
Time (months)

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9205107 B, Klaerner
- WO 2019236636 A1
- WO 2016094685 A1
- WO 2014197725 A1
- WO 2017193050 A1
- WO 2017193064 A1
- WO 2017193024 A1
- WO 2019090176 A1
- WO 2019090177 A1
- WO 2019236639 A1
- WO 2019236124 A1
- US 20060076536 A
- US 20070084144 A
- US 20060260967 A


### Non-patent literature cited in the description


- **WESSON et al.** Long-term safety and efficacy of veverimer in patients with metabolic acidosis in chronic kidney disease: a multicentre, randomised, blinded, placebo-controlled, 40-week extension. *The Lancet*, 2019, vol. 394 (10196), 396-406
- **BUSHINSKY et al.** Randomized, Controlled Trial of TRC101 to Increase Serum Bicarbonate in Patients with CKD. *Clin. J. Am. Soc. Nephrol.*, 2018, vol. 13, 26-35
- **JUNG et al.** *European Journal of Pharmaceutics and Biopharmaceutics*, 2000, vol. 50, 147-160
- **JANI et al.** *Internation. Journal of Pharmaceutics*, 1992, vol. 84, 245-252
- **JANI et al.** *J. Pharm. Pharmacol.*, 1989, vol. 41, 809-812
- **STEVENS T** ; **CONWELL DL** ; **ZUCCARO G** ; **VAN LENTE F** ; **KHANDWALA F** ; **PURICH E et al.** Electrolyte composition of endoscopically collected duodenal drainage fluid after synthetic porcine secretin stimulation in healthy subjects. *Gastrointestinal endoscopy.*, 2004, vol. 60 (3), 351-5
- **FORDTRAN J** ; **LOCKLEAR T.** Ionic constituents and osmolality of gastric and small-intestinal fluids after eating.. *Digest Dis Sci.*, 1966, vol. 11 (7), 503-21
- **PHISITKUL S** ; **HACKER C** ; **SIMONI J** ; **TRAN RM** ; **WESSON DE**. ietary protein causes a decline in the glomerular filtration rate of the remnant kidney mediated by metabolic acidosis and endothelin receptors.. *Kidney international.*, 2008, vol. 73 (2), 192-9
- **TERAI K** ; **K MIZUKAMI** ; **M OKADA**. Comparison of chronic renal failure rats and modification of the preparation protocol as a hyperphosphatemia model. *Nephrol*, 2008, vol. 13, 139-146
- Remington's Pharmaceutical Sciences
- **GAIKWAD et al.** *Environmental Chemistry Letters*, 2018, vol. 16, 523-538
- **GILLIES et al.** *BBA - Reviews on Cancer*, 2019, vol. 1871, 273-280